# EUROPEAN PATENT APPLICATION

(11) **EP 1 505 150 A1**
(43) Date of publication of application: **09.02.2005**
(21) Application number: 03749815.1
(22) Date of filing: 13.05.2003
(51) Int. Cl.: C12N 15/09, C07K 14/47, C07K 16/18, C12N 5/10

(54) **NOVEL PROTEINS AND DNAS THEREOF**

(30) Priority: 14.05.2002 JP 2002139184; 30.05.2002 JP 2002157669; 26.07.2002 JP 2002218324
(71) Applicant: Takeda Pharmaceutical Company Limited, Osaka (JP)
(72) Inventor: NAKANISHI, Atsushi, Tsukuba-shi, Ibaraki 305-0025 (JP); MIYA, Hiroyuki, Tsukuba-shi, Ibaraki 305-0004 (JP); IWAMA, Toshi, Tsukuba-shi, Ibaraki 305-0032 (JP)
(74) Representative: Rickard, Timothy Mark Adrian
(86) International application number: PCT/JP2003/005941
(87) International publication number: WO 2003/095644

(57) **Abstract**

A protein having an amino acid sequence which is the same of substantially the same as an amino acid sequence represented by SEQ ID NO: 1, SEQ ID NO:8, SEQ ID NO:11 or SEQ ID NO: 15 is useful as , for example, a diagnostic marker for digestive diseases, immune diseases associating thymic disorders, pancreatic diseases, diabetes, genital diseases, central nerve system diseases, circulatory diseases, muscular diseases, cancer, etc. A compound promoting or inhibiting the activity of this protein which is obtained by a screening method with the use of the protein is usable as, for example, a preventative or a remedy for the above diseases.

SEQUENCE LISTING
<110> Takeda Chemical Industries, Ltd.
<120> Novel Protein and its DNA
<130> 3051WOOP
<150> JP 2002-139184
<151> 2002-5-14
<150> JP 2002-157669
<151> 2002-5-30
<150> JP 2002-218324
<151> 2002-7-26
<160> 44
<210> 1
<211> 1118
<212> PRT
<213> Homo sapiens
<400> 1
Met Val Asp Leu Glu Ser Glu Val Pro Pro Leu Pro Pro Arg Tyr Arg
                5                   10                  15
Phe Arg Asp Leu Leu Leu Gly Asp Gln Gly Trp Gln Asn Asp Asp Arg
            20                  25                  30
Val Gln Val Glu Phe Tyr Met Asn Glu Asn Thr Phe Lys Glu Arg Leu
        35                  40                  45
Lys Leu Phe Phe Ile Lys Asn Gln Arg Ser Ser Leu Arg Ile Arg Leu
    50                  55                  60
Phe Asn Phe Ser Leu Lys Leu Leu Ser Cys Leu Leu Tyr Ile Ile Arg
65                  70                  75                  80

Val Leu Leu Glu Asn Pro Ser Gln Gly Asn Glu Trp Ser His Ile Phe
                85                  90                  95
Trp Val Asn Arg Ser Leu Pro Leu Trp Gly Leu Gln Val Ser Val Ala
            100                 105                 110
Leu Ile Ser Leu Phe Glu Thr Ile Leu Leu Gly Tyr Leu Ser Tyr Lys
        115                 120                 125
Gly Asn Ile Trp Glu Gln Ile Leu Arg Ile Pro Phe Ile Leu Glu Ile
    130                 135                 140
Ile Asn Ala Val Pro Phe Ile Ile Ser Ile Phe Trp Pro Ser Leu Arg
145                 150                 155                 160
Asn Leu Phe Val Pro Val Phe Leu Asn Cys Trp Leu Ala Lys His Ala
                165                 170                 175
Leu Glu Asn Met Ile Asn Asp Leu His Arg Ala Ile Gln Arg Thr Gln
            180                 185                 190
Ser Ala Met Phe Asn Gln Val Leu Ile Leu Ile Ser Thr Leu Leu Cys
        195                 200                 205
Leu Ile Phe Thr Cys Ile Cys Gly Ile Gln His Leu Glu Arg Ile Gly
    210                 215                 220
Lys Lys Leu Asn Leu Phe Asp Ser Leu Tyr Phe Cys Ile Val Thr Phe
225                 230                 235                 240
Ser Thr Val Gly Phe Gly Asp Val Thr Pro Glu Thr Trp Ser Ser Lys
                245                 250                 255
Leu Phe Val Val Ala Met Ile Cys Val Ala Leu Val Val Leu Pro Ile
            260                 265                 270
Gln Phe Glu Gln Leu Ala Tyr Leu Trp Met Glu Arg Gln Lys Ser Gly
        275                 280                 285
Gly Asn Tyr Ser Arg His Arg Ala Gln Thr Glu Lys His Val Val Leu
    290                 295                 300
Cys Val Ser Ser Leu Lys Ile Asp Leu Leu Met Asp Phe Leu Asn Glu

305                 310                 315                 320
Phe Tyr Ala His Pro Arg Leu Gln Asp Tyr Tyr Val Val Ile Leu Cys
                325                 330                 335
Pro Thr Glu Met Asp Val Gln Val Arg Arg Val Leu Gln Ile Pro Met
            340                 345                 350
Trp Ser Gln Arg Val Ile Tyr Leu Gln Gly Ser Ala Leu Lys Asp Gln
        355                 360                 365
Asp Leu Leu Arg Ala Lys Met Asp Asp Ala Glu Ala Cys Phe Ile Leu
    370                 375                 380
Ser Ser Arg Cys Glu Val Asp Arg Thr Ser Ser Asp His Gln Thr Ile
385                 390                 395                 400
Leu Arg Ala Trp Ala Val Lys Asp Phe Ala Pro Asn Cys Pro Leu Tyr
                405                 410                 415
Val Gln Ile Leu Lys Pro Glu Asn Lys Phe His Ile Lys Phe Ala Asp
            420                 425                 430
His Val Val Cys Glu Glu Glu Phe Lys Tyr Ala Met Leu Ala Leu Asn
        435                 440                 445
Cys Ile Cys Pro Ala Thr Ser Thr Leu Ile Thr Leu Leu Val His Thr
    450                 455                 460
Ser Arg Gly Gln Cys Val Cys Leu Cys Cys Arg Glu Gly Gln Gln Ser
465                 470                 475                 480
Pro Glu Gln Trp Gln Lys Met Tyr Gly Arg Cys Ser Gly Asn Glu Val
                485                 490                 495
Tyr His Ile Val Leu Glu Glu Ser Thr Phe Phe Ala Glu Tyr Glu Gly
            500                 505                 510
Lys Ser Phe Thr Tyr Ala Ser Phe His Ala His Lys Lys Phe Gly Val
        515                 520                 525
Cys Leu Ile Gly Val Arg Arg Glu Asp Asn Lys Asn Ile Leu Leu Asn
    530                 535                 540

Pro Gly Pro Arg Tyr Ile Met Asn Ser Thr Asp Ile Cys Phe Tyr Ile
545                 550                 555                 560
Asn Ile Thr Lys Glu Glu Asn Ser Ala Phe Lys Asn Gln Asp Gln Gln
                565                 570                 575
Arg Lys Ser Asn Val Ser Arg Ser Phe Tyr His Gly Pro Ser Arg Leu
            580                 585                 590
Pro Val His Ser Ile Ile Ala Ser Met Gly Thr Val Ala Ile Asp Leu
        595                 600                 605
Gln Asp Thr Ser Cys Arg Ser Ala Ser Gly Pro Thr Leu Ser Leu Pro
    610                 615                 620
Thr Glu Gly Ser Lys Glu Ile Arg Arg Pro Ser Ile Ala Pro Val Leu
625                 630                 635                 640
Glu Val Ala Asp Thr Ser Ser Ile Gln Thr Cys Asp Leu Leu Ser Asp
                645                 650                 655
Gln Ser Glu Asp Glu Thr Thr Pro Asp Glu Glu Met Ser Ser Asn Leu
            660                 665                 670
Glu Tyr Ala Lys Gly Tyr Pro Pro Tyr Ser Pro Tyr Ile Gly Ser Ser
        675                 680                 685
Pro Thr Phe Cys His Leu Leu His Glu Lys Val Pro Phe Cys Cys Leu
    690                 695                 700
Arg Leu Asp Lys Ser Cys Gln His Asn Tyr Tyr Glu Asp Ala Lys Ala
705                 710                 715                 720
Tyr Gly Phe Lys Asn Lys Leu Ile Ile Val Ala Ala Glu Thr Ala Gly
                725                 730                 735
Asn Gly Leu Tyr Asn Phe Ile Val Pro Leu Arg Ala Tyr Tyr Arg Pro
            740                 745                 750
Lys Lys Glu Leu Asn Pro Ile Val Leu Leu Leu Asp Asn Pro Leu Asp
        755                 760                 765
Asp Leu Leu Arg Cys Gly Val Thr Phe Ala Ala Asn Met Val Val Val

770                 775                 780
Asp Lys Glu Ser Thr Met Ser Ala Glu Glu Asp Tyr Met Ala Asp Ala
785                 790                 795                 800
Lys Thr Ile Val Asn Val Gln Thr Leu Phe Arg Leu Phe Ser Ser Leu
                805                 810                 815
Ser Ile Ile Thr Glu Leu Thr His Pro Ala Asn Met Arg Phe Met Gln
            820                 825                 830
Phe Arg Ala Lys Asp Cys Tyr Ser Leu Ala Leu Ser Lys Leu Glu Lys
        835                 840                 845
Lys Glu Arg Glu Arg Gly Ser Asn Leu Ala Phe Met Phe Arg Leu Pro
    850                 855                 860
Phe Ala Ala Gly Arg Val Phe Ser Ile Ser Met Leu Asp Thr Leu Leu
865                 870                 875                 880
Tyr Gln Ser Phe Val Lys Asp Tyr Met Ile Ser Ile Thr Arg Leu Leu
                885                 890                 895
Leu Gly Leu Asp Thr Thr Pro Gly Ser Gly Phe Leu Cys Ser Met Lys
            900                 905                 910
Ile Thr Ala Asp Asp Leu Trp Ile Arg Thr Tyr Ala Arg Leu Tyr Gln
        915                 920                 925
Lys Leu Cys Ser Ser Thr Gly Asp Val Pro Ile Gly Ile Tyr Arg Thr
    930                 935                 940
Glu Ser Gln Lys Leu Thr Thr Ser Glu Ser Gln Ile Ser Ile Ser Val
945                 950                 955                 960
Glu Glu Trp Glu Asp Thr Lys Asp Ser Lys Glu Gln Gly His His Arg
                965                 970                 975
Ser Asn His Arg Asn Ser Thr Ser Ser Asp Gln Ser Asp His Pro Leu
            980                 985                 990
Leu Arg Arg Lys Ser Met Gln Trp Ala Arg Arg Leu Ser Arg Lys Gly
        995                 1000                1005

Pro Lys His Ser Gly Lys Thr Ala Glu Lys Ile Thr Gln Gln Arg Leu
    1010                1015                1020
Asn Leu Tyr Arg Arg Ser Glu Arg Gln Glu Leu Ala Glu Leu Val Lys
1025                1030                1035                1040
Asn Arg Met Lys His Leu Gly Leu Ser Thr Val Gly Tyr Asp Glu Met
                1045                1050                1055
Asn Asp His Gln Ser Thr Leu Ser Tyr Ile Leu Ile Asn Pro Ser Pro
            1060                1065                1070
Asp Thr Arg Ile Glu Leu Asn Asp Val Val Tyr Leu Ile Arg Pro Asp
        1075                1080                1085
Pro Leu Ala Tyr Leu Pro Asn Ser Glu Pro Ser Arg Arg Asn Ser Ile
    1090                1095                1100
Cys Asn Val Thr Gly Gln Asp Ser Arg Glu Glu Thr Gln Leu
1105                1110                1115

<210> 2
<211> 3354
<212> DNA
<213> Homo sapiens
<400> 2
atggttgatt tggagagcga agtgccccct ctgcctccca ggtacaggtt tcgagatttg 60
ctgctagggg accaaggatg gcaaaacgac gacagggtac aagttgaatt ctatatgaat 120
gaaaatacat ttaaagaaag actaaaatta tttttcataa aaaaccagag atcaagtcta 180
aggatacgcc tgttcaattt ttctctcaaa ttactaagct gcttattata cataatccga 240
gtactactag aaaacccttc acaaggaaat gaatggtctc atatcttttg ggtgaacaga 300
agtctacctt tgtggggctt acaggtttca gtggcattga taagtctgtt tgaaacaata 360
ttacttggtt atcttagtta taagggaaac atctgggaac agattttacg aatacccttc 420
atcttggaaa taattaatgc agttcccttc attatctcaa tattctggcc ttccttaagg 480
aatctatttg tcccagtctt tctgaactgt tggcttgcca aacatgcctt ggaaaatatg 540

attaatgatc tacacagagc cattcagcgt acacagtctg caatgtttaa tcaagttttg 600
attttaatat ctacattact atgccttatc ttcacctgca tttgtgggat ccaacatctg 660
gaacgaatag gaaagaagct gaatctcttt gactcccttt atttctgcat tgtgacgttt 720
tctactgtgg gcttcgggga tgtcactcct gaaacatggt cctccaagct ttttgtagtt 780
gctatgattt gtgttgctct tgtggttcta cccatacagt ttgaacagct ggcttatttg 840
tggatggaga gacaaaagtc aggaggaaac tatagtcgac atagagctca aactgaaaag 900
catgtcgtcc tgtgtgtcag ctcactgaag attgatttac ttatggattt tttaaatgaa 960
ttctatgctc atcctaggct ccaggattat tatgtggtga ttttgtgtcc tactgaaatg 1020
gatgtacagg ttcgaagggt actgcagatt ccaatgtggt cccaacgagt tatctacctt 1080
caaggttcag cccttaaaga tcaagaccta ttgagagcaa agatggatga cgctgaggcc 1140
tgttttattc tcagtagccg ttgtgaagtg gataggacat catctgatca ccaaacaatt 1200
ttgagagcat gggctgtgaa agattttgct ccaaattgtc ctttgtatgt ccagatatta 1260
aagcctgaaa ataaatttca catcaaattt gctgatcatg ttgtttgtga agaagagttt 1320
aaatacgcca tgttagcttt aaactgtata tgcccagcaa catctacact tattacacta 1380
ctggttcata cctctagagg gcagtgtgtg tgcctgtgtt gcagagaagg ccagcaatcg 1440
ccagaacaat ggcagaagat gtacggtaga tgctccggga atgaagtcta ccacattgtt 1500
ttggaagaaa gtacattttt tgctgaatat gaaggaaaga gttttacata tgcctctttc 1560
catgcacaca aaaagtttgg cgtctgcttg attggtgtta ggagggagga taataaaaac 1620
attttgctga atccaggtcc tcgatacatt atgaattcta cagacatatg cttttatatt 1680
aatattacca aagaagagaa ttcagcattt aaaaaccaag accagcagag aaaaagcaat 1740
gtgtccaggt cgttttatca tggaccttcc agattacctg tacatagcat aattgccagc 1800
atgggtactg tggctataga cttgcaagat acaagctgta gatcagcaag tggccctacc 1860
ctgtctcttc ctacagaggg aagcaaagaa ataagaagac ctagcattgc tcctgtttta 1920
gaggttgcag atacatcatc gattcaaaca tgtgatcttc taagtgacca atcagaagat 1980
gaaactacac cagatgaaga aatgtcttca aacttagagt atgctaaagg ttacccacct 2040
tattctccat atataggaag ttcacccact ttttgtcatc tccttcatga aaaagtacca 2100
ttttgctgct taagattaga caagagttgc caacataact actatgagga tgcaaaagcc 2160
tatggattca aaaataaact aattatagtt gcagctgaaa cagctggaaa tggattatat 2220
aactttattg ttcctctcag ggcatattat agaccaaaga aagaacttaa tcccatagta 2280

ctgctattgg ataaccccct agatgactta ctcaggtgtg gagtgacttt tgctgctaat 2340
atggtggttg tggataaaga gagcaccatg agtgccgagg aagactacat ggcagatgcc 2400
aaaaccattg tgaacgtgca gacactcttc aggttgtttt ccagtctcag tattatcaca 2460
gagctaactc accccgccaa catgagattc atgcaattca gagccaaaga ctgttactct 2520
cttgctcttt caaaactgga aaagaaagaa cgggagagag gctctaactt ggcctttatg 2580
tttcgactgc cttttgctgc tgggagggtg tttagcatca gtatgttgga cactctgctg 2640
tatcagtcat ttgtgaagga ttatatgatt tctatcacga gacttctgtt gggactggac 2700
actacaccag gatctgggtt tctttgttct atgaaaatca ctgcagatga cttatggatc 2760
agaacttatg ccagacttta tcagaagttg tgttcttcta ctggagatgt tcccattgga 2820
atctacagga ctgagtctca gaaacttact acatctgagt ctcaaatatc tatcagtgta 2880
gaagagtggg aagacaccaa agactccaaa gaacaggggc accaccgcag caaccaccgc 2940
aactcaacat ccagtgacca gtcggaccat cccttgctgc ggagaaaaag catgcagtgg 3000
gcccgaagac tgagcagaaa aggcccaaaa cactctggta aaacagctga aaaaataacc 3060
cagcagcgac tgaacctcta caggaggtca gaaagacaag agcttgctga acttgtgaaa 3120
aatagaatga aacacttggg tctttctaca gtgggatatg atgaaatgaa tgatcatcaa 3180
agtaccctct cctacatcct gattaaccca tctccagata ccagaataga gctgaatgat 3240
gttgtatact taattcgacc agatccactg gcctaccttc caaacagtga gcccagtcga 3300
agaaacagca tctgcaatgt cactggtcaa gattctcggg aggaaactca actt 3354

<210> 3
<211> 24
<212> DNA
<213> Artificial Sequence
<220>
<223> Primer
<400> 3
ccttcccctt ctcccatccc tctc 24

<210> 4

<211> 26

<212> DNA

<213> Artificial Sequence

<220>

<223> Primer

<400> 4

ttccatctag tttctttcgt gccagc 26

<210> 5

<211> 26

<212> DNA

<213> Artificial Sequence

<220>

<223> Primer

<400> 5

ctcggccaca gcgtcttgtt agtcct 26

<210> 6

<211> 26

<212> DNA

<213> Artificial Sequence

<220>

<223> Primer

<400> 6

gtggtttcaa gcaaggtctt tgtagg 26

<210> 7

<211> 3517

<212> DNA

<213> Homo sapiens

<400> 7

ctcggccaca gcgtcttgtt agtcctctcc ctgtactccg caatattttc tttctttctc 60

cctcctctcc tccatttgtt gtttgatgtt tcccactctt tgaggaagga tggttgattt 120

ggagagcgaa gtgccccctc tgcctcccag gtacaggttt cgagatttgc tgctagggga 180

ccaaggatgg caaaacgacg acagggtaca agttgaattc tatatgaatg aaaatacatt 240

taaagaaaga ctaaaattat ttttcataaa aaaccagaga tcaagtctaa ggatacgcct 300

gttcaatttt tctctcaaat tactaagctg cttattatac ataatccgag tactactaga 360

aaacccttca caaggaaatg aatggtctca tatcttttgg gtgaacagaa gtctaccttt 420

gtggggctta caggtttcag tggcattgat aagtctgttt gaaacaatat tacttggtta 480

tcttagttat aagggaaaca tctgggaaca gattttacga atacccttca tcttggaaat 540

aattaatgca gttcccttca ttatctcaat attctggcct tccttaagga atctatttgt 600

cccagtcttt ctgaactgtt ggcttgccaa acatgccttg gaaaatatga ttaatgatct 660

acacagagcc attcagcgta cacagtctgc aatgtttaat caagttttga ttttaatatc 720

tacattacta tgccttatct tcacctgcat ttgtgggatc caacatctgg aacgaatagg 780

aaagaagctg aatctctttg actcccttta tttctgcatt gtgacgtttt ctactgtggg 840

cttcggggat gtcactcctg aaacatggtc ctccaagctt tttgtagttg ctatgatttg 900

tgttgctctt gtggttctac ccatacagtt tgaacagctg gcttatttgt ggatggagag 960

acaaaagtca ggaggaaact atagtcgaca tagagctcaa actgaaaagc atgtcgtcct 1020

gtgtgtcagc tcactgaaga ttgatttact tatggatttt ttaaatgaat tctatgctca 1080

tcctaggctc caggattatt atgtggtgat tttgtgtcct actgaaatgg atgtacaggt 1140

tcgaagggta ctgcagattc caatgtggtc ccaacgagtt atctaccttc aaggttcagc 1200

ccttaaagat caagacctat tgagagcaaa gatggatgac gctgaggcct gttttattct 1260

cagtagccgt tgtgaagtgg ataggacatc atctgatcac caaacaattt tgagagcatg 1320

ggctgtgaaa gattttgctc caaattgtcc tttgtatgtc cagatattaa agcctgaaaa 1380

taaatttcac atcaaatttg ctgatcatgt tgtttgtgaa gaagagttta aatacgccat 1440

gttagcttta aactgtatat gcccagcaac atctacactt attacactac tggttcatac 1500

ctctagaggg cagtgtgtgt gcctgtgttg cagagaaggc cagcaatcgc cagaacaatg 1560

gcagaagatg tacggtagat gctccgggaa tgaagtctac cacattgttt tggaagaaag 1620

tacatttttt gctgaatatg aaggaaagag ttttacatat gcctctttcc atgcacacaa 1680
aaagtttggc gtctgcttga ttggtgttag gagggaggat aataaaaaca ttttgctgaa 1740
tccaggtcct cgatacatta tgaattctac agacatatgc ttttatatta atattaccaa 1800
agaagagaat tcagcattta aaaaccaaga ccagcagaga aaaagcaatg tgtccaggtc 1860
gttttatcat ggaccttcca gattacctgt acatagcata attgccagca tgggtactgt 1920
ggctatagac ttgcaagata caagctgtag atcagcaagt ggccctaccc tgtctcttcc 1980
tacagaggga agcaaagaaa taagaagacc tagcattgct cctgttttag aggttgcaga 2040
tacatcatcg attcaaacat gtgatcttct aagtgaccaa tcagaagatg aaactacacc 2100
agatgaagaa atgtcttcaa acttagagta tgctaaaggt tacccacctt attctccata 2160
tataggaagt tcacccactt tttgtcatct ccttcatgaa aaagtaccat tttgctgctt 2220
aagattagac aagagttgcc aacataacta ctatgaggat gcaaaagcct atggattcaa 2280
aaataaacta attatagttg cagctgaaac agctggaaat ggattatata actttattgt 2340
tcctctcagg gcatattata gaccaaagaa agaacttaat cccatagtac tgctattgga 2400
taacccccta gatgacttac tcaggtgtgg agtgactttt gctgctaata tggtggttgt 2460
ggataaagag agcaccatga gtgccgagga agactacatg gcagatgcca aaaccattgt 2520
gaacgtgcag acactcttca ggttgttttc cagtctcagt attatcacag agctaactca 2580
ccccgccaac atgagattca tgcaattcag agccaaagac tgttactctc ttgctctttc 2640
aaaactggaa aagaaagaac gggagagagg ctctaacttg gcctttatgt ttcgactgcc 2700
ttttgctgct gggagggtgt ttagcatcag tatgttggac actctgctgt atcagtcatt 2760
tgtgaaggat tatatgattt ctatcacgag acttctgttg ggactggaca ctacaccagg 2820
atctgggttt ctttgttcta tgaaaatcac tgcagatgac ttatggatca gaacttatgc 2880
cagactttat cagaagttgt gttcttctac tggagatgtt cccattggaa tctacaggac 2940
tgagtctcag aaacttacta catctgagtc tcaaatatct atcagtgtag aagagtggga 3000
agacaccaaa gactccaaag aacaggggca ccaccgcagc aaccaccgca actcaacatc 3060
cagtgaccag tcggaccatc ccttgctgcg gagaaaaagc atgcagtggg cccgaagact 3120
gagcagaaaa ggccccaaac actctggtaa aacagctgaa aaaataaccc agcagcgact 3180
gaacctctac aggaggtcag aaagacaaga gcttgctgaa cttgtgaaaa atagaatgaa 3240
acacttgggt ctttctacag tgggatatga tgaaatgaat gatcatcaaa gtaccctctc 3300
ctacatcctg attaacccat ctccagatac cagaatagag ctgaatgatg ttgtatactt 3360

aattcgacca gatccactgg cctaccttcc aaacagtgag cccagtcgaa gaaacagcat 3420
ctgcaatgtc actggtcaag attctcggga ggaaactcaa ctttgataaa aataaaatga 3480
gaaacttttt tcctacaaag accttgcttg aaaccac 3517

<210> 8
<211> 1111
<212> PRT
<213> Homo sapiens
<400> 8
Met Val Asp Leu Glu Ser Glu Val Pro Pro Leu Pro Pro Arg Tyr Arg
                5                   10                  15
Phe Arg Asp Leu Leu Leu Gly Asp Gln Gly Trp Gln Asn Asp Asp Arg
            20                  25                  30
Val Gln Val Glu Phe Tyr Met Asn Glu Asn Thr Phe Lys Glu Arg Leu
        35                  40                  45
Lys Leu Phe Phe Ile Lys Asn Gln Arg Ser Ser Leu Arg Ile Arg Leu
    50                  55                  60
Phe Asn Phe Ser Leu Lys Leu Leu Ser Cys Leu Leu Tyr Ile Ile Arg
65                  70                  75                  80
Val Leu Leu Glu Asn Pro Ser Gln Gly Asn Glu Trp Ser His Ile Phe
                85                  90                  95
Trp Val Asn Arg Ser Leu Pro Leu Trp Gly Leu Gln Val Ser Val Ala
            100                 105                 110
Leu Ile Ser Leu Phe Glu Thr Ile Leu Leu Gly Tyr Leu Ser Tyr Lys
        115                 120                 125
Gly Asn Ile Trp Glu Gln Ile Leu Arg Ile Pro Phe Ile Leu Glu Ile
    130                 135                 140
Ile Asn Ala Val Pro Phe Ile Ile Ser Ile Phe Trp Pro Ser Leu Arg
145                 150                 155                 160

Asn Leu Phe Val Pro Val Phe Leu Asn Cys Trp Leu Ala Lys His Ala
                165                 170                 175
Leu Glu Asn Met Ile Asn Asp Leu His Arg Ala Ile Gln Arg Thr Gln
            180                 185                 190
Ser Ala Met Phe Asn Gln Val Leu Ile Leu Ile Ser Thr Leu Leu Cys
        195                 200                 205
Leu Ile Phe Thr Cys Ile Cys Gly Ile Gln His Leu Glu Arg Ile Gly
    210                 215                 220
Lys Lys Leu Asn Leu Phe Asp Ser Leu Tyr Phe Cys Ile Val Thr Phe
225                 230                 235                 240
Ser Thr Val Gly Phe Gly Asp Val Thr Pro Glu Thr Trp Ser Ser Lys
                245                 250                 255
Leu Phe Val Val Ala Met Ile Cys Val Ala Leu Val Val Leu Pro Ile
            260                 265                 270
Gln Phe Glu Gln Leu Ala Tyr Leu Trp Met Glu Arg Gln Lys Ser Gly
        275                 280                 285
Gly Asn Tyr Ser Arg His Arg Ala Gln Thr Glu Lys His Val Val Leu
    290                 295                 300
Cys Val Ser Ser Leu Lys Ile Asp Leu Leu Met Asp Phe Leu Asn Glu
305                 310                 315                 320
Phe Tyr Ala His Pro Arg Leu Gln Asp Tyr Tyr Val Val Ile Leu Cys
                325                 330                 335
Pro Thr Glu Met Asp Val Gln Val Arg Arg Val Leu Gln Ile Pro Met
            340                 345                 350
Trp Ser Gln Arg Val Ile Tyr Leu Gln Gly Ser Ala Leu Lys Asp Gln
        355                 360                 365
Asp Leu Leu Arg Ala Lys Met Asp Asp Ala Glu Ala Cys Phe Ile Leu
    370                 375                 380
Ser Ser Arg Cys Glu Val Asp Arg Thr Ser Ser Asp His Gln Thr Ile

385                 390                 395                 400

Leu Arg Ala Trp Ala Val Lys Asp Phe Ala Pro Asn Cys Pro Leu Tyr

                405                 410                 415

Val Gln Ile Leu Lys Pro Glu Asn Lys Phe His Ile Lys Phe Ala Asp

            420                 425                 430

His Val Val Cys Glu Glu Glu Phe Lys Tyr Ala Met Leu Ala Leu Asn

        435                 440                 445

Cys Ile Cys Pro Ala Thr Ser Thr Leu Ile Thr Leu Leu Val His Thr

    450                 455                 460

Ser Arg Gly Gln Glu Gly Gln Gln Ser Pro Glu Gln Trp Gln Lys Met

465                 470                 475                 480

Tyr Gly Arg Cys Ser Gly Asn Glu Val Tyr His Ile Val Leu Glu Glu

                485                 490                 495

Ser Thr Phe Phe Ala Glu Tyr Glu Gly Lys Ser Phe Thr Tyr Ala Ser

            500                 505                 510

Phe His Ala His Lys Lys Phe Gly Val Cys Leu Ile Gly Val Arg Arg

        515                 520                 525

Glu Asp Asn Lys Asn Ile Leu Leu Asn Pro Gly Pro Arg Tyr Ile Met

    530                 535                 540

Asn Ser Thr Asp Ile Cys Phe Tyr Ile Asn Ile Thr Lys Glu Glu Asn

545                 550                 555                 560

Ser Ala Phe Lys Asn Gln Asp Gln Gln Arg Lys Ser Asn Val Ser Arg

                565                 570                 575

Ser Phe Tyr His Gly Pro Ser Arg Leu Pro Val His Ser Ile Ile Ala

            580                 585                 590

Ser Met Gly Thr Val Ala Ile Asp Leu Gln Asp Thr Ser Cys Arg Ser

        595                 600                 605

Ala Ser Gly Pro Thr Leu Ser Leu Pro Thr Glu Gly Ser Lys Glu Ile

    610                 615                 620

Arg Arg Pro Ser Ile Ala Pro Val Leu Glu Val Ala Asp Thr Ser Ser
625                 630                 635                 640
Ile Gln Thr Cys Asp Leu Leu Ser Asp Gln Ser Glu Asp Glu Thr Thr
                645                 650                 655
Pro Asp Glu Glu Met Ser Ser Asn Leu Glu Tyr Ala Lys Gly Tyr Pro
            660                 665                 670
Pro Tyr Ser Pro Tyr Ile Gly Ser Ser Pro Thr Phe Cys His Leu Leu
        675                 680                 685
His Glu Lys Val Pro Phe Cys Cys Leu Arg Leu Asp Lys Ser Cys Gln
    690                 695                 700
His Asn Tyr Tyr Glu Asp Ala Lys Ala Tyr Gly Phe Lys Asn Lys Leu
705                 710                 715                 720
Ile Ile Val Ala Ala Glu Thr Ala Gly Asn Gly Leu Tyr Asn Phe Ile
                725                 730                 735
Val Pro Leu Arg Ala Tyr Tyr Arg Pro Lys Lys Glu Leu Asn Pro Ile
            740                 745                 750
Val Leu Leu Leu Asp Asn Pro Leu Asp Asp Leu Leu Arg Cys Gly Val
        755                 760                 765
Thr Phe Ala Ala Asn Met Val Val Val Asp Lys Glu Ser Thr Met Ser
    770                 775                 780
Ala Glu Glu Asp Tyr Met Ala Asp Ala Lys Thr Ile Val Asn Val Gln
785                 790                 795                 800
Thr Leu Phe Arg Leu Phe Ser Ser Leu Ser Ile Ile Thr Glu Leu Thr
                805                 810                 815
His Pro Ala Asn Met Arg Phe Met Gln Phe Arg Ala Lys Asp Cys Tyr
            820                 825                 830
Ser Leu Ala Leu Ser Lys Leu Glu Lys Lys Glu Arg Glu Arg Gly Ser
        835                 840                 845
Asn Leu Ala Phe Met Phe Arg Leu Pro Phe Ala Ala Gly Arg Val Phe

    850                 855                 860

Ser Ile Ser Met Leu Asp Thr Leu Leu Tyr Gln Ser Phe Val Lys Asp

865                 870                 875                 880

Tyr Met Ile Ser Ile Thr Arg Leu Leu Leu Gly Leu Asp Thr Thr Pro

                885                 890                 895

Gly Ser Gly Phe Leu Cys Ser Met Lys Ile Thr Ala Asp Asp Leu Trp

            900                 905                 910

Ile Arg Thr Tyr Ala Arg Leu Tyr Gln Lys Leu Cys Ser Ser Thr Gly

        915                 920                 925

Asp Val Pro Ile Gly Ile Tyr Arg Thr Glu Ser Gln Lys Leu Thr Thr

    930                 935                 940

Ser Glu Ser Gln Ile Ser Ile Ser Val Glu Glu Trp Glu Asp Thr Lys

945                 950                 955                 960

Asp Ser Lys Glu Gln Gly His His Arg Ser Asn His Arg Asn Ser Thr

                965                 970                 975

Ser Ser Asp Gln Ser Asp His Pro Leu Leu Arg Arg Lys Ser Met Gln

            980                 985                 990

Trp Ala Arg Arg Leu Ser Arg Lys Gly Pro Lys His Ser Gly Lys Thr

        995                 1000                1005

Ala Glu Lys Ile Thr Gln Gln Arg Leu Asn Leu Tyr Arg Arg Ser Glu

    1010                1015                1020

Arg Gln Glu Leu Ala Glu Leu Val Lys Asn Arg Met Lys His Leu Gly

1025                1030                1035                1040

Leu Ser Thr Val Gly Tyr Asp Glu Met Asn Asp His Gln Ser Thr Leu

                1045                1050                1055

Ser Tyr Ile Leu Ile Asn Pro Ser Pro Asp Thr Arg Ile Glu Leu Asn

            1060                1065                1070

Asp Val Val Tyr Leu Ile Arg Pro Asp Pro Leu Ala Tyr Leu Pro Asn

        1075                1080                1085

Ser Glu Pro Ser Arg Arg Asn Ser Ile Cys Asn Val Thr Gly Gln Asp
    1090                1095                1100
Ser Arg Glu Glu Thr Gln Leu
1105                1110

<210> 9
<211> 3333
<212> DNA
<213> Homo sapiens
<400> 9
atggttgatt tggagagcga agtgccccct ctgcctccca ggtacaggtt tcgagatttg 60
ctgctagggg accaaggatg gcaaaacgac gacagggtac aagttgaatt ctatatgaat 120
gaaaatacat ttaaagaaag actaaaatta tttttcataa aaaaccagag atcaagtcta 180
aggatacgcc tgttcaattt ttctctcaaa ttactaagct gcttattata cataatccga 240
gtactactag aaaacccttc acaaggaaat gaatggtctc atatcttttg ggtgaacaga 300
agtctacctt tgtggggctt acaggtttca gtggcattga taagtctgtt tgaaacaata 360
ttacttggtt atcttagtta taagggaaac atctgggaac agattttacg aatacccttc 420
atcttggaaa taattaatgc agttcccttc attatctcaa tattctggcc ttccttaagg 480
aatctatttg tcccagtctt tctgaactgt tggcttgcca aacatgcctt ggaaaatatg 540
attaatgatc tacacagagc cattcagcgt acacagtctg caatgtttaa tcaagttttg 600
attttaatat ctacattact atgccttatc ttcacctgca tttgtgggat ccaacatctg 660
gaacgaatag gaaagaagct gaatctcttt gactcccttt atttctgcat tgtgacgttt 720
tctactgtgg gcttcgggga tgtcactcct gaaacatggt cctccaagct ttttgtagtt 780
gctatgattt gtgttgctct tgtggttcta cccatacagt ttgaacagct ggcttatttg 840
tggatggaga gacaaaagtc aggaggaaac tatagtcgac atagagctca aactgaaaag 900
catgtcgtcc tgtgtgtcag ctcactgaag attgatttac ttatggattt tttaaatgaa 960
ttctatgctc atcctaggct ccaggattat tatgtggtga ttttgtgtcc tactgaaatg 1020
gatgtacagg ttcgaagggt actgcagatt ccaatgtggt cccaacgagt tatctacctt 1080
caaggttcag cccttaaaga tcaagaccta ttgagagcaa agatggatga cgctgaggcc 1140

tgttttattc tcagtagccg ttgtgaagtg gataggacat catctgatca ccaaacaatt 1200

ttgagagcat gggctgtgaa agattttgct ccaaattgtc ctttgtatgt ccagatatta 1260

aagcctgaaa ataaatttca catcaaattt gctgatcatg ttgtttgtga agaagagttt 1320

aaatacgcca tgttagcttt aaactgtata tgcccagcaa catctacact tattacacta 1380

ctggttcata cctctagagg gcaagaaggc cagcaatcgc cagaacaatg gcagaagatg 1440

tacggtagat gctccgggaa tgaagtctac cacattgttt tggaagaaag tacatttttt 1500

gctgaatatg aaggaaagag ttttacatat gcctctttcc atgcacacaa aaagtttggc 1560

gtctgcttga ttggtgttag gagggaggat aataaaaaca ttttgctgaa tccaggtcct 1620

cgatacatta tgaattctac agacatatgc ttttatatta atattaccaa agaagagaat 1680

tcagcattta aaaaccaaga ccagcagaga aaaagcaatg tgtccaggtc gttttatcat 1740

ggaccttcca gattacctgt acatagcata attgccagca tgggtactgt ggctatagac 1800

ttgcaagata caagctgtag atcagcaagt ggccctaccc tgtctcttcc tacagaggga 1860

agcaaagaaa taagaagacc tagcattgct cctgttttag aggttgcaga tacatcatcg 1920

attcaaacat gtgatcttct aagtgaccaa tcagaagatg aaactacacc agatgaagaa 1980

atgtcttcaa acttagagta tgctaaaggt tacccacctt attctccata tataggaagt 2040

tcacccactt tttgtcatct ccttcatgaa aaagtaccat tttgctgctt aagattagac 2100

aagagttgcc aacataacta ctatgaggat gcaaaagcct atggattcaa aaataaacta 2160

attatagttg cagctgaaac agctggaaat ggattatata actttattgt tcctctcagg 2220

gcatattata gaccaaagaa agaacttaat cccatagtac tgctattgga taacccccta 2280

gatgacttac tcaggtgtgg agtgactttt gctgctaata tggtggttgt ggataaagag 2340

agcaccatga gtgccgagga agactacatg gcagatgcca aaaccattgt gaacgtgcag 2400

acactcttca ggttgttttc cagtctcagt attatcacag agctaactca ccccgccaac 2460

atgagattca tgcaattcag agccaaagac tgttactctc ttgctctttc aaaactggaa 2520

aagaaagaac gggagagagg ctctaacttg gcctttatgt ttcgactgcc ttttgctgct 2580

gggagggtgt ttagcatcag tatgttggac actctgctgt atcagtcatt tgtgaaggat 2640

tatatgattt ctatcacgag acttctgttg ggactggaca ctacaccagg atctgggttt 2700

ctttgttcta tgaaaatcac tgcagatgac ttatggatca gaacttatgc cagactttat 2760

cagaagttgt gttcttctac tggagatgtt cccattggaa tctacaggac tgagtctcag 2820

aaacttacta catctgagtc tcaaatatct atcagtgtag aagagtggga agacaccaaa 2880

gactccaaag aacaagggca ccaccgcagc aaccaccgca actcaacatc cagtgaccag 2940
tcggaccatc ccttgctgcg gagaaaaagc atgcagtggg cccgaagact gagcagaaaa 3000
ggcccaaaac actctggtaa aacagctgaa aaaataaccc agcagcgact gaacctctac 3060
aggaggtcag aaagacaaga gcttgctgaa cttgtgaaaa atagaatgaa acacttgggt 3120
ctttctacag tgggatatga tgaaatgaat gatcatcaaa gtaccctctc ctacatcctg 3180
attaacccat ctccagatac cagaatagag ctgaatgatg ttgtatactt aattcgacca 3240
gatccactgg cctaccttcc aaacagtgag cccagtcgaa gaaacagcat ctgcaatgtc 3300
actggtcaag attctcggga ggaaactcaa ctt 3333

<210> 10
<211> 3496
<212> DNA
<213> Homo sapiens
<400> 10
   ctcggccaca gcgtcttgtt agtcctctcc ctgtactccg caatattttc tttctttctc 60
   cctcctctcc tccatttgtt gtttgatgtt tcccactctt tgaggaagga tggttgattt 120
   ggagagcgaa gtgccccctc tgcctcccag gtacaggttt cgagatttgc tgctagggga 180
   ccaaggatgg caaaacgacg acagggtaca agttgaattc tatatgaatg aaaatacatt 240
   taaagaaaga ctaaaattat ttttcataaa aaaccagaga tcaagtctaa ggatacgcct 300
   gttcaatttt tctctcaaat tactaagctg cttattatac ataatccgag tactactaga 360
   aaacccttca caaggaaatg aatggtctca tatcttttgg gtgaacagaa gtctaccttt 420
   gtggggctta caggtttcag tggcattgat aagtctgttt gaaacaatat tacttggtta 480
   tcttagttat aagggaaaca tctgggaaca gattttacga atacccttca tcttggaaat 540
   aattaatgca gttcccttca ttatctcaat attctggcct tccttaagga atctatttgt 600
   cccagtcttt ctgaactgtt ggcttgccaa acatgccttg gaaaatatga ttaatgatct 660
   acacagagcc attcagcgta cacagtctgc aatgtttaat caagttttga ttttaatatc 720
   tacattacta tgccttatct tcacctgcat ttgtgggatc caacatctgg aacgaatagg 780
   aaagaagctg aatctctttg actcccttta tttctgcatt gtgacgtttt ctactgtggg 840
   cttcggggat gtcactcctg aaacatggtc ctccaagctt tttgtagttg ctatgatttg 900
   tgttgctctt gtggttctac ccatacagtt tgaacagctg gcttatttgt ggatggagag 960
   acaaaagtca ggaggaaact atagtcgaca tagagctcaa actgaaaagc atgtcgtcct 1020
   gtgtgtcagc tcactgaaga ttgatttact tatggatttt ttaaatgaat tctatgctca 1080
   tcctaggctc caggattatt atgtggtgat tttgtgtcct actgaaatgg atgtacaggt 1140
   tcgaagggta ctgcagattc caatgtggtc ccaacgagtt atctaccttc aaggttcagc 1200
   ccttaaagat caagacctat tgagagcaaa gatggatgac gctgaggcct gttttattct 1260
   cagtagccgt tgtgaagtgg ataggacatc atctgatcac caaacaattt tgagagcatg 1320
   ggctgtgaaa gattttgctc caaattgtcc tttgtatgtc cagatattaa agcctgaaaa 1380
   taaatttcac atcaaatttg ctgatcatgt tgtttgtgaa gaagagttta aatacgccat 1440
   gttagcttta aactgtatat gcccagcaac atctacactt attacactac tggttcatac 1500
   ctctagaggg caagaaggcc agcaatcgcc agaacaatgg cagaagatgt acggtagatg 1560
   ctccgggaat gaagtctacc acattgtttt ggaagaaagt acattttttg ctgaatatga 1620
   aggaaagagt tttacatatg cctctttcca tgcacacaaa aagtttggcg tctgcttgat 1680
   tggtgttagg agggaggata ataaaaacat tttgctgaat ccaggtcctc gatacattat 1740
   gaattctaca gacatatgct tttatattaa tattaccaaa gaagagaatt cagcatttaa 1800
   aaaccaagac cagcagagaa aaagcaatgt gtccaggtcg ttttatcatg gaccttccag 1860
   attacctgta catagcataa ttgccagcat gggtactgtg gctatagact tgcaagatac 1920
   aagctgtaga tcagcaagtg gccctaccct gtctcttcct acagagggaa gcaaagaaat 1980
   aagaagacct agcattgctc ctgttttaga ggttgcagat acatcatcga ttcaaacatg 2040
   tgatcttcta agtgaccaat cagaagatga aactacacca gatgaagaaa tgtcttcaaa 2100
   cttagagtat gctaaaggtt acccacctta ttctccatat ataggaagtt cacccacttt 2160
   ttgtcatctc cttcatgaaa aagtaccatt ttgctgctta agattagaca agagttgcca 2220
   acataactac tatgaggatg caaaagccta tggattcaaa aataaactaa ttatagttgc 2280
   agctgaaaca gctggaaatg gattatataa ctttattgtt cctctcaggg catattatag 2340
   accaaagaaa gaacttaatc ccatagtact gctattggat aaccccctag atgacttact 2400
   caggtgtgga gtgacttttg ctgctaatat ggtggttgtg gataaagaga gcaccatgag 2460
   tgccgaggaa gactacatgg cagatgccaa aaccattgtg aacgtgcaga cactcttcag 2520
   gttgttttcc agtctcagta ttatcacaga gctaactcac cccgccaaca tgagattcat 2580
   gcaattcaga gccaaagact gttactctct tgctctttca aaactggaaa agaaagaacg 2640
   ggagagaggc tctaacttgg cctttatgtt tcgactgcct tttgctgctg ggagggtgtt 2700
   tagcatcagt atgttggaca ctctgctgta tcagtcattt gtgaaggatt atatgatttc 2760
   tatcacgaga cttctgttgg gactggacac tacaccagga tctgggtttc tttgttctat 2820
   gaaaatcact gcagatgact tatggatcag aacttatgcc agactttatc agaagttgtg 2880
   ttcttctact ggagatgttc ccattggaat ctacaggact gagtctcaga aacttactac 2940
   atctgagtct caaatatcta tcagtgtaga agagtgggaa gacaccaaag actccaaaga 3000
   acaagggcac caccgcagca accaccgcaa ctcaacatcc agtgaccagt cggaccatcc 3060
   cttgctgcgg agaaaaagca tgcagtgggc ccgaagactg agcagaaaag gcccaaaaca 3120
   ctctggtaaa acagctgaaa aaataaccca gcagcgactg aacctctaca ggaggtcaga 3180
   aagacaagag cttgctgaac ttgtgaaaaa tagaatgaaa cacttgggtc tttctacagt 3240
   gggatatgat gaaatgaatg atcatcaaag taccctctcc tacatcctga ttaacccatc 3300
   tccagatacc agaatagagc tgaatgatgt tgtatactta attcgaccag atccactggc 3360
   ctaccttcca aacagtgagc ccagtcgaag aaacagcatc tgcaatgtca ctggtcaaga 3420
   ttctcgggag gaaactcaac tttgataaaa ataaaatgag aaactttttt cctacaaaga 3480
   ccttgcttga aaccac 3496

<210> 11
<211> 1061
<212> PRT
<213> Homo sapiens
<400> 11
Met Val Asp Leu Glu Ser Glu Val Pro Pro Leu Pro Pro Arg Tyr Arg
                5                   10                  15
Phe Arg Asp Leu Leu Leu Gly Asp Gln Gly Trp Gln Asn Asp Asp Arg
            20                  25                  30
Val Gln Val Glu Phe Tyr Met Asn Glu Asn Thr Phe Lys Glu Arg Leu
        35                  40                  45
Lys Leu Phe Phe Ile Lys Asn Gln Arg Ser Ser Leu Arg Ile Arg Leu
    50                  55                  60

Phe Asn Phe Ser Leu Lys Leu Leu Ser Cys Leu Leu Tyr Ile Ile Arg
65                  70                  75                  80
Val Leu Leu Glu Asn Pro Ser Gln Gly Asn Glu Trp Ser His Ile Phe
                85                  90                  95
Trp Val Asn Arg Ser Leu Pro Leu Trp Gly Leu Gln Val Ser Val Ala
            100                 105                 110
Leu Ile Ser Leu Phe Glu Thr Ile Leu Leu Gly Tyr Leu Ser Tyr Lys
        115                 120                 125
Gly Asn Ile Trp Glu Gln Ile Leu Arg Ile Pro Phe Ile Leu Glu Ile
    130                 135                 140
Ile Asn Ala Val Pro Phe Ile Ile Ser Ile Phe Trp Pro Ser Leu Arg
145                 150                 155                 160
Asn Leu Phe Val Pro Val Phe Leu Asn Cys Trp Leu Ala Lys His Ala
                165                 170                 175
Leu Glu Asn Met Ile Asn Asp Leu His Arg Ala Ile Gln Arg Thr Gln
            180                 185                 190
Ser Ala Met Phe Asn Gln Val Leu Ile Leu Ile Ser Thr Leu Leu Cys
        195                 200                 205
Leu Ile Phe Thr Cys Ile Cys Gly Ile Gln His Leu Glu Arg Ile Gly
    210                 215                 220
Lys Lys Leu Asn Leu Phe Asp Ser Leu Tyr Phe Cys Ile Val Thr Phe
225                 230                 235                 240
Ser Thr Val Gly Phe Gly Asp Val Thr Pro Glu Thr Trp Ser Ser Lys
                245                 250                 255
Leu Phe Val Val Ala Met Ile Cys Val Ala Leu Val Val Leu Pro Ile
            260                 265                 270
Gln Phe Glu Gln Leu Ala Tyr Leu Trp Met Glu Arg Gln Lys Ser Gly
        275                 280                 285
Gly Asn Tyr Ser Arg His Arg Ala Gln Thr Glu Lys His Val Val Leu

290                 295                 300
Cys Val Ser Ser Leu Lys Ile Asp Leu Leu Met Asp Phe Leu Asn Glu
305                 310                 315                 320
Phe Tyr Ala His Pro Arg Leu Gln Asp Tyr Tyr Val Val Ile Leu Cys
                325                 330                 335
Pro Thr Glu Met Asp Val Gln Val Arg Arg Val Leu Gln Ile Pro Met
            340                 345                 350
Trp Ser Gln Arg Val Ile Tyr Leu Gln Gly Ser Ala Leu Lys Asp Gln
        355                 360                 365
Asp Leu Leu Arg Ala Lys Met Asp Asp Ala Glu Ala Cys Phe Ile Leu
    370                 375                 380
Ser Ser Arg Cys Glu Val Asp Arg Thr Ser Ser Asp His Gln Thr Ile
385                 390                 395                 400
Leu Arg Ala Trp Ala Val Lys Asp Phe Ala Pro Asn Cys Pro Leu Tyr
                405                 410                 415
Val Gln Ile Leu Lys Pro Glu Asn Lys Phe His Ile Lys Phe Ala Asp
            420                 425                 430
His Val Val Cys Glu Glu Glu Phe Lys Tyr Ala Met Leu Ala Leu Asn
        435                 440                 445
Cys Ile Cys Pro Ala Thr Ser Thr Leu Ile Thr Leu Leu Val His Thr
    450                 455                 460
Ser Arg Gly Gln Phe Gly Val Cys Leu Ile Gly Val Arg Arg Glu Asp
465                 470                 475                 480
Asn Lys Asn Ile Leu Leu Asn Pro Gly Pro Arg Tyr Ile Met Asn Ser
                485                 490                 495
Thr Asp Ile Cys Phe Tyr Ile Asn Ile Thr Lys Glu Glu Asn Ser Ala
            500                 505                 510
Phe Lys Asn Gln Asp Gln Gln Arg Lys Ser Asn Val Ser Arg Ser Phe
        515                 520                 525

Tyr His Gly Pro Ser Arg Leu Pro Val His Ser Ile Ile Ala Ser Met
    530                 535                 540
Gly Thr Val Ala Ile Asp Leu Gln Asp Thr Ser Cys Arg Ser Ala Ser
545                 550                 555                 560
Gly Pro Thr Leu Ser Leu Pro Thr Glu Gly Ser Lys Glu Ile Arg Arg
                565                 570                 575
Pro Ser Ile Ala Pro Val Leu Glu Val Ala Asp Thr Ser Ser Ile Gln
            580                 585                 590
Thr Cys Asp Leu Leu Ser Asp Gln Ser Glu Asp Glu Thr Thr Pro Asp
        595                 600                 605
Glu Glu Met Ser Ser Asn Leu Glu Tyr Ala Lys Gly Tyr Pro Pro Tyr
    610                 615                 620
Ser Pro Tyr Ile Gly Ser Ser Pro Thr Phe Cys His Leu Leu His Glu
625                 630                 635                 640
Lys Val Pro Phe Cys Cys Leu Arg Leu Asp Lys Ser Cys Gln His Asn
                645                 650                 655
Tyr Tyr Glu Asp Ala Lys Ala Tyr Gly Phe Lys Asn Lys Leu Ile Ile
            660                 665                 670
Val Ala Ala Glu Thr Ala Gly Asn Gly Leu Tyr Asn Phe Ile Val Pro
        675                 680                 685
Leu Arg Ala Tyr Tyr Arg Pro Lys Lys Glu Leu Asn Pro Ile Val Leu
    690                 695                 700
Leu Leu Asp Asn Pro Leu Asp Asp Leu Leu Arg Cys Gly Val Thr Phe
705                 710                 715                 720
Ala Ala Asn Met Val Val Val Asp Lys Glu Ser Thr Met Ser Ala Glu
                725                 730                 735
Glu Asp Tyr Met Ala Asp Ala Lys Thr Ile Val Asn Val Gln Thr Leu
            740                 745                 750
Phe Arg Leu Phe Ser Ser Leu Ser Ile Ile Thr Glu Leu Thr His Pro

        755                 760                 765
Ala Asn Met Arg Phe Met Gln Phe Arg Ala Lys Asp Cys Tyr Ser Leu
    770                 775                 780
Ala Leu Ser Lys Leu Glu Lys Lys Glu Arg Glu Arg Gly Ser Asn Leu
785                 790                 795                 800
Ala Phe Met Phe Arg Leu Pro Phe Ala Ala Gly Arg Val Phe Ser Ile
                805                 810                 815
Ser Met Leu Asp Thr Leu Leu Tyr Gln Ser Phe Val Lys Asp Tyr Met
            820                 825                 830
Ile Ser Ile Thr Arg Leu Leu Leu Gly Leu Asp Thr Thr Pro Gly Ser
        835                 840                 845
Gly Phe Leu Cys Ser Met Lys Ile Thr Ala Asp Asp Leu Trp Ile Arg
    850                 855                 860
Thr Tyr Ala Arg Leu Tyr Gln Lys Leu Cys Ser Ser Thr Gly Asp Val
865                 870                 875                 880
Pro Ile Gly Ile Tyr Arg Thr Glu Ser Gln Lys Leu Thr Thr Ser Glu
                885                 890                 895
Ser Gln Ile Ser Ile Ser Val Glu Glu Trp Glu Asp Thr Lys Asp Ser
            900                 905                 910
Lys Glu Gln Gly His His Arg Ser Asn His Arg Asn Ser Thr Ser Ser
        915                 920                 925
Asp Gln Ser Asp His Pro Leu Leu Arg Arg Lys Ser Met Gln Trp Ala
    930                 935                 940
Arg Arg Leu Ser Arg Lys Gly Pro Lys His Ser Gly Lys Thr Ala Glu
945                 950                 955                 960
Lys Ile Thr Gln Gln Arg Leu Asn Leu Tyr Arg Arg Ser Glu Arg Gln
                965                 970                 975
Glu Leu Ala Glu Leu Val Lys Asn Arg Met Lys His Leu Gly Leu Ser
            980                 985                 990

Thr Val Gly Tyr Asp Glu Met Asn Asp His Gln Ser Thr Leu Ser Tyr
        995                 1000                1005
Ile Leu Ile Asn Pro Ser Pro Asp Thr Arg Ile Glu Leu Asn Asp Val
    1010                1015                1020
Val Tyr Leu Ile Arg Pro Asp Pro Leu Ala Tyr Leu Pro Asn Ser Glu
1025                1030                1035                1040
Pro Ser Arg Arg Asn Ser Ile Cys Asn Val Thr Gly Gln Asp Ser Arg
                1045                1050                1055
Glu Glu Thr Gln Leu
            1060

<210> 12
<211> 3183
<212> DNA
<213> Homo sapiens
<400> 12
atggttgatt tggagagcga agtgccccct ctgcctccca ggtacaggtt tcgagatttg 60
ctgctagggg accaaggatg gcaaaacgac gacagggtac aagttgaatt ctatatgaat 120
gaaaatacat ttaaagaaag actaaaatta tttttcataa aaaaccagag atcaagtcta 180
aggatacgcc tgttcaattt ttctctcaaa ttactaagct gcttattata cataatccga 240
gtactactag aaaacccttc acaaggaaat gaatggtctc atatcttttg ggtgaacaga 300
agtctacctt tgtggggctt acaggtttca gtggcattga taagtctgtt tgaaacaata 360
ttacttggtt atcttagtta taagggaaac atctgggaac agattttacg aatacccttc 420
atcttggaaa taattaatgc agttcccttc attatctcaa tattctggcc ttccttaagg 480
aatctatttg tcccagtctt tctgaactgt tggcttgcca aacatgcctt ggaaaatatg 540
attaatgatc tacacagagc cattcagcgt acacagtctg caatgtttaa tcaagttttg 600
attttaatat ctacattact atgccttatc ttcacctgca tttgtgggat ccaacatctg 660
gaacgaatag gaaagaagct gaatctcttt gactcccttt atttctgcat tgtgacgttt 720
tctactgtgg gcttcgggga tgtcactcct gaaacatggt cctccaagct ttttgtagtt 780

gctatgattt gtgttgctct tgtggttcta cccatacagt ttgaacagct ggcttatttg 840
tggatggaga gacaaaagtc aggaggaaac tatagtcgac atagagctca aactgaaaag 900
catgtcgtcc tgtgtgtcag ctcactgaag attgatttac ttatggattt tttaaatgaa 960
ttctatgctc atcctaggct ccaggattat tatgtggtga ttttgtgtcc tactgaaatg 1020
gatgtacagg ttcgaagggt actgcagatt ccaatgtggt cccaacgagt tatctacctt 1080
caaggttcag cccttaaaga tcaagaccta ttgagagcaa agatggatga cgctgaggcc 1140
tgttttattc tcagtagccg ttgtgaagtg gataggacat catctgatca ccaaacaatt 1200
ttgagagcat gggctgtgaa agattttgct ccaaattgtc ctttgtatgt ccagatatta 1260
aagcctgaaa ataaatttca catcaaattt gctgatcatg ttgtttgtga agaagagttt 1320
aaatacgcca tgttagcttt aaactgtata tgcccagcaa catctacact tattacacta 1380
ctggttcata cctctagagg gcagtttggc gtctgcttga ttggtgttag gagggaggat 1440
aataaaaaca ttttgctgaa tccaggtcct cgatacatta tgaattctac agacatatgc 1500
ttttatatta atattaccaa agaagagaat tcagcattta aaaaccaaga ccagcagaga 1560
aaaagcaatg tgtccaggtc gttttatcat ggaccttcca gattacctgt acatagcata 1620
attgccagca tgggtactgt ggctatagac ttgcaagata caagctgtag atcagcaagt 1680
ggccctaccc tgtctcttcc tacagaggga agcaaagaaa taagaagacc tagcattgct 1740
cctgttttag aggttgcaga tacatcatcg attcaaacat gtgatcttct aagtgaccaa 1800
tcagaagatg aaactacacc agatgaagaa atgtcttcaa acttagagta tgctaaaggt 1860
tacccacctt attctccata tataggaagt tcacccactt tttgtcatct ccttcatgaa 1920
aaagtaccat tttgctgctt aagattagac aagagttgcc aacataacta ctatgaggat 1980
gcaaaagcct atggattcaa aaataaacta attatagttg cagctgaaac agctggaaat 2040
ggattatata actttattgt tcctctcagg gcatattata gaccaaagaa agaacttaat 2100
cccatagtac tgctattgga taacccccta gatgacttac tcaggtgtgg agtgactttt 2160
gctgctaata tggtggttgt ggataaagag agcaccatga gtgccgagga agactacatg 2220
gcagatgcca aaaccattgt gaacgtgcag acactcttca ggttgttttc cagtctcagt 2280
attatcacag agctaactca ccccgccaac atgagattca tgcaattcag agccaaagac 2340
tgttactctc ttgctctttc aaaactggaa aagaaagaac gggagagagg ctctaacttg 2400
gcctttatgt ttcgactgcc ttttgctgct gggagggtgt ttagcatcag tatgttggac 2460
actctgctgt atcagtcatt tgtgaaggat tatatgattt ctatcacgag acttctgttg 2520

ggactggaca ctacaccagg atctgggttt ctttgttcta tgaaaatcac tgcagatgac 2580

ttatggatca gaacttatgc cagactttat cagaagttgt gttcttctac tggagatgtt 2640

cccattggaa tctacaggac tgagtctcag aaacttacta catctgagtc tcaaatatct 2700

atcagtgtag aagagtggga agacaccaaa gactccaaag aacaagggca ccaccgcagc 2760

aaccaccgca actcaacatc cagtgaccag tcggaccatc ccttgctgcg gagaaaaagc 2820

atgcagtggg cccgaagact gagcagaaaa ggcccaaaac actctggtaa aacagctgaa 2880

aaaataaccc agcagcgact gaacctctac aggaggtcag aaagacaaga gcttgctgaa 2940

cttgtgaaaa atagaatgaa acacttgggt ctttctacag tgggatatga tgaaatgaat 3000

gatcatcaaa gtaccctctc ctacatcctg attaacccat ctccagatac cagaatagag 3060

ctgaatgatg ttgtatactt aattcgacca gatccactgg cctaccttcc aaacagtgag 3120

cccagtcgaa gaaacagcat ctgcaatgtc actggtcaag attctcggga ggaaactcaa 3180

ctt 3183

<210> 13

<211> 3346

<212> DNA

<213> Homo sapiens

<400> 13

ctcggccaca gcgtcttgtt agtcctctcc ctgtactccg caatattttc tttctttctc 60

cctcctctcc tccatttgtt gtttgatgtt tcccactctt tgaggaagga tggttgattt 120

ggagagcgaa gtgccccctc tgcctcccag gtacaggttt cgagatttgc tgctagggga 180

ccaaggatgg caaaacgacg acagggtaca agttgaattc tatatgaatg aaaatacatt 240

taaagaaaga ctaaaattat ttttcataaa aaaccagaga tcaagtctaa ggatacgcct 300

gttcaatttt tctctcaaat tactaagctg cttattatac ataatccgag tactactaga 360

aaacccttca caaggaaatg aatggtctca tatcttttgg gtgaacagaa gtctaccttt 420

gtggggctta caggtttcag tggcattgat aagtctgttt gaaacaatat tacttggtta 480

tcttagttat aagggaaaca tctgggaaca gattttacga atacccttca tcttggaaat 540

aattaatgca gttcccttca ttatctcaat attctggcct tccttaagga atctatttgt 600

cccagtcttt ctgaactgtt ggcttgccaa acatgccttg gaaaatatga ttaatgatct 660

acacagagcc attcagcgta cacagtctgc aatgtttaat caagttttga ttttaatatc 720
tacattacta tgccttatct tcacctgcat ttgtgggatc caacatctgg aacgaatagg 780
aaagaagctg aatctctttg actcccttta tttctgcatt gtgacgtttt ctactgtggg 840
cttcggggat gtcactcctg aaacatggtc ctccaagctt tttgtagttg ctatgatttg 900
tgttgctctt gtggttctac ccatacagtt tgaacagctg gcttatttgt ggatggagag 960
acaaaagtca ggaggaaact atagtcgaca tagagctcaa actgaaaagc atgtcgtcct 1020
gtgtgtcagc tcactgaaga ttgatttact tatggatttt ttaaatgaat tctatgctca 1080
tcctaggctc caggattatt atgtggtgat tttgtgtcct actgaaatgg atgtacaggt 1140
tcgaagggta ctgcagattc caatgtggtc ccaacgagtt atctaccttc aaggttcagc 1200
ccttaaagat caagacctat tgagagcaaa gatggatgac gctgaggcct gttttattct 1260
cagtagccgt tgtgaagtgg ataggacatc atctgatcac caaacaattt tgagagcatg 1320
ggctgtgaaa gattttgctc caaattgtcc tttgtatgtc cagatattaa agcctgaaaa 1380
taaatttcac atcaaatttg ctgatcatgt tgtttgtgaa gaagagttta aatacgccat 1440
gttagcttta aactgtatat gcccagcaac atctacactt attacactac tggttcatac 1500
ctctagaggg cagtttggcg tctgcttgat tggtgttagg agggaggata ataaaaacat 1560
tttgctgaat ccaggtcctc gatacattat gaattctaca gacatatgct tttatattaa 1620
tattaccaaa gaagagaatt cagcatttaa aaaccaagac cagcagagaa aaagcaatgt 1680
gtccaggtcg ttttatcatg gaccttccag attacctgta catagcataa ttgccagcat 1740
gggtactgtg gctatagact tgcaagatac aagctgtaga tcagcaagtg gccctaccct 1800
gtctcttcct acagagggaa gcaaagaaat aagaagacct agcattgctc ctgttttaga 1860
ggttgcagat acatcatcga ttcaaacatg tgatcttcta agtgaccaat cagaagatga 1920
aactacacca gatgaagaaa tgtcttcaaa cttagagtat gctaaaggtt acccacctta 1980
ttctccatat ataggaagtt cacccacttt ttgtcatctc cttcatgaaa aagtaccatt 2040
ttgctgctta agattagaca agagttgcca acataactac tatgaggatg caaaagccta 2100
tggattcaaa aataaactaa ttatagttgc agctgaaaca gctggaaatg gattatataa 2160
ctttattgtt cctctcaggg catattatag accaaagaaa gaacttaatc ccatagtact 2220
gctattggat aaccccctag atgacttact caggtgtgga gtgacttttg ctgctaatat 2280
ggtggttgtg gataaagaga gcaccatgag tgccgaggaa gactacatgg cagatgccaa 2340
aaccattgtg aacgtgcaga cactcttcag gttgttttcc agtctcagta ttatcacaga 2400

gctaactcac cccgccaaca tgagattcat gcaattcaga gccaaagact gttactctct 2460
tgctctttca aaactggaaa agaaagaacg ggagagaggc tctaacttgg cctttatgtt 2520
tcgactgcct tttgctgctg ggagggtgtt tagcatcagt atgttggaca ctctgctgta 2580
tcagtcattt gtgaaggatt atatgatttc tatcacgaga cttctgttgg gactggacac 2640
tacaccagga tctgggtttc tttgttctat gaaaatcact gcagatgact tatggatcag 2700
aacttatgcc agactttatc agaagttgtg ttcttctact ggagatgttc ccattggaat 2760
ctacaggact gagtctcaga aacttactac atctgagtct caaatatcta tcagtgtaga 2820
agagtgggaa gacaccaaag actccaaaga acaagggcac caccgcagca accaccgcaa 2880
ctcaacatcc agtgaccagt cggaccatcc cttgctgcgg agaaaaagca tgcagtgggc 2940
ccgaagactg agcagaaaag gcccaaaaca ctctggtaaa acagctgaaa aaataaccca 3000
gcagcgactg aacctctaca ggaggtcaga aagacaagag cttgctgaac ttgtgaaaaa 3060
tagaatgaaa cacttgggtc tttctacagt gggatatgat gaaatgaatg atcatcaaag 3120
taccctctcc tacatcctga ttaacccatc tccagatacc agaatagagc tgaatgatgt 3180
tgtatactta attcgaccag atccactggc ctaccttcca aacagtgagc ccagtcgaag 3240
aaacagcatc tgcaatgtca ctggtcaaga ttctcgggag gaaactcaac tttgataaaa 3300
ataaaatgag aaactttttt cctacaaaga ccttgcttga aaccac 3346

<210> 14
<211> 26
<212> DNA
<213> Artificial Sequence
<220>
<223> Primer
<400> 14
ctccctcctc tcctccattt gttgtt 26

<210> 15
<211> 759
<212> PRT

<213> Homo sapiens

<400> 15

Met Val Asp Leu Glu Ser Glu Val Pro Pro Leu Pro Pro Arg Tyr Arg

                5                   10                  15

Phe Arg Asp Leu Leu Leu Gly Asp Gln Gly Trp Gln Asn Asp Asp Arg

            20                  25                  30

Val Gln Val Glu Phe Tyr Met Asn Glu Asn Thr Phe Lys Glu Arg Leu

        35                  40                  45

Lys Leu Phe Phe Ile Lys Asn Gln Arg Ser Ser Leu Arg Ile Arg Leu

    50                  55                  60

Phe Asn Phe Ser Leu Lys Leu Leu Ser Cys Leu Leu Tyr Ile Ile Arg

65                  70                  75                  80

Val Leu Leu Glu Asn Pro Ser Gln Gly Asn Glu Trp Ser His Ile Phe

                85                  90                  95

Trp Val Asn Arg Ser Leu Pro Leu Trp Gly Leu Gln Val Ser Val Ala

            100                 105                 110

Leu Ile Ser Leu Phe Glu Thr Ile Leu Leu Gly Tyr Leu Ser Tyr Lys

        115                 120                 125

Gly Asn Ile Trp Glu Gln Ile Leu Arg Ile Pro Phe Ile Leu Glu Ile

    130                 135                 140

Ile Asn Ala Val Pro Phe Ile Ile Ser Ile Phe Trp Pro Ser Leu Arg

145                 150                 155                 160

Asn Leu Phe Val Pro Val Phe Leu Asn Cys Trp Leu Ala Lys His Ala

                165                 170                 175

Leu Glu Asn Met Ile Asn Asp Leu His Arg Ala Ile Gln Arg Thr Gln

            180                 185                 190

Ser Ala Met Phe Asn Gln Val Leu Ile Leu Ile Ser Thr Leu Leu Cys

        195                 200                 205

Leu Ile Phe Thr Cys Ile Cys Gly Ile Gln His Leu Glu Arg Ile Gly

    210                 215                 220
Lys Lys Leu Asn Leu Phe Asp Ser Leu Tyr Phe Cys Ile Val Thr Phe
225                 230                 235                 240
Ser Thr Val Gly Phe Gly Asp Val Thr Pro Glu Thr Trp Ser Ser Lys
                245                 250                 255
Leu Phe Val Val Ala Met Ile Cys Val Ala Leu Val Val Leu Pro Ile
            260                 265                 270
Gln Phe Glu Gln Leu Ala Tyr Leu Trp Met Glu Arg Gln Lys Ser Gly
        275                 280                 285
Gly Asn Tyr Ser Arg His Arg Ala Gln Thr Glu Lys His Val Val Leu
    290                 295                 300
Cys Val Ser Ser Leu Lys Ile Asp Leu Leu Met Asp Phe Leu Asn Glu
305                 310                 315                 320
Phe Tyr Ala His Pro Arg Leu Gln Asp Tyr Tyr Val Val Ile Leu Cys
                325                 330                 335
Pro Thr Glu Met Asp Val Gln Val Arg Arg Val Leu Gln Ile Pro Met
            340                 345                 350
Trp Ser Gln Arg Val Ile Tyr Leu Gln Gly Ser Ala Leu Lys Asp Gln
        355                 360                 365
Asp Leu Leu Arg Ala Lys Met Asp Asp Ala Glu Ala Cys Phe Ile Leu
    370                 375                 380
Ser Ser Arg Cys Glu Val Asp Arg Thr Ser Ser Asp His Gln Thr Ile
385                 390                 395                 400
Leu Arg Ala Trp Ala Val Lys Asp Phe Ala Pro Asn Cys Pro Leu Tyr
                405                 410                 415
Val Gln Ile Leu Lys Pro Glu Asn Lys Phe His Ile Lys Phe Ala Asp
            420                 425                 430
His Val Val Cys Glu Glu Glu Phe Lys Tyr Ala Met Leu Ala Leu Asn
        435                 440                 445

Cys Ile Cys Pro Ala Thr Ser Thr Leu Ile Thr Leu Leu Val His Thr
    450                 455                 460
Ser Arg Gly Gln Glu Gly Gln Gln Ser Pro Glu Gln Trp Gln Lys Met
465                 470                 475                 480
Tyr Gly Arg Cys Ser Gly Asn Glu Val Tyr His Ile Val Leu Glu Glu
                485                 490                 495
Ser Thr Phe Phe Ala Glu Tyr Glu Gly Lys Ser Phe Thr Tyr Ala Ser
            500                 505                 510
Phe His Ala His Lys Lys Phe Gly Val Cys Leu Ile Gly Val Arg Arg
        515                 520                 525
Glu Asp Asn Lys Asn Ile Leu Leu Asn Pro Gly Pro Arg Tyr Ile Met
    530                 535                 540
Asn Ser Thr Asp Ile Cys Phe Tyr Ile Asn Ile Thr Lys Glu Glu Asn
545                 550                 555                 560
Ser Ala Phe Lys Asn Gln Asp Gln Gln Arg Lys Ser Asn Val Ser Arg
                565                 570                 575
Ser Phe Tyr His Gly Pro Ser Arg Leu Pro Val His Ser Ile Ile Ala
            580                 585                 590
Ser Met Gly Thr Val Ala Ile Asp Leu Gln Asp Thr Ser Cys Arg Ser
        595                 600                 605
Ala Ser Gly Pro Thr Leu Ser Leu Pro Thr Glu Gly Ser Lys Glu Ile
    610                 615                 620
Arg Arg Pro Ser Ile Ala Pro Val Leu Glu Val Ala Asp Thr Ser Ser
625                 630                 635                 640
Ile Gln Thr Cys Asp Leu Leu Ser Asp Gln Ser Glu Asp Glu Thr Thr
                645                 650                 655
Pro Asp Glu Glu Met Ser Ser Asn Leu Glu Tyr Ala Lys Gly Tyr Pro
            660                 665                 670
Pro Tyr Ser Pro Tyr Ile Gly Ser Ser Pro Thr Phe Cys His Leu Leu

675                 680                 685

His Glu Lys Val Pro Phe Cys Cys Leu Arg Leu Asp Lys Ser Cys Gln

    690                 695                 700

His Asn Tyr Tyr Glu Asp Ala Lys Ala Tyr Gly Phe Lys Asn Lys Leu

705                 710                 715                 720

Ile Ile Val Ala Ala Glu Thr Ala Gly Asn Gly Leu Tyr Asn Phe Ile

                725                 730                 735

Val Pro Leu Arg Ala Tyr Tyr Arg Pro Lys Lys Glu Leu Asn Pro Ile

            740                 745                 750

Val Leu Leu Leu Asp Asn Pro

        755

<210> 16

<211> 2277

<212> DNA

<213> Homo sapiens

<400> 16

atggttgatt tggagagcga agtgccccct ctgcctccca ggtacaggtt tcgagatttg    60

ctgctagggg accaaggatg gcaaaacgac gacagggtac aagttgaatt ctatatgaat    120

gaaaatacat ttaaagaaag actaaaatta tttttcataa aaaaccagag atcaagtcta    180

aggatacgcc tgttcaattt ttctctcaaa ttactaagct gcttattata cataatccga    240

gtactactag aaaacccttc acaaggaaat gaatggtctc atatcttttg ggtgaacaga    300

agtctacctt tgtggggctt acaggtttca gtggcattga taagtctgtt tgaaacaata    360

ttacttggtt atcttagtta taagggaaac atctgggaac agattttacg aatacccttc    420

atcttggaaa taattaatgc agttcccttc attatctcaa tattctggcc ttccttaagg    480

aatctatttg tcccagtctt tctgaactgt tggcttgcca aacatgcctt ggaaaatatg    540

attaatgatc tacacagagc cattcagcgt acacagtctg caatgtttaa tcaagttttg    600

attttaatat ctacattact atgccttatc ttcacctgca tttgtgggat ccaacatctg    660

gaacgaatag gaaagaagct gaatctcttt gactcccttt atttctgcat tgtgacgttt    720

tctactgtgg gcttcgggga tgtcactcct gaaacatggt cctccaagct ttttgtagtt 780
gctatgattt gtgttgctct tgtggttcta cccatacagt ttgaacagct ggcttatttg 840
tggatggaga gacaaaagtc aggaggaaac tatagtcgac atagagctca aactgaaaag 900
catgtcgtcc tgtgtgtcag ctcactgaag attgatttac ttatggattt tttaaatgaa 960
ttctatgctc atcctaggct ccaggattat tatgtggtga ttttgtgtcc tactgaaatg 1020
gatgtacagg ttcgaagggt actgcagatt ccaatgtggt cccaacgagt tatctacctt 1080
caaggttcag cccttaaaga tcaagaccta ttgagagcaa agatggatga cgctgaggcc 1140
tgttttattc tcagtagccg ttgtgaagtg gataggacat catctgatca ccaaacaatt 1200
ttgagagcat gggctgtgaa agattttgct ccaaattgtc ctttgtatgt ccagatatta 1260
aagcctgaaa ataaatttca catcaaattt gctgatcatg ttgtttgtga agaagagttt 1320
aaatacgcca tgttagcttt aaactgtata tgcccagcaa catctacact tattacacta 1380
ctggttcata cctctagagg gcaagaaggc cagcaatcgc cagaacaatg gcagaagatg 1440
tacggtagat gctccgggaa tgaagtctac cacattgttt tggaagaaag tacatttttt 1500
gctgaatatg aaggaaagag ttttacatat gcctctttcc atgcacacaa aaagtttggc 1560
gtctgcttga ttggtgttag gagggaggat aataaaaaca ttttgctgaa tccaggtcct 1620
cgatacatta tgaattctac agacatatgc ttttatatta atattaccaa agaagagaat 1680
tcagcattta aaaaccaaga ccagcagaga aaaagcaatg tgtccaggtc gttttatcat 1740
ggaccttcca gattacctgt acatagcata attgccagca tgggtactgt ggctatagac 1800
ttgcaagata caagctgtag atcagcaagt ggccctaccc tgtctcttcc tacagaggga 1860
agcaaagaaa taagaagacc tagcattgct cctgttttag aggttgcaga tacatcatcg 1920
attcaaacat gtgatcttct aagtgaccaa tcagaagatg aaactacacc agatgaagaa 1980
atgtcttcaa acttagagta tgctaaaggt tacccacctt attctccata tataggaagt 2040
tcacccactt tttgtcatct ccttcatgaa aaagtaccat tttgctgctt aagattagac 2100
aagagttgcc aacataacta ctatgaggat gcaaaagcct atggattcaa aaataaacta 2160
attatagttg cagctgaaac agctggaaat ggattatata actttattgt tcctctcagg 2220
gcatattata gaccaaagaa agaacttaat cccatagtac tgctattgga taaccca 2277

<210> 17
<211> 2576

<212> DNA

<213> Homo sapiens

<400> 17

ctccctcctc tcctccattt gttgtttgat gtttcccact ctttgaggaa ggatggttga 60

tttggagagc gaagtgcccc ctctgcctcc caggtacagg tttcgagatt tgctgctagg 120

ggaccaagga tggcaaaacg acgacagggt acaagttgaa ttctatatga atgaaaatac 180

atttaaagaa agactaaaat tatttttcat aaaaaaccag agatcaagtc taaggatacg 240

cctgttcaat ttttctctca aattactaag ctgcttatta tacataatcc gagtactact 300

agaaaaccct tcacaaggaa atgaatggtc tcatatcttt tgggtgaaca gaagtctacc 360

tttgtggggc ttacaggttt cagtggcatt gataagtctg tttgaaacaa tattacttgg 420

ttatcttagt tataagggaa acatctggga acagatttta cgaataccct tcatcttgga 480

aataattaat gcagttccct tcattatctc aatattctgg ccttccttaa ggaatctatt 540

tgtcccagtc tttctgaact gttggcttgc caaacatgcc ttggaaaata tgattaatga 600

tctacacaga gccattcagc gtacacagtc tgcaatgttt aatcaagttt tgattttaat 660

atctacatta ctatgcctta tcttcacctg catttgtggg atccaacatc tggaacgaat 720

aggaaagaag ctgaatctct ttgactccct ttatttctgc attgtgacgt tttctactgt 780

gggcttcggg gatgtcactc ctgaaacatg gtcctccaag ctttttgtag ttgctatgat 840

ttgtgttgct cttgtggttc tacccataca gtttgaacag ctggcttatt tgtggatgga 900

gagacaaaag tcaggaggaa actatagtcg acatagagct caaactgaaa agcatgtcgt 960

cctgtgtgtc agctcactga agattgattt acttatggat tttttaaatg aattctatgc 1020

tcatcctagg ctccaggatt attatgtggt gattttgtgt cctactgaaa tggatgtaca 1080

ggttcgaagg gtactgcaga ttccaatgtg gtcccaacga gttatctacc ttcaaggttc 1140

agcccttaaa gatcaagacc tattgagagc aaagatggat gacgctgagg cctgttttat 1200

tctcagtagc cgttgtgaag tggataggac atcatctgat caccaaacaa ttttgagagc 1260

atgggctgtg aaagattttg ctccaaattg tcctttgtat gtccagatat taaagcctga 1320

aaataaattt cacatcaaat ttgctgatca tgttgtttgt gaagaagagt ttaaatacgc 1380

catgttagct ttaaactgta tatgcccagc aacatctaca cttattacac tactggttca 1440

tacctctaga gggcaagaag gccagcaatc gccagaacaa tggcagaaga tgtacggtag 1500

atgctccggg aatgaagtct accacattgt tttggaagaa agtacatttt ttgctgaata 1560

tgaaggaaag agttttacat atgcctcttt ccatgcacac aaaaagtttg gcgtctgctt 1620
gattggtgtt aggagggagg ataataaaaa cattttgctg aatccaggtc ctcgatacat 1680
tatgaattct acagacatat gcttttatat taatattacc aaagaagaga attcagcatt 1740
taaaaaccaa gaccagcaga gaaaaagcaa tgtgtccagg tcgttttatc atggaccttc 1800
cagattacct gtacatagca taattgccag catgggtact gtggctatag acttgcaaga 1860
tacaagctgt agatcagcaa gtggccctac cctgtctctt cctacagagg gaagcaaaga 1920
aataagaaga cctagcattg ctcctgtttt agaggttgca gatacatcat cgattcaaac 1980
atgtgatctt ctaagtgacc aatcagaaga tgaaactaca ccagatgaag aaatgtcttc 2040
aaacttagag tatgctaaag gttacccacc ttattctcca tatataggaa gttcacccac 2100
tttttgtcat ctccttcatg aaaaagtacc attttgctgc ttaagattag acaagagttg 2160
ccaacataac tactatgagg atgcaaaagc ctatggattc aaaaataaac taattatagt 2220
tgcagctgaa acagctggaa atggattata taactttatt gttcctctca gggcatatta 2280
tagaccaaag aaagaactta atcccatagt actgctattg gataacccat gaaatgaatg 2340
atcatcaaag taccctctcc tacatcctga ttaacccatc tccagatacc agaatagagc 2400
tgaatgatgt tgtatactta attcgaccag atccactggc ctaccttcca aacagtgagc 2460
ccagtcgaag aaacagcatc tgcaatgtca ctggtcaaga ttctcgggag gaaactcaac 2520
tttgataaaa ataaaatgag aaactttttt cctacaaaga ccttgcttga aaccac 2576

<210> 18
<211> 1135
<212> PRT
<213> Mus spp
<400> 18
Met Val Asp Leu Glu Ser Glu Val Pro Pro Leu Pro Pro Arg Tyr Arg
                5                   10                  15
Phe Arg Asp Leu Leu Leu Gly Asp Gln Gly Trp Gln Asn Asp Asp Arg
            20                  25                  30
Val Gln Val Glu Phe Tyr Met Asn Glu Asn Thr Phe Lys Glu Arg Leu
        35                  40                  45

Lys Leu Phe Phe Ile Lys Asn Gln Arg Ser Ser Leu Arg Ile Arg Leu
50                  55                  60
Phe Asn Phe Ser Leu Lys Leu Leu Ser Cys Leu Leu Tyr Ile Ile Arg
65                  70                  75                  80
Val Leu Leu Glu Lys Pro Ser Gln Gly Asn Glu Trp Ser His Ile Phe
                85                  90                  95
Trp Val Asn Arg Ser Leu Pro Leu Trp Gly Leu Gln Val Ser Val Ala
            100                 105                 110
Leu Ile Ser Leu Phe Glu Thr Ile Leu Leu Gly Tyr Leu Ser Tyr Lys
        115                 120                 125
Gly Asn Ile Trp Glu Gln Ile Leu Arg Ile Pro Phe Ile Leu Glu Ile
    130                 135                 140
Ile Asn Ala Val Pro Phe Ile Ile Ser Ile Phe Trp Pro Thr Leu Arg
145                 150                 155                 160
Asn Leu Phe Val Pro Val Phe Leu Asn Cys Trp Leu Ala Lys His Ala
                165                 170                 175
Leu Glu Asn Met Ile Asn Asp Leu His Arg Ala Ile Gln Arg Thr Gln
            180                 185                 190
Ser Ala Met Phe Asn Gln Val Leu Ile Leu Ile Ser Thr Leu Leu Cys
        195                 200                 205
Leu Ile Phe Thr Cys Ile Cys Gly Ile Gln His Leu Glu Arg Ile Gly
    210                 215                 220
Lys Lys Leu Asn Leu Phe Asp Ser Leu Tyr Phe Cys Ile Val Thr Phe
225                 230                 235                 240
Ser Thr Val Gly Phe Gly Asp Val Thr Pro Glu Thr Trp Ser Ser Lys
                245                 250                 255
Leu Phe Val Val Ala Met Ile Cys Val Ala Leu Val Val Leu Pro Ile
            260                 265                 270
Gln Phe Glu Gln Leu Ala Tyr Leu Trp Met Glu Arg Gln Lys Ser Gly

        275                 280                 285
Gly Asn Tyr Ser Arg His Arg Ala Gln Thr Glu Lys His Val Val Leu
    290                 295                 300
Cys Val Ser Ser Leu Lys Ile Asp Leu Leu Met Asp Phe Leu Asn Glu
305                 310                 315                 320
Phe Tyr Ala His Pro Arg Leu Gln Asp Tyr Tyr Val Val Ile Leu Cys
                325                 330                 335
Pro Thr Glu Met Asp Val Gln Val Arg Arg Val Leu Gln Ile Pro Met
            340                 345                 350
Trp Ser Gln Arg Val Ile Tyr Leu Gln Gly Ser Ala Leu Lys Asp Gln
        355                 360                 365
Asp Leu Leu Arg Ala Lys Met Asp Asn Ala Glu Ala Cys Phe Ile Leu
370 375 . 380
Ser Ser Arg Cys Glu Val Asp Arg Thr Ser Ser Asp His Gln Thr Ile
385 390 395 400
Leu Arg Ala Trp Ala Val Lys Asp Phe Ala Pro Asn Cys Pro Leu Tyr
405 410 415
Val Gln Ile Leu Lys Pro Glu Asn Lys Phe His Ile Lys Phe Ala Asp
420 425 430
His Val Val Cys Glu Glu Glu Phe Lys Tyr Ala Met Leu Ala Leu Asn
435 440 445
Cys Ile Cys Pro Ala Thr Ser Thr Leu Ile Thr Leu Leu Val His Thr
450 455 460
Ser Arg Gly Gln Glu Gly Gln Gln Ser Pro Glu Gln Trp Gln Lys Thr
465 470 475 480
Tyr Gly Arg Cys Ser Gly Asn Glu Val Tyr His Ile Val Leu Glu Glu
485 490 495
Ser Thr Phe Phe Ala Glu Tyr Glu Gly Lys Ser Phe Thr Tyr Ala Ser
500 505 510

Phe His Ala His Lys Lys Phe Gly Val Cys Leu Val Gly Val Arg Arg
515 520 525
Glu Asp Asn Lys Asn Ile Leu Leu Asn Pro Gly Pro Arg Tyr Ile Met
530 535 540
Asn Ala Ser Asp Ile Cys Phe Tyr Ile Asn Ile Thr Lys Glu Glu Asn
545 550 555 560
Ser Ala Phe Lys Asn Gln Asp Gln Gln Arg Lys Ser Asn Val Ser Arg
565 570 575
Ser Phe Tyr His Gly Pro Ser Arg Leu Pro Val His Ser Ile Ile Ala
580 585 590
Ser Met Gly Thr Val Ala Ile Asp Leu Gln Asp Thr Ser Cys Arg Ala
595 600 605
Thr Ser Gly Pro Thr Leu Ala Leu Pro Ser Glu Gly Gly Lys Glu Leu
610 615 620
Arg Arg Pro Ser Ile Ala Pro Val Leu Glu Val Ala Asp Thr Ser Ser
625 630 635 640
Ile Gln Thr Cys Asp Leu Leu Ser Asp Gln Ser Glu Asp Glu Thr Thr
645 650 655
Pro Asp Glu Glu Thr Ser Ser Asn Leu Glu Tyr Ala Lys Gly Tyr Pro
660 665 670
Pro Tyr Ser Pro Tyr Ile Gly Ser Ser Pro Thr Phe Cys His Leu Leu
675 680 685
Gln Glu Lys Val Pro Phe Cys Cys Leu Arg Leu Asp Lys Ser Cys Gln
690 695 700
His Asn Tyr Tyr Glu Asp Ala Lys Ala Tyr Gly Phe Lys Asn Lys Leu
705 710 715 720
Ile Ile Val Ala Ala Glu Thr Ala Gly Asn Gly Leu Tyr Asn Phe Ile
725 730 735
Val Pro Leu Arg Ala Tyr Tyr Arg Pro Lys Lys Glu Leu Asn Pro Ile

740 745 750
Val Leu Leu Leu Asp Asn Pro Pro Asp Met His Phe Leu Asp Ala Ile
755 760 765
Cys Trp Phe Pro Met Val Tyr Tyr Met Val Gly Ser Ile Asp Asn Leu
770 775 780
Asp Asp Leu Leu Arg Cys Gly Val Thr Phe Ala Ala Asn Met Val Val
785 790 795 800
Val Asp Lys Glu Ser Thr Met Ser Ala Glu Glu Asp Tyr Met Ala Asp
805 810 815
Ala Lys Thr Ile Val Asn Val Gln Thr Leu Phe Arg Leu Phe Ser Ser
820 825 830
Leu Ser Ile Ile Thr Glu Leu Thr His Pro Ala Asn Met Arg Phe Met
835 840 845
Gln Phe Arg Ala Lys Asp Cys Tyr Ser Leu Ala Leu Ser Lys Leu Glu
850 855 860
Lys Lys Glu Arg Glu Arg Gly Ser Asn Leu Ala Phe Met Phe Arg Leu
865 870 875 880
Pro Phe Ala Ala Gly Arg Val Phe Ser Ile Ser Met Leu Asp Thr Leu
885 890 895
Leu Tyr Gln Ser Phe Val Lys Asp Tyr Met Ile Ser Ile Thr Arg Leu
900 905 910
Leu Leu Gly Leu Asp Thr Ile Pro Gly Ser Gly Phe Leu Cys Ser Met
915 920 925
Lys Ile Thr Glu Asp Asp Leu Trp Ile Arg Thr Tyr Ala Arg Leu Tyr
930 935 940
Gln Lys Leu Cys Ser Ser Thr Gly Asp Val Pro Ile Gly Ile Tyr Arg
945 950 955 960
Thr Glu Ser Gln Lys Leu Thr Thr Ser Glu Ser Gln Ile Ser Ile Ser
965 970 975

Val Glu Glu Trp Glu Asp Thr Lys Asp Val Lys Asp Pro Gly His His
980 985 990
Arg Ser Leu His Arg Asn Ser Thr Ser Ser Asp Gln Ser Asp His Pro
995 1000 1005
Leu Leu Arg Arg Lys Ser Met Gln Trp Ala Arg Arg Leu Ser Arg Lys
1010 1015 1020
Gly Pro Lys His Ser Gly Lys Thr Ala Glu Lys Ile Thr Gln Gln Arg
1025 1030 1035 1040
Leu Asn Leu Tyr Arg Arg Ser Glu Arg Gln Glu Leu Ala Glu Leu Val
1045 1050 1055
Lys Asn Arg Met Lys His Leu Gly Leu Ser Thr Val Gly Tyr Asp Glu
1060 1065 1070
Met Asn Asp His Gln Ser Thr Leu Ser Tyr Ile Leu Ile Asn Pro Ser
1075 1080 1085
Pro Asp Thr Arg Leu Glu Leu Asn Asp Val Val Tyr Leu Ile Arg Pro
1090 1095 1100
Asp Pro Leu Ser Tyr Leu Pro Asn Ser Glu Pro Ser Arg Lys Asn Ser
1105 1110 1115 1120
Ile Cys Asn Ala Ala Val Gln Asp Ser Arg Glu Glu Thr Gln Leu
1125 1130 1135

<210> 19
<211> 3405
<212> DNA
<213> Mus spp
<400> 19
atggttgatt tggagagcga agtgccccct ctgcctccca ggtacagatt ccgggatttg 60
ctgctagggg accaaggctg gcaaaatgat gacagggtcc aagttgaatt ctacatgaac 120
gaaaacacat ttaaagagag attaaaactg tttttcataa aaaaccagcg atcaagtcta 180

agaatccgct tgttcaactt ttccctgaaa ttgttaagct gcttattata tattatccgt 240
gtgttgcttg agaaaccgtc acagggaaat gaatggtctc acatattttg ggttaaccga 300
agtctacctt tatggggttt acaggtctca gtggcattga taagtctatt tgaaacaata 360
ctccttggtt atctcagtta taagggaaat atctgggagc agattttacg aattccattc 420
atcttggaaa taatcaatgc agtccccttc atcatctcaa tattctggcc caccttaaga 480
aatctatttg ttccagtttt tctaaattgt tggcttgcca aacatgcctt ggaaaatatg 540
attaatgatc tccatagagc cattcagcgt acacagtctg caatgtttaa tcaagttttg 600
attttgatat ctactttact atgccttatc ttcacttgca tttgtgggat tcagcatcta 660
gaacgaattg gcaagaagct caatctcttt gactcccttt atttctgcat tgtgacattt 720
tctactgtgg gctttgggga tgtcactcct gaaacatggt cctccaagct ctttgtcgtt 780
gctatgatct gcgttgccct tgtagtactc cctatacagt ttgaacagtt ggcctatttg 840
tggatggaga gacaaaagtc aggaggaaac tacagtcgac acagagctca gacagagaag 900
catgttgtcc tgtgtgtcag ctccttgaag attgacttac ttatggattt tctaaatgag 960
ttctatgcgc atccaagact acaggattat tacgtggtga ttttgtgtcc cacggagatg 1020
gatgtgcaag ttcggagagt actacagatt ccaatgtggt cccagagagt gatctacctt 1080
caaggctctg cccttaaaga ccaggatctc ctgagagcaa agatggacaa tgctgaggcc 1140
tgtttcattt tgagtagccg ctgcgaagtg gatagaacct catctgatca ccaaacaatc 1200
ttgagagcat gggctgtgaa agattttgcc ccaaattgtc ctctgtatgt tcagatatta 1260
aaaccagaaa acaaattcca catcaaattt gcagatcatg ttgtttgtga agaagagttt 1320
aagtacgcca tgttagcttt aaactgtata tgcccagcaa catctacact cattacacta 1380
ctggttcata cctctagagg gcaagaaggg cagcagtcac ctgaacagtg gcagaagact 1440
tacgggaggt gctcaggaaa cgaagtctat cacatcgttt tggaagaaag tacatttttt 1500
gctgaatatg aagggaagag ttttacctat gcttcttttc atgcccacaa aaagtttggt 1560
gtctgcttgg ttggtgttag gagggaggat aataaaaaca ttttgctgaa tccaggtcct 1620
cgatacatta tgaatgcttc agacatatgt ttttatatta atattaccaa agaagaaaat 1680
tcagcattta agaatcaaga ccaacagaga aaaagtaatg tgtcaaggtc attttatcat 1740
gggccttcca gattgcctgt ccacagcatc attgccagca tgggtactgt ggctatagac 1800
ttgcaagaca caagctgcag agcaacaagt ggccccaccc tggctcttcc ttcagaggga 1860
ggcaaagaat taagaagacc tagcattgct cctgttttag aggtggcaga tacatcatca 1920

attcaaacat gcgatctcct aagtgaccag tccgaagatg aaactacacc agatgaagaa 1980
acatcttcaa atttagagta tgccaaaggc tacccacctt attccccata cataggaagt 2040
tcacctactt tttgtcacct acttcaagaa aaagtgccat tttgctgctt aagattagac 2100
aagagttgcc agcataacta ctatgaggac gcaaaagcct atggattcaa aaataaacta 2160
attatagttg cagctgaaac agctggaaat ggactatata attttattgt acctctcagg 2220
gcatattata gaccaaagaa agaattaaat cccatagttc tgctattgga taacccgcca 2280
gatatgcatt ttctggatgc aatctgttgg tttccaatgg tttactacat ggtgggctct 2340
attgacaacc tagatgattt gctcaggtgt ggagtgacct ttgctgccaa catggtggtg 2400
gtggacaaag aaagcaccat gagtgcagag gaagactaca tggcagatgc caagacgatt 2460
gtgaatgtgc agactctgtt caggttgttt tcaagtctca gtatcatcac tgagcttacg 2520
catccagcaa atatgagatt catgcaattc agagccaagg attgttactc tctcgcgctt 2580
tcaaaactgg aaaagaaaga aagagaacga ggttctaact tggcctttat gtttcgactg 2640
ccttttgctg cgggaagagt gttcagcatc agtatgttag acacccttct ttatcagtca 2700
tttgtgaaag attatatgat ttctatcacc agacttcttt tgggactgga caccatacca 2760
ggatcggggt ttctttgttc tatgaaaatc actgaagatg acttgtggat cagaacgtat 2820
gccagactct atcagaagtt gtgttcttct actggagatg ttcccatcgg gatctacaga 2880
acagaatctc agaaactaac gacatctgag tctcaaatat ccattagtgt ggaagagtgg 2940
gaagacacca aagatgtcaa agacccaggg caccaccgca gccttcaccg caactcaacg 3000
tctagtgacc agtcggacca tcccttgctg cggaggaaga gcatgcagtg gcccgaaggg 3060
ctgagcagaa aagggccgaa gcactctggt aaaactgcag aaaaaataac tcaacagcga 3120
ctgaacctct accggagatc cgaaagacag gagcttgctg aacttgtgaa aaacagaatg 3180
aaacacctgg gcctctcgac ggtgggctac gatgaaatga atgatcatca gagcaccctg 3240
tcctacatcc tgattaatcc gtctccagac accaggcttg agctgaatga tgttgtatat 3300
ttgatccgac cagatccact gtcctacctt ccaaacagtg aacccagtag gaaaaacagc 3360
atctgcaatg ccgctgttca agattctcgg gaggaaactc aactc 3405

<210> 20
<211> 26
<212> DNA

<213> Artificial Sequence

<220>

<223> Primer

<400> 20

cctcctccat ccctccatcc cttgtt                                          26

<210> 21

<211> 26

<212> DNA

<213> Artificial Sequence

<220>

<223> Primer

<400> 21

gatccctgca aggactacaa tgtctc                                          26

<210> 22

<211> 3498

<212> DNA

<213> Mus spp

<400> 22

tctccatccc tccatccctt gttggatgtt gcccactcct taaggaagga tggttgattt    60

ggagagcgaa gtgccccctc tgcctcccag gtacagattc cgggatttgc tgctagggga   120

ccaaggctgg caaaatgatg acagggtcca agttgaattc tacatgaacg aaaacacatt   180

taaagagaga ttaaaactgt ttttcataaa aaaccagcga tcaagtctaa gaatccgctt   240

gttcaacttt tccctgaaat tgttaagctg cttattatat attatccgtg tgttgcttga   300

gaaaccgtca cagggaaatg aatggtctca catattttgg gttaaccgaa gtctaccttt   360

atggggttta caggtctcag tggcattgat aagtctattt gaaacaatac tccttggtta   420

tctcagttat aagggaaata tctgggagca gattttacga attccattca tcttggaaat   480

aatcaatgca gtccccttca tcatctcaat attctggccc accttaagaa atctatttgt   540

tccagttttt ctaaattgtt ggcttgccaa acatgccttg gaaaatatga ttaatgatct 600
ccatagagcc attcagcgta cacagtctgc aatgtttaat caagttttga ttttgatatc 660
tactttacta tgccttatct tcacttgcat ttgtgggatt cagcatctag aacgaattgg 720
caagaagctc aatctctttg actcccttta tttctgcatt gtgacatttt ctactgtggg 780
ctttggggat gtcactcctg aaacatggtc ctccaagctc tttgtcgttg ctatgatctg 840
cgttgccctt gtagtactcc ctatacagtt tgaacagttg gcctatttgt ggatggagag 900
acaaaagtca ggaggaaact acagtcgaca cagagctcag acagagaagc atgttgtcct 960
gtgtgtcagc tccttgaaga ttgacttact tatggatttt ctaaatgagt tctatgcgca 1020
tccaagacta caggattatt acgtggtgat tttgtgtccc acggagatgg atgtgcaagt 1080
tcggagagta ctacagattc caatgtggtc ccagagagtg atctaccttc aaggctctgc 1140
ccttaaagac caggatctcc tgagagcaaa gatggacaat gctgaggcct gtttcatttt 1200
gagtagccgc tgcgaagtgg atagaacctc atctgatcac caaacaatct tgagagcatg 1260
ggctgtgaaa gattttgccc caaattgtcc tctgtatgtt cagatattaa aaccagaaaa 1320
caaattccac atcaaatttg cagatcatgt tgtttgtgaa gaagagttta agtacgccat 1380
gttagcttta aactgtatat gcccagcaac atctacactc attacactac tggttcatac 1440
ctctagaggg caagaagggc agcagtcacc tgaacagtgg cagaagactt acgggaggtg 1500
ctcaggaaac gaagtctatc acatcgtttt ggaagaaagt acattttttg ctgaatatga 1560
agggaagagt tttacctatg cttcttttca tgcccacaaa aagtttggtg tctgcttggt 1620
tggtgttagg agggaggata ataaaaacat tttgctgaat ccaggtcctc gatacattat 1680
gaatgcttca gacatatgtt tttatattaa tattaccaaa gaagaaaatt cagcatttaa 1740
gaatcaagac caacagagaa aaagtaatgt gtcaaggtca ttttatcatg ggccttccag 1800
attgcctgtc cacagcatca ttgccagcat gggtactgtg gctatagact tgcaagacac 1860
aagctgcaga gcaacaagtg gccccaccct ggctcttcct tcagagggag gcaaagaatt 1920
aagaagacct agcattgctc ctgttttaga ggtggcagat acatcatcaa ttcaaacatg 1980
cgatctccta agtgaccagt ccgaagatga aactacacca gatgaagaaa catcttcaaa 2040
tttagagtat gccaaaggct acccacctta ttccccatac ataggaagtt cacctacttt 2100
ttgtcaccta cttcaagaaa aagtgccatt ttgctgctta agattagaca agagttgcca 2160
gcataactac tatgaggacg caaaagccta tggattcaaa aataaactaa ttatagttgc 2220
agctgaaaca gctggaaatg gactatataa ttttattgta cctctcaggg catattatag 2280

accaaagaaa gaattaaatc ccatagttct gctattggat aacccgccag atatgcattt 2340
tctggatgca atctgttggt ttccaatggt ttactacatg gtgggctcta ttgacaacct 2400
agatgatttg ctcaggtgtg gagtgacctt tgctgccaac atggtggtgg tggacaaaga 2460
aagcaccatg agtgcagagg aagactacat ggcagatgcc aagacgattg tgaatgtgca 2520
gactctgttc aggttgtttt caagtctcag tatcatcact gagcttacgc atccagcaaa 2580
tatgagattc atgcaattca gagccaagga ttgttactct ctcgcgcttt caaaactgga 2640
aaagaaagaa agagaacgag gttctaactt ggcctttatg tttcgactgc cttttgctgc 2700
gggaagagtg ttcagcatca gtatgttaga cacccttctt tatcagtcat ttgtgaaaga 2760
ttatatgatt tctatcacca gacttctttt gggactggac accataccag gatcggggtt 2820
tctttgttct atgaaaatca ctgaagatga cttgtggatc agaacgtatg ccagactcta 2880
tcagaagttg tgttcttcta ctggagatgt tcccatcggg atctacagaa cagaatctca 2940
gaaactaacg acatctgagt ctcaaatatc cattagtgtg gaagagtggg aagacaccaa 3000
agatgtcaaa gacccagggc accaccgcag ccttcaccgc aactcaacgt ctagtgacca 3060
gtcggaccat cccttgctgc ggaggaagag catgcagtgg gcccgaaggc tgagcagaaa 3120
agggccgaag cactctggta aaactgcaga aaaaataact caacagcgac tgaacctcta 3180
ccggagatcc gaaagacagg agcttgctga acttgtgaaa aacagaatga aacacctggg 3240
cctctcgacg gtgggctacg atgaaatgaa tgatcatcag agcaccctgt cctacatcct 3300
gattaatccg tctccagaca ccaggcttga gctgaatgat gttgtatatt tgatccgacc 3360
agatccactg tcctaccttc caaacagtga acccagtagg aaaaacagca tctgcaatgc 3420
cgctgttcaa gattctcggg aggaaactca actctgatga aaagaaaata agagacattg 3480
tagtccttgc agggatca 3498

<210> 23
<211> 1142
<212> PRT
<213> Mus spp
<400> 23
Met Val Asp Leu Glu Ser Glu Val Pro Pro Leu Pro Pro Arg Tyr Arg
                5                   10                  15

Phe Arg Asp Leu Leu Leu Gly Asp Gln Gly Trp Gln Asn Asp Asp Arg
            20                  25                  30
Val Gln Val Glu Phe Tyr Met Asn Glu Asn Thr Phe Lys Glu Arg Leu
        35                  40                  45
Lys Leu Phe Phe Ile Lys Asn Gln Arg Ser Ser Leu Arg Ile Arg Leu
    50                  55                  60
Phe Asn Phe Ser Leu Lys Leu Leu Ser Cys Leu Leu Tyr Ile Ile Arg
65                  70                  75                  80
Val Leu Leu Glu Lys Pro Ser Gln Gly Asn Glu Trp Ser His Ile Phe
                85                  90                  95
Trp Val Asn Arg Ser Leu Pro Leu Trp Gly Leu Gln Val Ser Val Ala
            100                 105                 110
Leu Ile Ser Leu Phe Glu Thr Ile Leu Leu Gly Tyr Leu Ser Tyr Lys
        115                 120                 125
Gly Asn Ile Trp Glu Gln Ile Leu Arg Ile Pro Phe Ile Leu Glu Ile
    130                 135                 140
Ile Asn Ala Val Pro Phe Ile Ile Ser Ile Phe Trp Pro Thr Leu Arg
145                 150                 155                 160
Asn Leu Phe Val Pro Val Phe Leu Asn Cys Trp Leu Ala Lys His Ala
                165                 170                 175
Leu Glu Asn Met Ile Asn Asp Leu His Arg Ala Ile Gln Arg Thr Gln
            180                 185                 190
Ser Ala Met Phe Asn Gln Val Leu Ile Leu Ile Ser Thr Leu Leu Cys
        195                 200                 205
Leu Ile Phe Thr Cys Ile Cys Gly Ile Gln His Leu Glu Arg Ile Gly
    210                 215                 220
Lys Lys Leu Asn Leu Phe Asp Ser Leu Tyr Phe Cys Ile Val Thr Phe
225                 230                 235                 240
Ser Thr Val Gly Phe Gly Asp Val Thr Pro Glu Thr Trp Ser Ser Lys

                245                 250                 255
Leu Phe Val Val Ala Met Ile Cys Val Ala Leu Val Val Leu Pro Ile
            260                 265                 270
Gln Phe Glu Gln Leu Ala Tyr Leu Trp Met Glu Arg Gln Lys Ser Gly
        275                 280                 285
Gly Asn Tyr Ser Arg His Arg Ala Gln Thr Glu Lys His Val Val Leu
    290                 295                 300
Cys Val Ser Ser Leu Lys Ile Asp Leu Leu Met Asp Phe Leu Asn Glu
305                 310                 315                 320
Phe Tyr Ala His Pro Arg Leu Gln Asp Tyr Tyr Val Val Ile Leu Cys
                325                 330                 335
Pro Thr Glu Met Asp Val Gln Val Arg Arg Val Leu Gln Ile Pro Met
            340                 345                 350
Trp Ser Gln Arg Val Ile Tyr Leu Gln Gly Ser Ala Leu Lys Asp Gln
        355                 360                 365
Asp Leu Leu Arg Ala Lys Met Asp Asn Ala Glu Ala Cys Phe Ile Leu
    370                 375                 380
Ser Ser Arg Cys Glu Val Asp Arg Thr Ser Ser Asp His Gln Thr Ile
385                 390                 395                 400
Leu Arg Ala Trp Ala Val Lys Asp Phe Ala Pro Asn Cys Pro Leu Tyr
                405                 410                 415
Val Gln Ile Leu Lys Pro Glu Asn Lys Phe His Ile Lys Phe Ala Asp
            420                 425                 430
His Val Val Cys Glu Glu Glu Phe Lys Tyr Ala Met Leu Ala Leu Asn
        435                 440                 445
Cys Ile Cys Pro Ala Thr Ser Thr Leu Ile Thr Leu Leu Val His Thr
    450                 455                 460
Ser Arg Gly Gln Glu Gly Gln Gln Ser Pro Glu Gln Trp Gln Lys Thr
465                 470                 475                 480

Tyr Gly Arg Cys Ser Gly Asn Glu Val Tyr His Ile Val Leu Glu Glu
                485                 490                 495
Ser Thr Phe Phe Ala Glu Tyr Glu Gly Lys Ser Phe Thr Tyr Ala Ser
            500                 505                 510
Phe His Ala His Lys Lys Phe Gly Val Cys Leu Val Gly Val Arg Arg
        515                 520                 525
Glu Asp Asn Lys Asn Ile Leu Leu Asn Pro Gly Pro Arg Tyr Ile Met
    530                 535                 540
Asn Ala Ser Asp Ile Cys Phe Tyr Ile Asn Ile Thr Lys Glu Glu Asn
545                 550                 555                 560
Ser Ala Phe Lys Asn Gln Asp Gln Gln Arg Lys Ser Asn Val Ser Arg
                565                 570                 575
Ser Phe Tyr His Gly Pro Ser Arg Leu Pro Val His Ser Ile Ile Ala
            580                 585                 590
Ser Met Gly Thr Val Ala Ile Asp Leu Gln Asp Thr Ser Cys Arg Ala
        595                 600                 605
Thr Ser Gly Pro Thr Leu Ala Leu Pro Ser Glu Gly Gly Lys Glu Leu
    610                 615                 620
Arg Arg Pro Ser Ile Ala Pro Val Leu Glu Val Ala Asp Thr Ser Ser
625                 630                 635                 640
Ile Gln Thr Cys Asp Leu Leu Ser Asp Gln Ser Glu Asp Glu Thr Thr
                645                 650                 655
Pro Asp Glu Glu Thr Ser Ser Asn Leu Glu Tyr Ala Lys Gly Tyr Pro
            660                 665                 670
Pro Tyr Ser Pro Tyr Ile Gly Ser Ser Pro Thr Phe Cys His Leu Leu
        675                 680                 685
Gln Glu Lys Val Pro Phe Cys Cys Leu Arg Leu Asp Lys Ser Cys Gln
    690                 695                 700
His Asn Tyr Tyr Glu Asp Ala Lys Ala Tyr Gly Phe Lys Asn Lys Leu

705                 710                 715                 720
Ile Ile Val Ala Ala Glu Thr Ala Gly Asn Gly Leu Tyr Asn Phe Ile
                725                 730                 735
Val Pro Leu Arg Ala Tyr Tyr Arg Pro Lys Lys Glu Leu Asn Pro Ile
            740                 745                 750
Val Leu Leu Leu Asp Asn Pro Pro Asp Met His Phe Leu Asp Ala Ile
        755                 760                 765
Cys Trp Phe Pro Met Val Tyr Tyr Met Val Gly Ser Ile Asp Asn Leu
    770                 775                 780
Asp Asp Leu Leu Arg Cys Gly Val Thr Phe Ala Ala Asn Met Val Val
785                 790                 795                 800
Val Asp Lys Glu Ser Thr Met Ser Ala Glu Glu Asp Tyr Met Ala Asp
                805                 810                 815
Ala Lys Thr Ile Val Asn Val Gln Thr Leu Phe Arg Leu Phe Ser Ser
            820                 825                 830
Leu Ser Ile Ile Thr Glu Leu Thr His Pro Ala Asn Met Arg Phe Met
        835                 840                 845
Gln Phe Arg Ala Lys Asp Cys Tyr Ser Leu Ala Leu Ser Lys Leu Glu
    850                 855                 860
Lys Lys Glu Arg Glu Arg Gly Ser Asn Leu Ala Phe Met Phe Arg Leu
865                 870                 875                 880
Pro Phe Ala Ala Gly Arg Val Phe Ser Ile Ser Met Leu Asp Thr Leu
                885                 890                 895
Leu Tyr Gln Ser Phe Val Lys Asp Tyr Met Ile Ser Ile Thr Arg Leu
            900                 905                 910
Leu Leu Gly Leu Asp Thr Ile Pro Gly Ser Gly Phe Leu Cys Ser Met
        915                 920                 925
Lys Ile Thr Glu Asp Asp Leu Trp Ile Arg Thr Tyr Ala Arg Leu Tyr
    930                 935                 940

Gln Lys Leu Cys Ser Ser Thr Gly Asp Val Pro Ile Gly Ile Tyr Arg
945                 950                 955                 960
Thr Glu Ser Gln Lys Leu Thr Thr Ser Glu Ser Arg Glu Ile Gly Ser
                965                 970                 975
Gln Ser Gln Ile Ser Ile Ser Val Glu Glu Trp Glu Asp Thr Lys Asp
            980                 985                 990
Val Lys Asp Pro Gly His His Arg Ser Leu His Arg Asn Ser Thr Ser
        995                 1000                1005
Ser Asp Gln Ser Asp His Pro Leu Leu Arg Arg Lys Ser Met Gln Trp
    1010                1015                1020
Ala Arg Arg Leu Ser Arg Lys Gly Pro Lys His Ser Gly Lys Thr Ala
1025                1030                1035                1040
Glu Lys Ile Thr Gln Gln Arg Leu Asn Leu Tyr Arg Arg Ser Glu Arg
                1045                1050                1055
Gln Glu Leu Ala Glu Leu Val Lys Asn Arg Met Lys His Leu Gly Leu
            1060                1065                1070
Ser Thr Val Gly Tyr Asp Glu Met Asn Asp His Gln Ser Thr Leu Ser
        1075                1080                1085
Tyr Ile Leu Ile Asn Pro Ser Pro Asp Thr Arg Leu Glu Leu Asn Asp
    1090                1095                1100
Val Val Tyr Leu Ile Arg Pro Asp Pro Leu Ser Tyr Leu Pro Asn Ser
1105                1110                1115                1120
Glu Pro Ser Arg Lys Asn Ser Ile Cys Asn Ala Ala Val Gln Asp Ser
                1125                1130                1135
Arg Glu Glu Thr Gln Leu
            1140

<210> 24
<211> 3426

<212> DNA

<213> Mus spp

<400> 24

atggttgatt tggagagcga agtgccccct ctgcctccca ggtacagatt ccgggatttg 60

ctgctagggg accaaggctg gcaaaatgat gacagggtcc aagttgaatt ctacatgaac 120

gaaaacacat ttaaagagag attaaaactg tttttcataa aaaaccagcg atcaagtcta 180

agaatccgct tgttcaactt ttccctgaaa ttgttaagct gcttattata tattatccgt 240

gtgttgcttg agaaaccgtc acagggaaat gaatggtctc acatattttg ggttaaccga 300

agtctacctt tatggggttt acaggtctca gtggcattga taagtctatt tgaaacaata 360

ctccttggtt atctcagtta taagggaaat atctgggagc agattttacg aattccattc 420

atcttggaaa taatcaatgc agtccccttc atcatctcaa tattctggcc caccttaaga 480

aatctatttg ttccagtttt tctaaattgt tggcttgcca aacatgcctt ggaaaatatg 540

attaatgatc tccatagagc cattcagcgt acacagtctg caatgtttaa tcaagttttg 600

attttgatat ctactttact atgccttatc ttcacttgca tttgtgggat tcagcatcta 660

gaacgaattg gcaagaagct caatctcttt gactcccttt atttctgcat tgtgacattt 720

tctactgtgg gctttgggga tgtcactcct gaaacatggt cctccaagct ctttgtcgtt 780

gctatgatct gcgttgccct tgtagtactc cctatacagt ttgaacagtt ggcctatttg 840

tggatggaga gacaaaagtc aggaggaaac tacagtcgac acagagctca gacagagaag 900

catgttgtcc tgtgtgtcag ctccttgaag attgacttac ttatggattt tctaaatgag 960

ttctatgcgc atccaagact acaggattat tacgtggtga ttttgtgtcc cacggagatg 1020

gatgtgcaag ttcggagagt actacagatt ccaatgtggt cccagagagt gatctacctt 1080

caaggctctg cccttaaaga ccaggatctc ctgagagcaa agatggacaa tgctgaggcc 1140

tgtttcattt tgagtagccg ctgcgaagtg gatagaacct catctgatca ccaaacaatc 1200

ttgagagcat gggctgtgaa agattttgcc ccaaattgtc ctctgtatgt tcagatatta 1260

aaaccagaaa acaaattcca catcaaattt gcagatcatg ttgtttgtga agaagagttt 1320

aagtacgcca tgttagcttt aaactgtata tgcccagcaa catctacact cattacacta 1380

ctggttcata cctctagagg gcaagaaggg cagcagtcac ctgaacagtg gcagaagact 1440

tacgggaggt gctcaggaaa cgaagtctat cacatcgttt tggaagaaag tacatttttt 1500

gctgaatatg aagggaagag ttttacctat gcttcttttc atgcccacaa aaagtttggt 1560

gtctgcttgg ttggtgttag gagggaggat aataaaaaca ttttgctgaa tccaggtcct 1620
cgatacatta tgaatgcttc agacatatgt ttttatatta atattaccaa agaagaaaat 1680
tcagcattta agaatcaaga ccaacagaga aaaagtaatg tgtcaaggtc attttatcat 1740
gggccttcca gattgcctgt ccacagcatc attgccagca tgggtactgt ggctatagac 1800
ttgcaagaca caagctgcag agcaacaagt ggccccaccc tggctcttcc ttcagaggga 1860
ggcaaagaat taagaagacc tagcattgct cctgttttag aggtggcaga tacatcatca 1920
attcaaacat gcgatctcct aagtgaccag tccgaagatg aaactacacc agatgaagaa 1980
acatcttcaa atttagagta tgccaaaggc tacccacctt attccccata cataggaagt 2040
tcacctactt tttgtcacct acttcaagaa aaagtgccat tttgctgctt aagattagac 2100
aagagttgcc agcataacta ctatgaggac gcaaaagcct atggattcaa aaataaacta 2160
attatagttg cagctgaaac agctggaaat ggactatata attttattgt acctctcagg 2220
gcatattata gaccaaagaa agaattaaat cccatagttc tgctattgga taacccgcca 2280
gatatgcatt ttctggatgc aatctgttgg tttccaatgg tttactacat ggtgggctct 2340
attgacaacc tagatgattt gctcaggtgt ggagtgacct ttgctgccaa catggtggtg 2400
gtggacaaag aaagcaccat gagtgcagag gaagactaca tggcagatgc caagacgatt 2460
gtgaatgtgc agactctgtt caggttgttt tcaagtctca gtatcatcac tgagcttacg 2520
catccagcaa atatgagatt catgcaattc agagccaagg attgttactc tctcgcgctt 2580
tcaaaactgg aaaagaaaga aagagaacga ggttctaact tggcctttat gtttcgactg 2640
ccttttgctg cgggaagagt gttcagcatc agtatgttag acacccttct ttatcagtca 2700
tttgtgaaag attatatgat ttctatcacc agacttcttt tgggactgga caccatacca 2760
ggatcggggt ttctttgttc tatgaaaatc actgaagatg acttgtggat cagaacgtat 2820
gccagactct atcagaagtt gtgttcttct actggagatg ttcccatcgg gatctacaga 2880
acagaatctc agaaactaac gacatctgag tctcgagaaa taggatctca atctcaaata 2940
tccattagtg tggaagagtg ggaagacacc aaagatgtca aagacccagg gcaccaccgc 3000
agccttcacc gcaactcaac gtctagtgac cagtcggacc atcccttgct gcggaggaag 3060
agcatgcagt gggcccgaag gctgagcaga aaagggccga agcactctgg taaaactgca 3120
gaaaaaataa ctcaacagcg actgaacctc taccggagat ccgaaagaca ggagcttgct 3180
gaacttgtga aaaacagaat gaaacacctg ggcctctcga cggtgggcta cgatgaaatg 3240
aatgatcatc agagcaccct gtcctacatc ctgattaatc cgtctccaga caccaggctt 3300

gagctgaatg atgttgtata tttgatccga ccagatccac tgtcctacct tccaaacagt 3360
gaacccagta ggaaaaacag catctgcaat gccgctgttc aagattctcg ggaggaaact 3420
caactc 3426

<210> 25
<211> 3523
<212> DNA
<213> Mus spp
<400> 25
tcctcctcca tccctccatc ccttgttgga tgttgcccac tccttaagga aggatggttg 60
atttggagag cgaagtgccc cctctgcctc ccaggtacag attccgggat ttgctgctag 120
gggaccaagg ctggcaaaat gatgacaggg tccaagttga attctacatg aacgaaaaca 180
catttaaaga gagattaaaa ctgtttttca taaaaaacca gcgatcaagt ctaagaatcc 240
gcttgttcaa cttttccctg aaattgttaa gctgcttatt atatattatc cgtgtgttgc 300
ttgagaaacc gtcacaggga aatgaatggt ctcacatatt ttgggttaac cgaagtctac 360
ctttatgggg tttacaggtc tcagtggcat tgataagtct atttgaaaca atactccttg 420
gttatctcag ttataaggga aatatctggg agcagatttt acgaattcca ttcatcttgg 480
aaataatcaa tgcagtcccc ttcatcatct caatattctg gcccacctta agaaatctat 540
ttgttccagt ttttctaaat tgttggcttg ccaaacatgc cttggaaaat atgattaatg 600
atctccatag agccattcag cgtacacagt ctgcaatgtt taatcaagtt ttgattttga 660
tatctacttt actatgcctt atcttcactt gcatttgtgg gattcagcat ctagaacgaa 720
ttggcaagaa gctcaatctc tttgactccc tttatttctg cattgtgaca ttttctactg 780
tgggctttgg ggatgtcact cctgaaacat ggtcctccaa gctctttgtc gttgctatga 840
tctgcgttgc ccttgtagta ctccctatac agtttgaaca gttggcctat ttgtggatgg 900
agagacaaaa gtcaggagga aactacagtc gacacagagc tcagacagag aagcatgttg 960
tcctgtgtgt cagctccttg aagattgact tacttatgga ttttctaaat gagttctatg 1020
cgcatccaag actacaggat tattacgtgg tgattttgtg tcccacggag atggatgtgc 1080
aagttcggag agtactacag attccaatgt ggtcccagag agtgatctac cttcaaggct 1140
ctgcccttaa agaccaggat ctcctgagag caaagatgga caatgctgag gcctgtttca 1200

ttttgagtag ccgctgcgaa gtggatagaa cctcatctga tcaccaaaca atcttgagag 1260
catgggctgt gaaagatttt gccccaaatt gtcctctgta tgttcagata ttaaaaccag 1320
aaaacaaatt ccacatcaaa tttgcagatc atgttgtttg tgaagaagag tttaagtacg 1380
ccatgttagc tttaaactgt atatgcccag caacatctac actcattaca ctactggttc 1440
atacctctag agggcaagaa gggcagcagt cacctgaaca gtggcagaag acttacggga 1500
ggtgctcagg aaacgaagtc tatcacatcg ttttggaaga aagtacattt tttgctgaat 1560
atgaagggaa gagttttacc tatgcttctt ttcatgccca caaaaagttt ggtgtctgct 1620
tggttggtgt taggagggag gataataaaa acattttgct gaatccaggt cctcgataca 1680
ttatgaatgc ttcagacata tgtttttata ttaatattac caaagaagaa aattcagcat 1740
ttaagaatca agaccaacag agaaaaagta atgtgtcaag gtcattttat catgggcctt 1800
ccagattgcc tgtccacagc atcattgcca gcatgggtac tgtggctata gacttgcaag 1860
acacaagctg cagagcaaca agtggcccca ccctggctct tccttcagag ggaggcaaag 1920
aattaagaag acctagcatt gctcctgttt tagaggtggc agatacatca tcaattcaaa 1980
catgcgatct cctaagtgac cagtccgaag atgaaactac accagatgaa gaaacatctt 2040
caaatttaga gtatgccaaa ggctacccac cttattcccc atacatagga agttcaccta 2100
ctttttgtca cctacttcaa gaaaaagtgc cattttgctg cttaagatta gacaagagtt 2160
gccagcataa ctactatgag gacgcaaaag cctatggatt caaaaataaa ctaattatag 2220
ttgcagctga aacagctgga aatggactat ataattttat tgtacctctc agggcatatt 2280
atagaccaaa gaaagaatta aatcccatag ttctgctatt ggataacccg ccagatatgc 2340
attttctgga tgcaatctgt tggtttccaa tggtttacta catggtgggc tctattgaca 2400
acctagatga tttgctcagg tgtggagtga cctttgctgc caacatggtg gtggtggaca 2460
aagaaagcac catgagtgca gaggaagact acatggcaga tgccaagacg attgtgaatg 2520
tgcagactct gttcaggttg ttttcaagtc tcagtatcat cactgagctt acgcatccag 2580
caaatatgag attcatgcaa ttcagagcca aggattgtta ctctctcgcg ctttcaaaac 2640
tggaaaagaa agaaagagaa cgaggttcta acttggcctt tatgtttcga ctgccttttg 2700
ctgcgggaag agtgttcagc atcagtatgt tagacaccct tctttatcag tcatttgtga 2760
aagattatat gatttctatc accagacttc ttttgggact ggacaccata ccaggatcgg 2820
ggtttctttg ttctatgaaa atcactgaag atgacttgtg gatcagaacg tatgccagac 2880
tctatcagaa gttgtgttct tctactggag atgttcccat cgggatctac agaacagaat 2940

ctcagaaact aacgacatct gagtctcgag aaataggatc tcaatctcaa atatccatta 3000
gtgtggaaga gtgggaagac accaaagatg tcaaagaccc agggcaccac cgcagccttc 3060
accgcaactc aacgtctagt gaccagtcgg accatccctt gctgcggagg aagagcatgc 3120
agtgggcccg aaggctgagc agaaaagggc cgaagcactc tggtaaaact gcagaaaaaa 3180
taactcaaca gcgactgaac ctctaccgga gatccgaaag acaggagctt gctgaacttg 3240
tgaaaaacag aatgaaacac ctgggcctct cgacggtggg ctacgatgaa atgaatgatc 3300
atcagagcac cctgtcctac atcctgatta atccgtctcc agacaccagg cttgagctga 3360
atgatgttgt atatttgatc cgaccagatc cactgtccta ccttccaaac agtgaaccca 3420
gtaggaaaaa cagcatctgc aatgccgctg ttcaagattc tcgggaggaa actcaactct 3480
gatgaaaaga aaataagaga cattgtagtc cttgcaggga tca 3523

<210> 26
<211> 1111
<212> PRT
<213> Mus spp
<400> 26
Met Val Asp Leu Glu Ser Glu Val Pro Pro Leu Pro Pro Arg Tyr Arg
                5                   10                  15
Phe Arg Asp Leu Leu Leu Gly Asp Gln Gly Trp Gln Asn Asp Asp Arg
            20                  25                  30
Val Gln Val Glu Phe Tyr Met Asn Glu Asn Thr Phe Lys Glu Arg Leu
        35                  40                  45
Lys Leu Phe Phe Ile Lys Asn Gln Arg Ser Ser Leu Arg Ile Arg Leu
    50                  55                  60
Phe Asn Phe Ser Leu Lys Leu Leu Ser Cys Leu Leu Tyr Ile Ile Arg
65                  70                  75                  80
Val Leu Leu Glu Lys Pro Ser Gln Gly Asn Glu Trp Ser His Ile Phe
                85                  90                  95
Trp Val Asn Arg Ser Leu Pro Leu Trp Gly Leu Gln Val Ser Val Ala

            100                 105                 110
Leu Ile Ser Leu Phe Glu Thr Ile Leu Leu Gly Tyr Leu Ser Tyr Lys
        115                 120                 125
Gly Asn Ile Trp Glu Gln Ile Leu Arg Ile Pro Phe Ile Leu Glu Ile
    130                 135                 140
Ile Asn Ala Val Pro Phe Ile Ile Ser Ile Phe Trp Pro Thr Leu Arg
145                 150                 155                 160
Asn Leu Phe Val Pro Val Phe Leu Asn Cys Trp Leu Ala Lys His Ala
                165                 170                 175
Leu Glu Asn Met Ile Asn Asp Leu His Arg Ala Ile Gln Arg Thr Gln
            180                 185                 190
Ser Ala Met Phe Asn Gln Val Leu Ile Leu Ile Ser Thr Leu Leu Cys
        195                 200                 205
Leu Ile Phe Thr Cys Ile Cys Gly Ile Gln His Leu Glu Arg Ile Gly
    210                 215                 220
Lys Lys Leu Asn Leu Phe Asp Ser Leu Tyr Phe Cys Ile Val Thr Phe
225                 230                 235                 240
Ser Thr Val Gly Phe Gly Asp Val Thr Pro Glu Thr Trp Ser Ser Lys
                245                 250                 255
Leu Phe Val Val Ala Met Ile Cys Val Ala Leu Val Val Leu Pro Ile
            260                 265                 270
Gln Phe Glu Gln Leu Ala Tyr Leu Trp Met Glu Arg Gln Lys Ser Gly
        275                 280                 285
Gly Asn Tyr Ser Arg His Arg Ala Gln Thr Glu Lys His Val Val Leu
    290                 295                 300
Cys Val Ser Ser Leu Lys Ile Asp Leu Leu Met Asp Phe Leu Asn Glu
305                 310                 315                 320
Phe Tyr Ala His Pro Arg Leu Gln Asp Tyr Tyr Val Val Ile Leu Cys
                325                 330                 335

Pro Thr Glu Met Asp Val Gln Val Arg Arg Val Leu Gln Ile Pro Met
            340                 345                 350
Trp Ser Gln Arg Val Ile Tyr Leu Gln Gly Ser Ala Leu Lys Asp Gln
        355                 360                 365
Asp Leu Leu Arg Ala Lys Met Asp Asn Ala Glu Ala Cys Phe Ile Leu
    370                 375                 380
Ser Ser Arg Cys Glu Val Asp Arg Thr Ser Ser Asp His Gln Thr Ile
385                 390                 395                 400
Leu Arg Ala Trp Ala Val Lys Asp Phe Ala Pro Asn Cys Pro Leu Tyr
                405                 410                 415
Val Gln Ile Leu Lys Pro Glu Asn Lys Phe His Ile Lys Phe Ala Asp
            420                 425                 430
His Val Val Cys Glu Glu Glu Phe Lys Tyr Ala Met Leu Ala Leu Asn
        435                 440                 445
Cys Ile Cys Pro Ala Thr Ser Thr Leu Ile Thr Leu Leu Val His Thr
    450                 455                 460
Ser Arg Gly Gln Glu Gly Gln Gln Ser Pro Glu Gln Trp Gln Lys Thr
465                 470                 475                 480
Tyr Gly Arg Cys Ser Gly Asn Glu Val Tyr His Ile Val Leu Glu Glu
                485                 490                 495
Ser Thr Phe Phe Ala Glu Tyr Glu Gly Lys Ser Phe Thr Tyr Ala Ser
            500                 505                 510
Phe His Ala His Lys Lys Phe Gly Val Cys Leu Val Gly Val Arg Arg
        515                 520                 525
Glu Asp Asn Lys Asn Ile Leu Leu Asn Pro Gly Pro Arg Tyr Ile Met
    530                 535                 540
Asn Ala Ser Asp Ile Cys Phe Tyr Ile Asn Ile Thr Lys Glu Glu Asn
545                 550                 555                 560
Ser Ala Phe Lys Asn Gln Asp Gln Gln Arg Lys Ser Asn Val Ser Arg

                565                 570                 575
Ser Phe Tyr His Gly Pro Ser Arg Leu Pro Val His Ser Ile Ile Ala
            580                 585                 590
Ser Met Gly Thr Val Ala Ile Asp Leu Gln Asp Thr Ser Cys Arg Ala
        595                 600                 605
Thr Ser Gly Pro Thr Leu Ala Leu Pro Ser Glu Gly Gly Lys Glu Leu
    610                 615                 620
Arg Arg Pro Ser Ile Ala Pro Val Leu Glu Val Ala Asp Thr Ser Ser
625                 630                 635                 640
Ile Gln Thr Cys Asp Leu Leu Ser Asp Gln Ser Glu Asp Glu Thr Thr
                645                 650                 655
Pro Asp Glu Glu Thr Ser Ser Asn Leu Glu Tyr Ala Lys Gly Tyr Pro
            660                 665                 670
Pro Tyr Ser Pro Tyr Ile Gly Ser Ser Pro Thr Phe Cys His Leu Leu
        675                 680                 685
Gln Glu Lys Val Pro Phe Cys Cys Leu Arg Leu Asp Lys Ser Cys Gln
    690                 695                 700
His Asn Tyr Tyr Glu Asp Ala Lys Ala Tyr Gly Phe Lys Asn Lys Leu
705                 710                 715                 720
Ile Ile Val Ala Ala Glu Thr Ala Gly Asn Gly Leu Tyr Asn Phe Ile
                725                 730                 735
Val Pro Leu Arg Ala Tyr Tyr Arg Pro Lys Lys Glu Leu Asn Pro Ile
            740                 745                 750
Val Leu Leu Leu Asp Asn Pro Leu Asp Asp Leu Leu Arg Cys Gly Val
        755                 760                 765
Thr Phe Ala Ala Asn Met Val Val Val Asp Lys Glu Ser Thr Met Ser
    770                 775                 780
Ala Glu Glu Asp Tyr Met Ala Asp Ala Lys Thr Ile Val Asn Val Gln
785                 790                 795                 800

Thr Leu Phe Arg Leu Phe Ser Ser Leu Ser Ile Ile Thr Glu Leu Thr
                805                 810                 815
His Pro Ala Asn Met Arg Phe Met Gln Phe Arg Ala Lys Asp Cys Tyr
            820                 825                 830
Ser Leu Ala Leu Ser Lys Leu Glu Lys Lys Glu Arg Glu Arg Gly Ser
        835                 840                 845
Asn Leu Ala Phe Met Phe Arg Leu Pro Phe Ala Ala Gly Arg Val Phe
    850                 855                 860
Ser Ile Ser Met Leu Asp Thr Leu Leu Tyr Gln Ser Phe Val Lys Asp
865                 870                 875                 880
Tyr Met Ile Ser Ile Thr Arg Leu Leu Leu Gly Leu Asp Thr Ile Pro
                885                 890                 895
Gly Ser Gly Phe Leu Cys Ser Met Lys Ile Thr Glu Asp Asp Leu Trp
            900                 905                 910
Ile Arg Thr Tyr Ala Arg Leu Tyr Gln Lys Leu Cys Ser Ser Thr Gly
        915                 920                 925
Asp Val Pro Ile Gly Ile Tyr Arg Thr Glu Ser Gln Lys Leu Thr Thr
    930                 935                 940
Ser Glu Ser Gln Ile Ser Ile Ser Val Glu Glu Trp Glu Asp Thr Lys
945                 950                 955                 960
Asp Val Lys Asp Pro Gly His His Arg Ser Leu His Arg Asn Ser Thr
                965                 970                 975
Ser Ser Asp Gln Ser Asp His Pro Leu Leu Arg Arg Lys Ser Met Gln
            980                 985                 990
Trp Ala Arg Arg Leu Ser Arg Lys Gly Pro Lys His Ser Gly Lys Thr
        995                 1000                1005
Ala Glu Lys Ile Thr Gln Gln Arg Leu Asn Leu Tyr Arg Arg Ser Glu
    1010                1015                1020
Arg Gln Glu Leu Ala Glu Leu Val Lys Asn Arg Met Lys His Leu Gly

1025                1030                1035                1040

Leu Ser Thr Val Gly Tyr Asp Glu Met Asn Asp His Gln Ser Thr Leu

                1045                1050                1055

Ser Tyr Ile Leu Ile Asn Pro Ser Pro Asp Thr Arg Leu Glu Leu Asn

            1060                1065                1070

Asp Val Val Tyr Leu Ile Arg Pro Asp Pro Leu Ser Tyr Leu Pro Asn

        1075                1080                1085

Ser Glu Pro Ser Arg Lys Asn Ser Ile Cys Asn Ala Ala Val Gln Asp

    1090                1095                1100

Ser Arg Glu Glu Thr Gln Leu

1105                1110

<210> 27

<211> 3333

<212> DNA

<213> Mus spp

<400> 27
   atggttgatt tggagagcga agtgccccct ctgcctccca ggtacagatt ccgggatttg      60
   ctgctagggg accaaggctg gcaaaatgat gacagggtcc aagttgaatt ctacatgaac     120
   gaaaacacat ttaaagagag attaaaactg tttttcataa aaaaccagcg atcaagtcta     180
   agaatccgct tgttcaactt ttccctgaaa ttgttaagct gcttattata tattatccgt     240
   gtgttgcttg agaaaccgtc acagggaaat gaatggtctc acatatttgg ggttaaccga     300
   agtctacctt tatggggttt acaggtctca gtggcattga taagtctatt tgaaacaata     360
   ctccttggtt atctcagtta taagggaaat atctgggagc agattttacg aattccattc     420
   atcttggaaa taatcaatgc agtccccttc atcatctcaa tattctggcc caccttaaga     480
   aatctatttg ttccagtttt tctaaattgt tggcttgcca aacatgcctt ggaaaatatg     540
   attaatgatc tccatagagc cattcagcgt acacagtctg caatgtttaa tcaagttttg     600
   attttgatat ctactttact atgccttatc ttcacttgca tttgtgggat tcagcatcta     660
   gaacgaattg gcaagaagct caatctcttt gactcccttt atttctgcat tgtgacattt     720
   tctactgtgg gctttgggga tgtcactcct gaaacatggt cctccaagct ctttgtcgtt 780
   gctatgatct gcgttgccct tgtagtactc cctatacagt ttgaacagtt ggcctatttg 840
   tggatggaga gacaaaagtc aggaggaaac tacagtcgac acagagctca gacagagaag 900
   catgttgtcc tgtgtgtcag ctccttgaag attgacttac ttatggattt tctaaatgag 960
   ttctatgcgc atccaagact acaggattat tacgtggtga ttttgtgtcc cacggagatg 1020
   gatgtgcaag ttcggagagt actacagatt ccaatgtggt cccagagagt gatctacctt 1080
   caaggctctg cccttaaaga ccaggatctc ctgagagcaa agatggacaa tgctgaggcc 1140
   tgtttcattt tgagtagccg ctgcgaagtg gatagaacct catctgatca ccaaacaatc 1200
   ttgagagcat gggctgtgaa agattttgcc ccaaattgtc ctctgtatgt tcagatatta 1260
   aaaccagaaa acaaattcca catcaaattt gcagatcatg ttgtttgtga agaagagttt 1320
   aagtacgcca tgttagcttt aaactgtata tgcccagcaa catctacact cattacacta 1380
   ctggttcata cctctagagg gcaagaaggg cagcagtcac ctgaacagtg gcagaagact 1440
   tacgggaggt gctcaggaaa cgaagtctat cacatcgttt tggaagaaag tacatttttt 1500
   gctgaatatg aagggaagag ttttacctat gcttcttttc atgcccacaa aaagtttggt 1560
   gtctgcttgg ttggtgttag gagggaggat aataaaaaca ttttgctgaa tccaggtcct 1620
   cgatacatta tgaatgcttc agacatatgt ttttatatta atattaccaa agaagaaaat 1680
   tcagcattta agaatcaaga ccaacagaga aaaagtaatg tgtcaaggtc attttatcat 1740
   gggccttcca gattgcctgt ccacagcatc attgccagca tgggtactgt ggctatagac 1800
   ttgcaagaca caagctgcag agcaacaagt ggccccaccc tggctcttcc ttcagaggga 1860
   ggcaaagaat taagaagacc tagcattgct cctgttttag aggtggcaga tacatcatca 1920
   attcaaacat gcgatctcct aagtgaccag tccgaagatg aaactacacc agatgaagaa 1980
   acatcttcaa atttagagta tgccaaaggc tacccacctt attccccata catagaaagt 2040
   tcacctactt tttgtcacct acttcaagaa aaagtgccat tttgctgctt aagattagac 2100
   aagagttgcc agcataacta ctatgaggac gcaaaagcct atggattcaa aaataaacta 2160
   attatagttg cagctgaaac agctggaaat ggactatata attttattgt acctctcagg 2220
   gcatattata gaccaaagaa agaattaaat cccatagttc tgctattgga taacccccta 2280
   gatgatttgc tcaggtgtgg agtgaccttt gctgccaaca tggtggtggt ggacaaagaa 2340
   agcaccatga gtgcagagga agactacatg gcagatgcca agacgattgt gaatgtgcag 2400
   actctgttca ggttgttttc aagtctcagt atcatcactg agcttacgca tccagcaaat 2460
   atgagattca tgcaattcag agccaaggat tgttactctc tcgcgctttc aaaactggaa 2520
   aagaaagaaa gagaacgagg ttctaacctg gcctttatgt ttcgactgcc ttttgctgcg 2580
   ggaagagtgt tcagcatcag tatgttagac acccttcttt atcagtcatt tgtgaaagat 2640
   tatatgattt ctatcaccag acttcttttg ggactggaca ccataccagg atcggggttt 2700
   ctttgttcta tgaaaatcac tgaagatgac ttgtggatca gaacatatgc cagactctat 2760
   cagaagttgt gttcttctac tggagatgtt cccatcggga tctacagaac agaatctcag 2820
   aaactaacga catctgagtc tcaaatatcc attagtgtgg aagagtggga agacaccaaa 2880
   gatgtcaaag acccagggca ccaccgcagc cttcaccgca actcaacgtc tagtgaccag 2940
   tcagaccatc ccttgctgcg gaggaagagc atgcagtggg cccgaaggct gagcagaaaa 3000
   gggccgaagc actctggtaa aactgcagaa aaaataactc aacagcgact gaacctctac 3060
   cggagatccg aaagacagga gcttgctgaa cttgtgaaaa acagaatgaa acacctgggc 3120
   ctctcgacgg tgggctacga tgaaatgaat gatcatcaga gcaccctgtc ctacatcctg 3180
   attaatccgt ctccagacac caggcttgag ctgaatgatg ttgtatattt gatccgacca 3240
   gatccactgt cctaccttcc aaacagtgaa cccagtagga aaaacagcat ctgcaatgcc 3300
   gctgttcaag attctcggga ggaaactcaa ctc 3333

<210> 28
<211> 3430
<212> DNA
<213> Mus spp
<400> 28
tcctcctcca tccctccatc ccttgttgga tgttgcccac tccttaagga aggatggttg 60
atttggagag cgaagtgccc cctctgcctc ccaggtacag attccgggat ttgctgctag 120
gggaccaagg ctggcaaaat gatgacaggg tccaagttga attctacatg aacgaaaaca 180
catttaaaga gagattaaaa ctgtttttca taaaaaacca gcgatcaagt ctaagaatcc 240
gcttgttcaa cttttccctg aaattgttaa gctgcttatt atatattatc cgtgtgttgc 300
ttgagaaacc gtcacaggga aatgaatggt ctcacatatt ttgggttaac cgaagtctac 360
ctttatgggg tttacaggtc tcagtggcat tgataagtct atttgaaaca atactccttg 420
gttatctcag ttataaggga aatatctggg agcagatttt acgaattcca ttcatcttgg 480

aaataatcaa tgcagtcccc ttcatcatct caatattctg gcccacctta agaaatctat 540
ttgttccagt ttttctaaat tgttggcttg ccaaacatgc cttggaaaat atgattaatg 600
atctccatag agccattcag cgtacacagt ctgcaatgtt taatcaagtt ttgattttga 660
tatctacttt actatgcctt atcttcactt gcatttgtgg gattcagcat ctagaacgaa 720
ttggcaagaa gctcaatctc tttgactccc tttatttctg cattgtgaca ttttctactg 780
tgggctttgg ggatgtcact cctgaaacat ggtcctccaa gctctttgtc gttgctatga 840
tctgcgttgc ccttgtagta ctccctatac agtttgaaca gttggcctat ttgtggatgg 900
agagacaaaa gtcaggagga aactacagtc gacacagagc tcagacagag aagcatgttg 960
tcctgtgtgt cagctccttg aagattgact tacttatgga ttttctaaat gagttctatg 1020
cgcatccaag actacaggat tattacgtgg tgattttgtg tcccacggag atggatgtgc 1080
aagttcggag agtactacag attccaatgt ggtcccagag agtgatctac cttcaaggct 1140
ctgcccttaa agaccaggat ctcctgagag caaagatgga caatgctgag gcctgtttca 1200
ttttgagtag ccgctgcgaa gtggatagaa cctcatctga tcaccaaaca atcttgagag 1260
catgggctgt gaaagatttt gccccaaatt gtcctctgta tgttcagata ttaaaaccag 1320
aaaacaaatt ccacatcaaa tttgcagatc atgttgtttg tgaagaagag tttaagtacg 1380
ccatgttagc tttaaactgt atatgcccag caacatctac actcattaca ctactggttc 1440
atacctctag agggcaagaa gggcagcagt cacctgaaca gtggcagaag acttacggga 1500
ggtgctcagg aaacgaagtc tatcacatcg ttttggaaga aagtacattt tttgctgaat 1560
atgaagggaa gagttttacc tatgcttctt ttcatgccca caaaaagttt ggtgtctgct 1620
tggttggtgt taggagggag gataataaaa acattttgct gaatccaggt cctcgataca 1680
ttatgaatgc ttcagacata tgtttttata ttaatattac caaagaagaa aattcagcat 1740
ttaagaatca agaccaacag agaaaaagta atgtgtcaag gtcattttat catgggcctt 1800
ccagattgcc tgtccacagc atcattgcca gcatgggtac tgtggctata gacttgcaag 1860
acacaagctg cagagcaaca agtggcccca ccctggctct tccttcagag ggaggcaaag 1920
aattaagaag acctagcatt gctcctgttt tagaggtggc agatacatca tcaattcaaa 1980
catgcgatct cctaagtgac cagtccgaag atgaaactac accagatgaa gaaacatctt 2040
caaatttaga gtatgccaaa ggctacccac cttattcccc atacatagga agttcaccta 2100
ctttttgtca cctacttcaa gaaaaagtgc cattttgctg cttaagatta gacaagagtt 2160
gccagcataa ctactatgag gacgcaaaag cctatggatt caaaaataaa ctaattatag 2220

ttgcagctga aacagctgga aatggactat ataattttat tgtacctctc agggcatatt 2280
atagaccaaa gaaagaatta aatcccatag ttctgctatt ggataacccc ctagatgatt 2340
tgctcaggtg tggagtgacc tttgctgcca acatggtggt ggtggacaaa gaaagcacca 2400
tgagtgcaga ggaagactac atggcagatg ccaagacgat tgtgaatgtg cagactctgt 2460
tcaggttgtt ttcaagtctc agtatcatca ctgagcttac gcatccagca aatatgagat 2520
tcatgcaatt cagagccaag gattgttact ctctcgcgct ttcaaaactg gaaaagaaag 2580
aaagagaacg aggttctaac ctggccttta tgtttcgact gccttttgct gcgggaagag 2640
tgttcagcat cagtatgtta gacacccttc tttatcagtc atttgtgaaa gattatatga 2700
tttctatcac cagacttctt ttgggactgg acaccatacc aggatcgggg tttctttgtt 2760
ctatgaaaat cactgaagat gacttgtgga tcagaacata tgccagactc tatcagaagt 2820
tgtgttcttc tactggagat gttcccatcg ggatctacag aacagaatct cagaaactaa 2880
cgacatctga gtctcaaata tccattagtg tggaagagtg ggaagacacc aaagatgtca 2940
aagacccagg gcaccaccgc agccttcacc gcaactcaac gtctagtgac cagtcagacc 3000
atcccttgct gcggaggaag agcatgcagt gggcccgaag gctgagcaga aaagggccga 3060
agcactctgg taaaactgca gaaaaaataa ctcaacagcg actgaacctc taccggagat 3120
ccgaaagaca ggagcttgct gaacttgtga aaaacagaat gaaacacctg ggcctctcga 3180
cggtgggcta cgatgaaatg aatgatcatc agagcaccct gtcctacatc ctgattaatc 3240
cgtctccaga caccaggctt gagctgaatg atgttgtata tttgatccga ccagatccac 3300
tgtcctacct tccaaacagt gaacccagta ggaaaaacag catctgcaat gccgctgttc 3360
aagattctcg ggaggaaact caactctgat gaaaagaaaa taagagacat tgtagtcctt 3420
gcagggatca 3430

<210> 29
<211> 24
<212> DNA
<213> Artificial Sequence
<220>
<223> Primer
<400> 29

ctgtatatgc ccagcaacat ctac 24

<210> 30
<211> 22
<212> DNA
<213> Artificial Sequence
<220>
<223> Primer
<400> 30
cgtacatctt ctgccattgt tc 22

<210> 31
<211> 26
<212> DNA
<213> Artificial Sequence
<220>
<223> Probe
<400> 31
cagtgtgtgt gcctgtgttg cagaga 26

<210> 32
<211> 26
<212> DNA
<213> Artificial Sequence
<220>
<223> Primer
<400> 32
tgcccagcaa catctacact ta 22

<210> 33

<211> 30

<212> DNA

<213> Artificial Sequence

<220>

<223> Probe

<400> 33

tacctctaga gggcaagaag gccagcaatc 30

<210> 34

<211> 21

<212> DNA

<213> Artificial Sequence

<220>

<223> Primer

<400> 34

tcctccctcc taacaccaat c 21

<210> 35

<211> 26

<212> DNA

<213> Artificial Sequence

<220>

<223> Probe

<400> 35

tacctctaga gggcagtttg gcgtctgctt 30

<210> 36

<211> 20

<212> DNA

<213> Artificial Sequence

<220>

<223> Primer

<400> 36

gaggatgcaa aagcctatgg                                                    20

<210> 37

<211> 22

<212> DNA

<213> Artificial Sequence

<220>

<223> Primer

<400> 37

cttgaccagt gacattgcag at                                                 22

<210> 38

<211> 30

<212> DNA

<213> Artificial Sequence

<220>

<223> Probe

<400> 38

attggataac ccatgaaatg aatgatcatc                                         30

<210> 39

<211> 19

<212> DNA

<213> Artificial Sequence

<220>

<223> Primer

<400> 39

tggagatgtt cccattgga 19

<210> 40

<211> 26

<212> DNA

<213> Artificial Sequence

<220>

<223> Primer

<400> 40

gtcttcccac tcttctacac tgatag 26

<210> 41

<211> 30

<212> DNA

<213> Artificial Sequence

<220>

<223> Probe

<400> 41

atctgagtct cgaaaaatag catcacaatc 30

<210> 42

<211> 26

<212> DNA

<213> Artificial Sequence

<220>

<223> Primer

<400> 42

ctggatgcaa tctgttggtt tc                                                22

<210> 43

<211> 26

<212> DNA

<213> Artificial Sequence

<220>

<223> Primer

<400> 43

gcacgttcac aatggttttg g                                                 21

<210> 44

<211> 30

<212> DNA

<213> Artificial Sequence

<220>

<223> Probe

<400> 44

ttgtggataa agagagcacc atgagtgcc                                         29

## Description

### TECHNICAL FIELD

The present invention provides a novel Ca²⁺ dependent K⁺ channel protein, a polynucleotide encoding the protein, a method for screening a compound that enhances or inhibits activities of the protein, a compound obtained by the screening method and the like.

### BACKGROUND ART

K⁺ channel, which is a membrane protein that selectively transmits a K⁺ ion, is generally present in prokaryotes to human and consists of numerous families. In vivo, it distributes in comprehensive tissues, and closely relates to important physiological functions such as generation of the resting membrane potential of cells, re-polarization, regulation of frequency for generating action potential and the like.

K⁺ channel has a huge variety of structures. In addition to six transmembrane type, two transmembrane type and one transmembrane type, a combined type channel molecule, wherein two basic structures are combined, is present.

Ca²⁺ dependent K⁺ channel (K_{Ca}) belongs to the six transmembrane type together with potential dependent K⁺ channel, and is classified into three families, Big-K (BK), Intermediate-K (IK) and Small-K (SK) based on channel conductance. The conductance of BK is 100 - 220 pS. BK is alternatively referred to as maxi-K or Slo (since Big-K from Drosophila is a gene accounting for the mutant Slowpoke). The human Slo was cloned in 1994 (Mol. Brain Res., Vol. 27, pp. 189 - 193, 1994). The conductance of IK and SK are 20 - 85 pS and 2 - 20 pS, respectively. The human IK1 (Proc. Natl. Acad. Sci. USA, Vol. 94, pp. 11651 - 11656, 1997) and the human SKI (Science, Vol. 273, pp. 1709 - 1714, 1996) were cloned. In either family, pore region is present in the region between the fifth and the sixth transmembrane regions. In addition, it is considered that a C-terminal portion thereof, which is located at the inside of the cell, plays a role of Ca²⁺ sensitivity. The C-terminal portion is essential for expression of the function. By splicing the region, channels, which possess different Ca²⁺ dependencies, are formed. In K_{Ca}, main unit for channel is α subunit. However, only recently, it has been found that in the channel, β subunit is present as well as that of potential dependent K⁺ channel (Proc. Natl. Acad. Sci. USA, Vol. 93, pp. 9200 - 9205, 1996). The β subunit has two transmembrane regions. It is presumed that the β subunit has a structure, wherein the N-terminus and the C-terminus are present inside the cells. Further, it is considered that the β subunit plays a role of regulating the a subunit.

Since the human SK is highly expressed in dopamine-actuated mesocephalic nerve cells, it is considered that the Ca²⁺ dependent K⁺ channel plays a physiological function in the nerve cells. In addition, since dysfunction of the nerve cells is observed in schizophrenia, relation between schizophrenia and the human SK is pointed out (J. Neurosci., Vol. 21, pp. 3443 - 3456, 2001). It has been reported that the human IK1 relates to volume regulation of erythrocyte (Current. Opin. Hematol., Vol. 4, pp. 122 - 127, 1997) and regulation of secretion of CI⁻ in small intestine (J. Clin. Invest., Vol. 98, pp. 2066 - 2075, 1996). Further, it has been known that IK1 inhibitor has a soothing effect on secretory diarrhea (J. Clin. Invest., Vol. 100, pp. 3111 - 3120, 1997).

For chicken-derived Ca²⁺ dependent K⁺ channel, cSlack has been reported (GenBank Accession No. AAM18770).

It is considered that K_{Ca} perceives membrane potential of cells and intracellular Ca²⁺ ion concentration, and regulates transmission of K⁺ ion. On the other hand, for K_{Ca}, various molecules, wherein channel conductance, sensitivity for Ca²⁺, response against channel activity regulating factor such as protein phosphorylase, and the like are different, are present. Their expression sites are also diverse. It is considered that K_{Ca} plays various important roles for the living body such as muscle contraction, incretion, transmission of K⁺ ion in kidney, signal transduction in nerve, etc. However, for the relation between each molecule and function thereof, there are various uncertain factors. Therefore, elucidation of the uncertainty may be conductive to development of medicament for treating various diseases relevant to K_{Ca}.

### DISCLOSURE OF THE INVENTION

The inventors have been intent on the study for solving the above-mentioned problems, and have found a novel Ca²⁺ dependent K⁺ channel protein. For a method of regulating the protein, it includes, for example, inhibition or enhancement of K⁺ ion transmission, lowering of expression level by suppression of transcription for the gene encoding the protein, activation of the promoter for the gene encoding the protein, accentuation of expression level by stabilizing mRNA, and the like. Based on the findings, the inventors have further studied and attained to perfection of the present invention.

That is, the present invention provides:
(1) A protein comprising the same or substantially the same amino acid sequence as the amino acid sequence represented by SEQ ID NO: 1, SEQ ID NO: 8, SEQ ID NO: 11 or SEQ ID NO: 15, or a salt thereof;
(2) The protein according to (1) or a salt thereof, wherein the protein comprising the same or substantially the same amino acid sequence as the amino acid sequence represented by SEQ ID NO: 1, SEQ ID NO: 8, SEQ ID NO: 11 or SEQ ID NO: 15 is a protein consisting of the amino acid sequence represented by SEQ ID NO: 18, SEQ ID NO: 23 or SEQ ID NO: 26;
(3) A protein consisting of the amino acid sequence represented by SEQ ID NO: 1, or a salt thereof;
(4) A protein consisting of the amino acid sequence represented by SEQ ID NO: 8, or a salt thereof;
(5) A protein consisting of the amino acid sequence represented by SEQ ID NO: 11, or a salt thereof;
(6) A protein consisting of the amino acid sequence represented by SEQ ID NO: 15, or a salt thereof;
(7) A partial peptide of the protein according to claim 1, or a salt thereof;
(8) A polynucleotide comprising the polynucleotide encoding the protein according to claim 1 or the partial peptide according to (7);
(9) The polynucleotide according to (8), which is a DNA;
(10) A polynucleotide consisting of the base sequence represented by SEQ ID NO: 2, SEQ ID NO: 9, SEQ ID NO: 12 or SEQ ID NO: 16;
(11) A polynucleotide consisting of the base sequence represented by SEQ ID NO: 19, SEQ ID NO: 24 or SEQ ID NO: 27;
(12) A recombinant vector comprising the polynucleotide according to (8);
(13) A transformant, which is transformed with the recombinant vector according to (12);
(14) A method for manufacturing the protein according to (1) or the partial peptide according to (7) or salts thereof, which comprises culturing the transformant according to (13), producing and accumulating the protein according to (1) or the partial peptide according to (7) and collecting them;
(15) A medicament comprising the protein according to (1) or the partial peptide according to (7) or salts thereof;
(16) A medicament comprising the polynucleotide according to (8);
(17) A diagnostic comprising the polynucleotide according to (8);
(18) An antibody to the protein according to (1) or the partial peptide according to (7) or salts thereof;
(19) A diagnostic comprising the antibody according to (18);
(20) A medicament comprising the antibody according to (18);
(21) A polynucleotide comprising a base sequence complementary or substantially complementary to the base sequence of the polynucleotide according to (8) or a portion thereof;
(22) A medicament comprising the polynucleotide according to (21);
(23) A method for screening a compound that enhances or inhibits activities of the protein according to (1) or the partial peptide according to (7) or salts thereof, or a salt thereof, which comprises using the protein according to (1) or the partial peptide according to (7) or salts thereof;
(24) A kit for screening a compound that enhances or inhibits activities of the protein according to (1) or the partial peptide according to (7) or salts thereof, or a salt thereof, which comprises the protein according to (1) or the partial peptide according to (7) or salts thereof;
(25) A compound that enhances or inhibits activities of the protein according to (1) or the partial peptide according to (7) or salts thereof, or a salt thereof, which is obtained by using the screening method according to (23) or the screening kit according to (24);
(26) A medicament comprising the compound according to (25) or a salt thereof;
(27) A method for screening a compound that enhances or inhibits an expression of the gene encoding the protein according to (1), which comprises using the polynucleotide according to (8);
(28) A kit for screening a compound that enhances or inhibits an expression of the gene encoding the protein according to (1), which comprises the polynucleotide according to (8);
(29) A compound that enhances or inhibits an expression of the gene encoding the protein according to (1), which is obtained by the screening method according to (27) or the screening kit according to (28);
(30) A medicament comprising the compound according to (29) or a salt thereof;
(31) A method for quantifying the protein according to (1), which comprises using the antibody according to (18);
(32) A method for diagnosing a disease associated with the function of the protein according to (1), which comprises using the quantification method according to (31);
(33) A method for screening a compound that enhances or inhibits an expression of the gene encoding the protein according to (1), which comprises using the antibody according to (18);
(34) A kit for screening a compound that enhances or inhibits an expression of the gene encoding the protein according to (1), which comprises the polynucleotide according to (18);
(35) A compound that enhances or inhibits an expression of the gene encoding the protein according to (1), which is obtained by the screening method according to (33) or the screening kit according to (34);
(36) A medicament comprising the compound according to (35) or a salt thereof;
(37) The medicament according to (16), (20), (22), (26), (30) or (36), which is a preventive and/or therapeutic agent for alimentary diseases, genital diseases or respiratory diseases;
(38) The diagnostic according to (17) or (19), which is a diagnostic for alimentary diseases, genital diseases or respiratory diseases;
(39) A preventing and/or treating method for alimentary diseases, genital diseases or respiratory diseases, which comprises administrating to mammal an effective amount of the compound according to (25), (29) or (35) or salts thereof;
(40) Use of the compound according to (25), (29) or (35) or salts thereof for manufacturing a preventive and/or therapeutic agent for alimentary diseases, genital diseases or respiratory diseases; and the like.

### BRIEF DESCRIPTION OF THE DRAWINGS

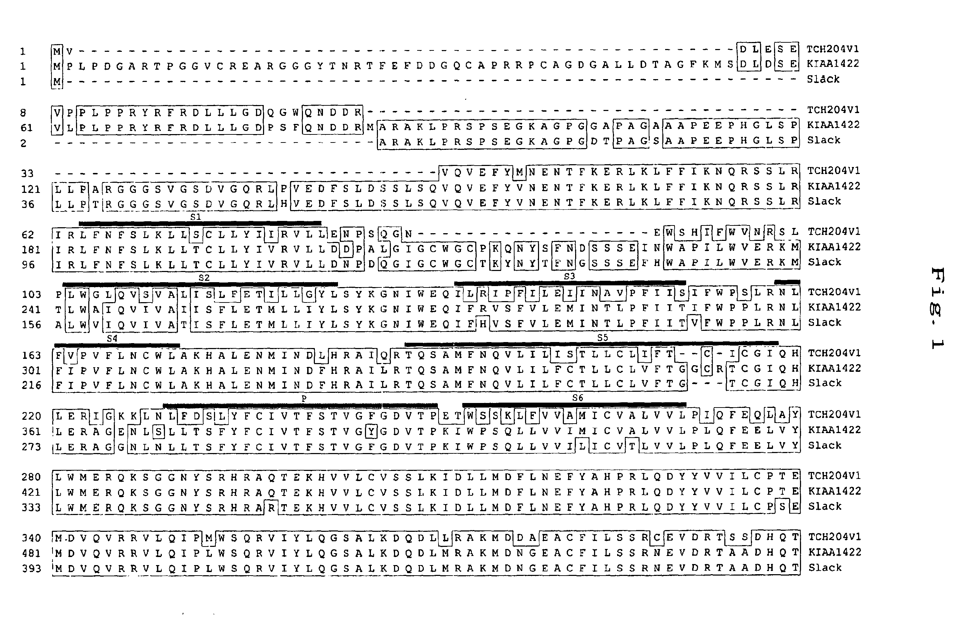

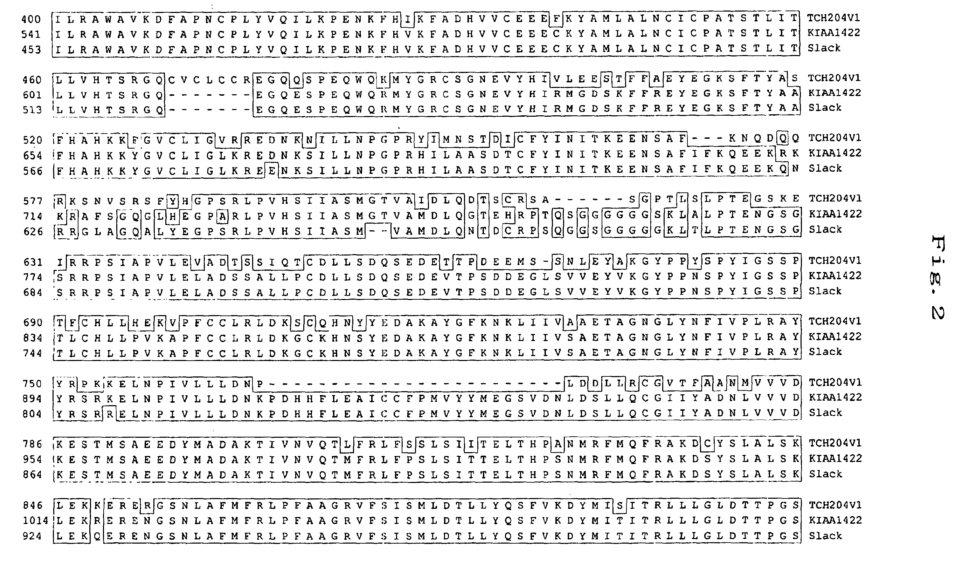

Fig. 1 is a drawing that shows a comparison of the amino acid sequences among human TCH204 variant 1 protein, KIAA1422 and Slack. In the figure, TCH204V1 shows the amino acid sequence represented by SEQ ID NO: 1. S 1 to S6 and P indicate transmembrane regions and a pore region, respectively. The amino acid residues embraced with square show amino acid residues identical to TCH204V1. (continued to Fig. 2)

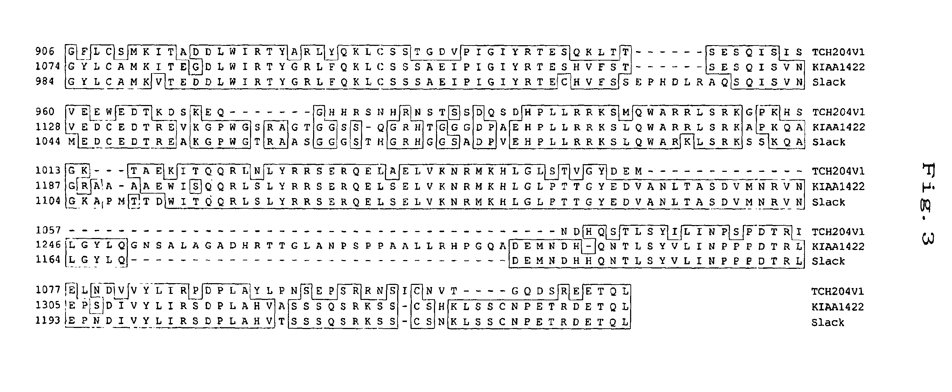

Fig. 2 is a drawing that shows a comparison of the amino acid sequences among human TCH204 variant I protein, KIAA1422 and Slack. In the figure, TCH204V1 shows the amino acid sequence represented by SEQ ID NO: 1. S1 to S6 and P indicate transmembrane regions and a pore region, respectively. The amino acid residues embraced with square show amino acid residues identical to TCH204V1. (continued from Fig. 1 and continued to Fig. 3)
Fig. 3 is a drawing that shows a comparison of the amino acid sequences among human TCH204 variant 1 protein, KIAA1422 and Slack. In the figure, TCH204V1 shows the amino acid sequence represented by SEQ ID NO: 1. S1 to S6 and P indicate transmembrane regions and a pore region, respectively. The amino acid residues embraced with square show amino acid residues identical to TCH204V1. (continued from Fig. 2)

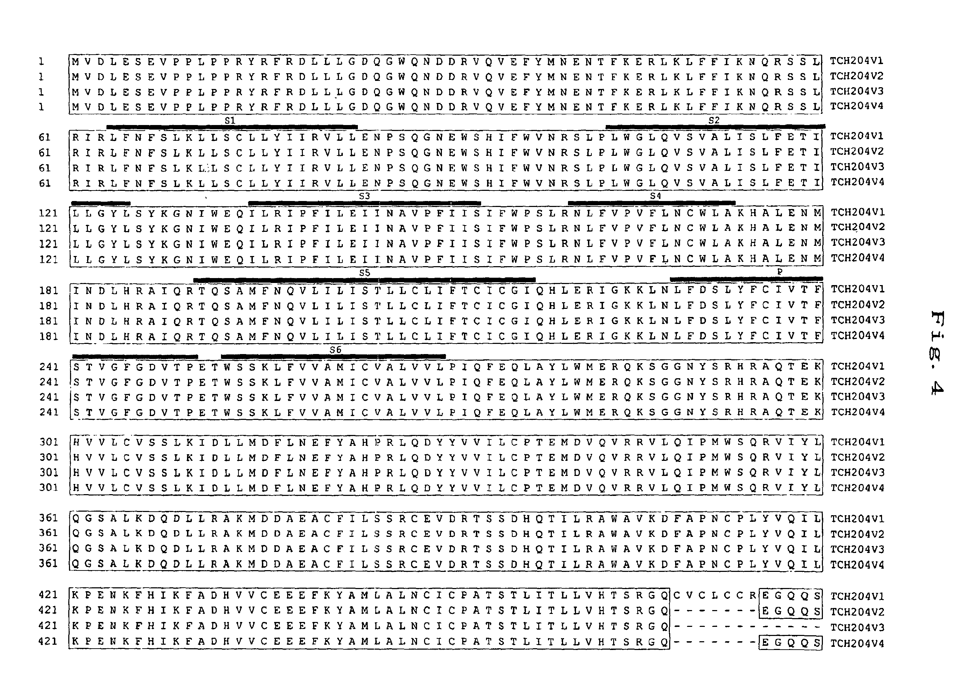

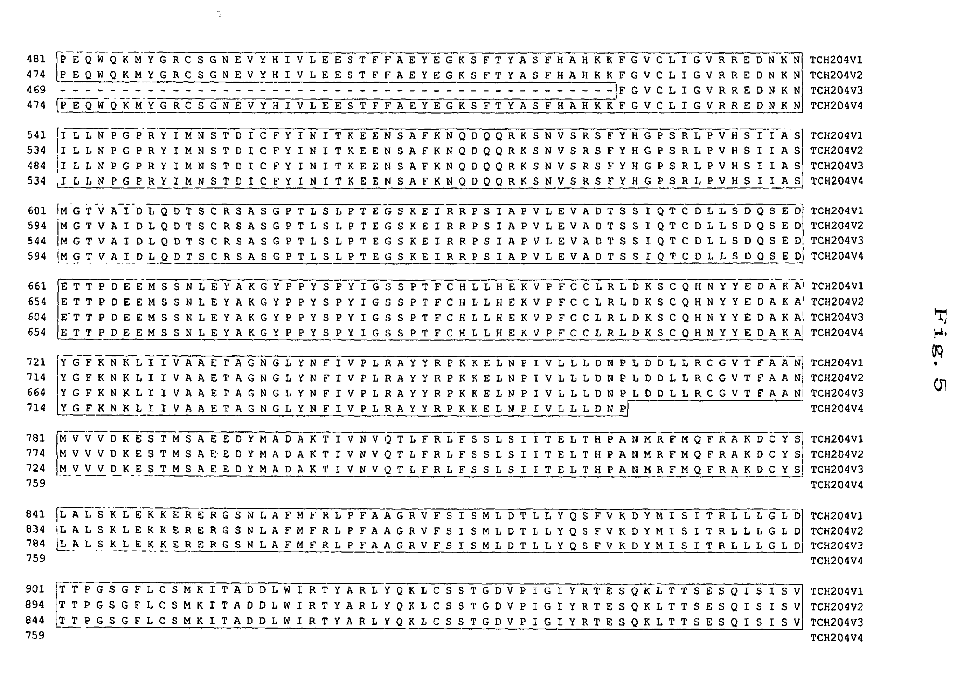

Fig. 4 is a drawing that shows a comparison of the amino acid sequences among human TCH204 variant 1 protein, human TCH204 variant 2 protein, human TCH204 variant 3 protein and human TCH204 variant 4 protein. In the figure, TCH204V1, TCH204V2, TCH204V3 and TCH204V4 represent the amino acid sequence of SEQ ID NO: 1, SEQ ID NO: 8, SEQ ID NO: 11 and SEQ ID NO: 15, respectively. S1 to S6 and P indicate transmembrane regions and a pore region, respectively. The amino acid residues embraced with square show amino acid residues identical to TCH204V1. (continued to Fig. 5)

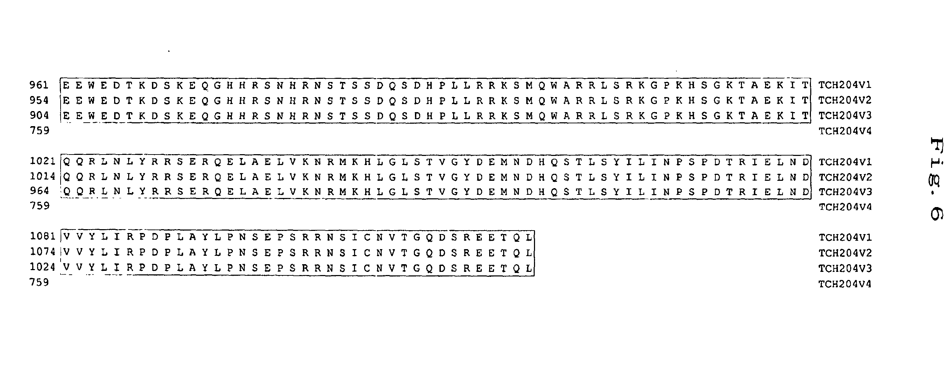

Fig. 5 is a drawing that shows a comparison of the amino acid sequences among human TCH204 variant 1 protein, human TCH204 variant 2 protein, human TCH204 variant 3 protein and human TCH204 variant 4 protein. In the figure, TCH204V1, TCH204V2, TCH204V3 and TCH204V4 represent the amino acid sequence of SEQ ID NO: 1, SEQ ID NO: 8, SEQ ID NO: 11 and SEQ ID NO: 15, respectively. S1 to S6 and P indicate transmembrane regions and a pore region, respectively. The amino acid residues embraced with square show amino acid residues identical to TCH204V1. (continued from Fig. 4 and continued to Fig. 6)
Fig. 6 is a drawing that shows a comparison of the amino acid sequences among human TCH204 variant 1 protein, human TCH204 variant 2 protein, human TCH204 variant 3 protein and human TCH204 variant 4 protein. In the figure, TCH204V1, TCH204V2, TCH204V3 and TCH204V4 represent the amino acid sequence of SEQ ID NO: 1, SEQ ID NO: 8, SEQ ID NO: 11 and SEQ ID NO: 15, respectively. S1 to S6 and P indicate transmembrane regions and a pore region, respectively. The amino acid residues embraced with square show amino acid residues identical to TCH204V1. (continued from Fig. 5)

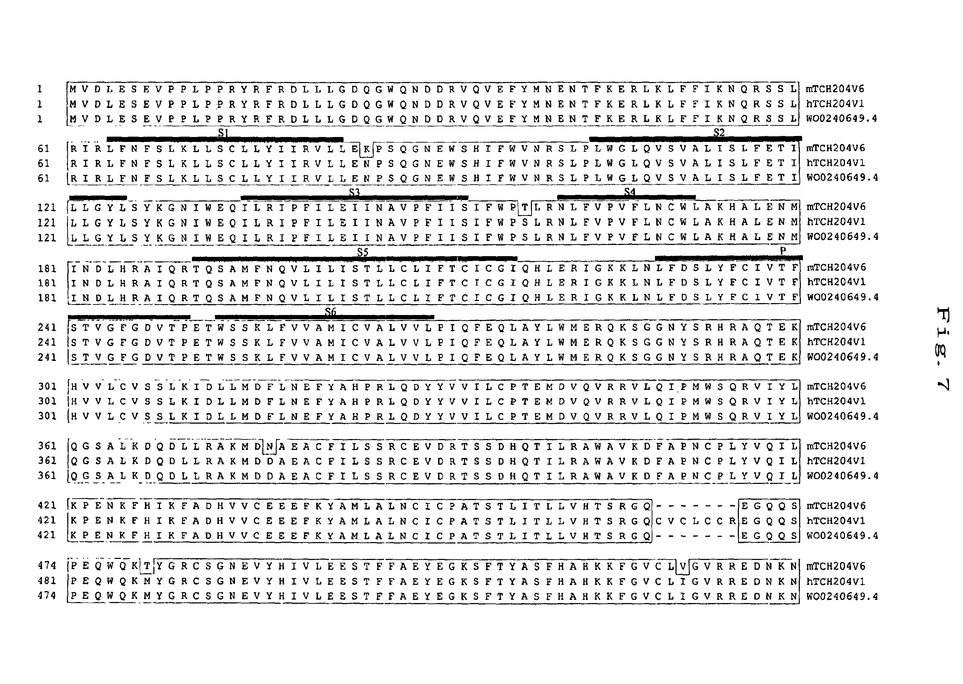

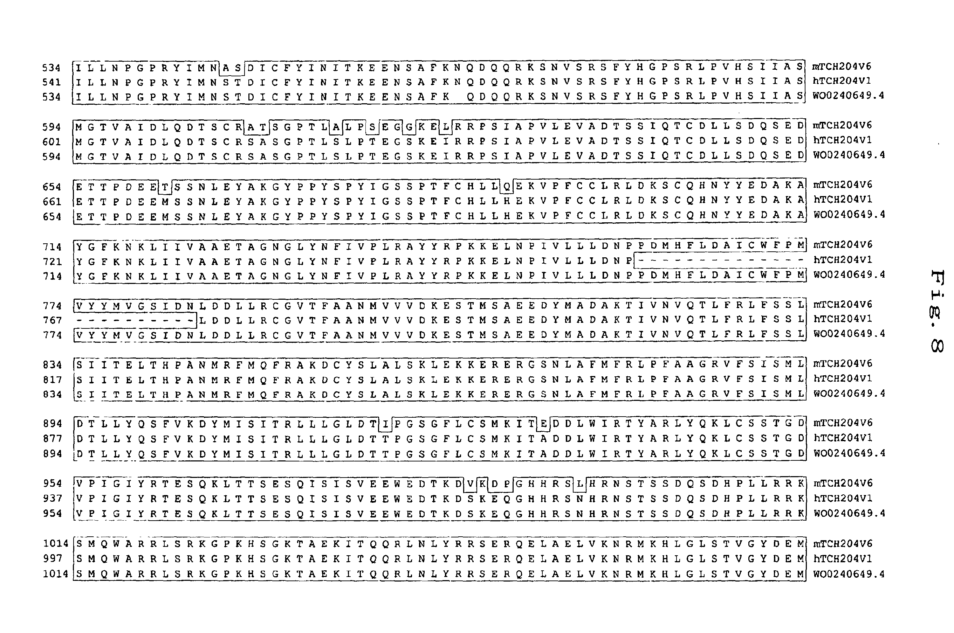

Fig. 7 is a drawing that shows a comparison of the amino acid sequences among mouse TCH204 variant 6 protein, human TCH204 variant 1 protein and human Slot 4 protein. In the figure, mTCH204V6, hTCH204V 1 and WO0240649.4 represent the amino acid sequence of SEQ ID NO: 18, SEQ ID NO: 1 and Slot 4, respectively. S1 to S6 and P indicate transmembrane regions and a pore region, respectively. The amino acid residues embraced with square show amino acid residues identical to mTCH204V6. (continued to Fig. 8)

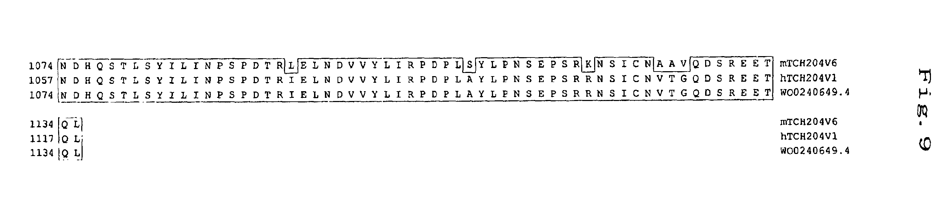

Fig. 8 is a drawing that shows a comparison of the amino acid sequences among mouse TCH204 variant 6 protein, human TCH204 variant 1 protein and human Slot 4 protein. In the figure, mTCH204V6, hTCH204V1 and WO0240649.4 represent the amino acid sequence of SEQ ID NO: 18, SEQ ID NO: 1 and Slot 4, respectively. S 1 to S6 and P indicate transmembrane regions and a pore region, respectively. The amino acid residues embraced with square show amino acid residues identical to mTCH204V6. (continued from Fig. 7 and continued to Fig. 9)
Fig. 9 is a drawing that shows a comparison of the amino acid sequences among mouse TCH204 variant 6 protein, human TCH204 variant 1 protein and human Slot 4 protein. In the figure, mTCH204V6, hTCH204V1 and WO0240649.4 represent the amino acid sequence of SEQ ID NO: 18, SEQ ID NO: 1 and Slot 4, respectively. S 1 to S6 and P indicate transmembrane regions and a pore region, respectively. The amino acid residues embraced with square show amino acid residues identical to mTCH204V6. (continued from Fig. 8)

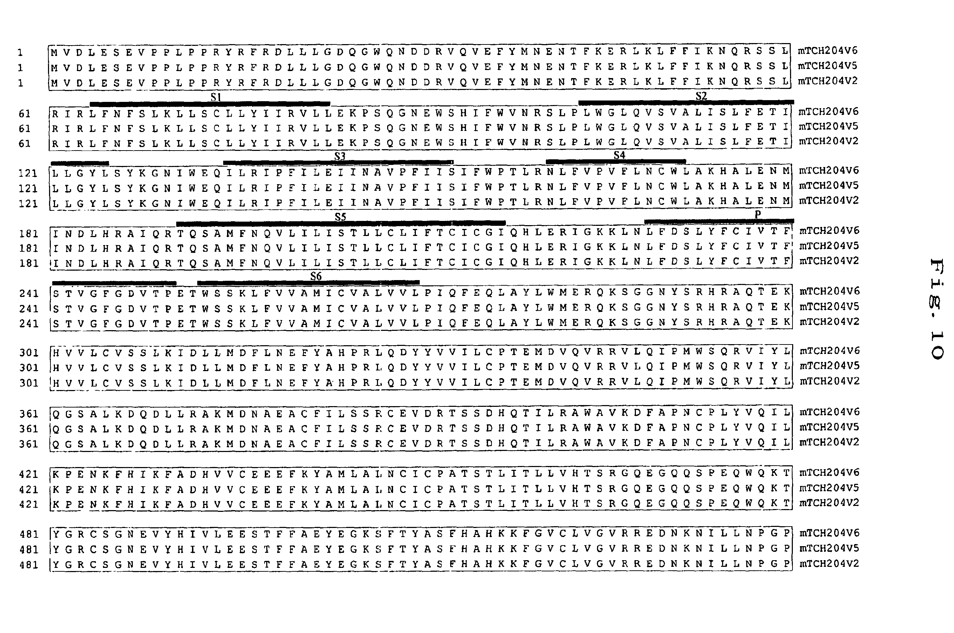

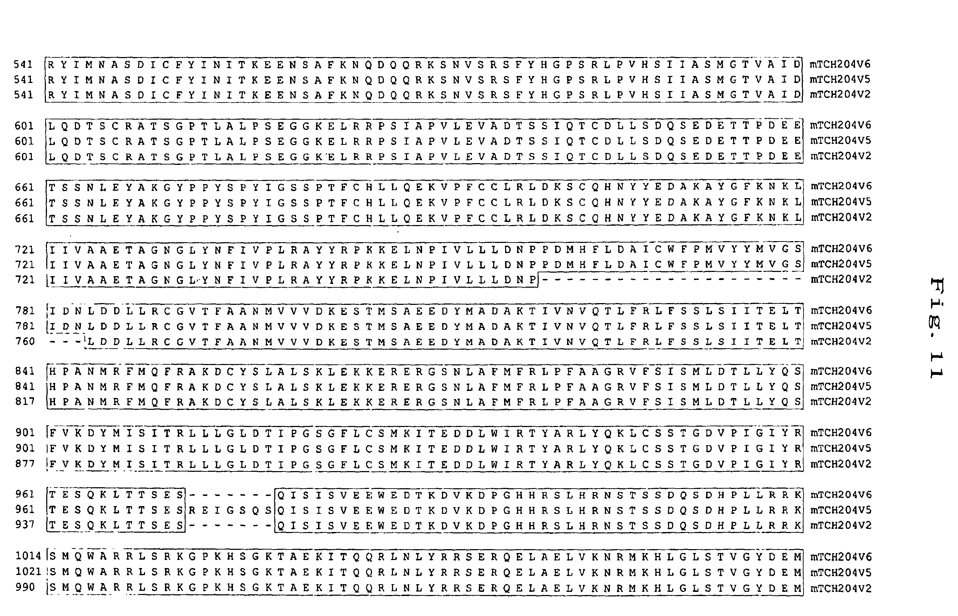

Fig. 10 is a drawing that shows a comparison of the amino acid sequences among mouse TCH204 variant 6 protein, mouse TCH204 variant 5 protein and mouse TCH204 variant 2 protein. In the figure, mTCH204V6, mTCH204V5 and mTCH204V2 represent the amino acid sequence of SEQ ID NO: 18, SEQ ID NO: 23 and SEQ ID NO: 26, respectively. S 1 to S6 and P indicate transmembrane regions and a pore region, respectively. The amino acid residues embraced with square show amino acid residues identical to mTCH204V6. (continued to Fig. 11)

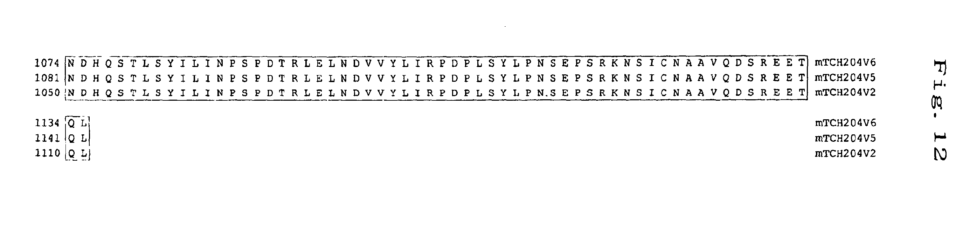

Fig. 11 is a drawing that shows a comparison of the amino acid sequences among mouse TCH204 variant 6 protein, mouse TCH204 variant 5 protein and mouse TCH204 variant 2 protein. In the figure, mTCH204V6, mTCH204V5 and mTCH204V2 represent the amino acid sequence of SEQ ID NO: 18, SEQ ID NO: 23 and SEQ ID NO: 26, respectively. S 1 to S6 and P indicate transmembrane regions and a pore region, respectively. The amino acid residues embraced with square show amino acid residues identical to mTCH204V6. (continued from Fig. 11 and continued to Fig. 12)
Fig. 12 is a drawing that shows a comparison of the amino acid sequences among mouse TCH204 variant 6 protein, mouse TCH204 variant 5 protein and mouse TCH204 variant 2 protein. In the figure, mTCH204V6, mTCH204V5 and mTCH204V2 represent the amino acid sequence of SEQ ID NO: 18, SEQ ID NO: 23 and SEQ ID NO: 26, respectively. S1 to S6 and P indicate transmembrane regions and a pore region, respectively. The amino acid residues embraced with square show amino acid residues identical to mTCH204V6. (continued from Fig. 11)

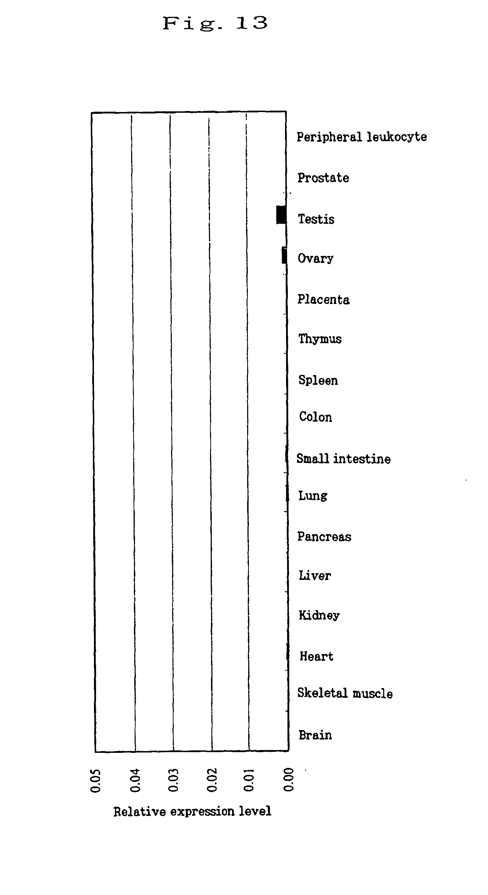

Fig. 13 is a drawing that shows an expression level of human TCH204 variant 1 gene in various human normal tissues derived cDNA. The relative expression level represented by ordinate indicates values multiplying the values obtained by dividing the expression level of the gene encoding the human TCH204 variant 1 per unit volume of cDNA (copies/µl) into the expression level of the GAPDH gene per unit volume of cDNA (copies/µl) by 100.

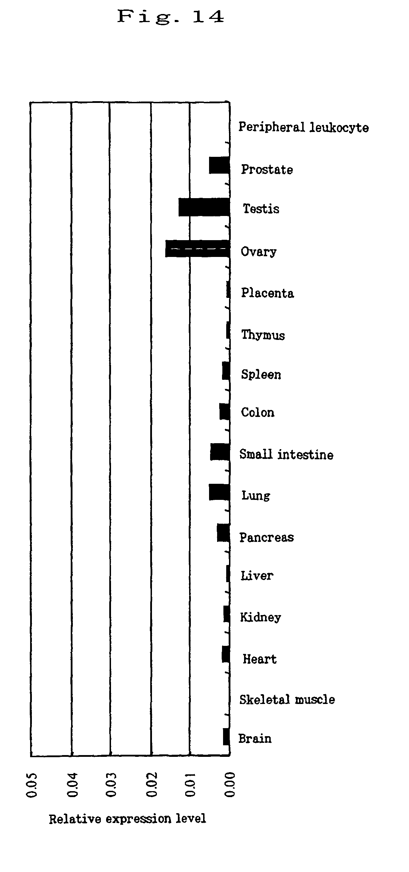

Fig. 14 is a drawing that shows an expression level of human TCH204 variant 2 gene in various human normal tissues derived cDNA. The relative expression level represented by ordinate indicates values multiplying the values obtained by dividing the expression level of the gene encoding the human TCH204 variant 2 per unit volume of cDNA (copies/µl) into the expression level of the GAPDH gene per unit volume of cDNA (copies/µl) by 100.

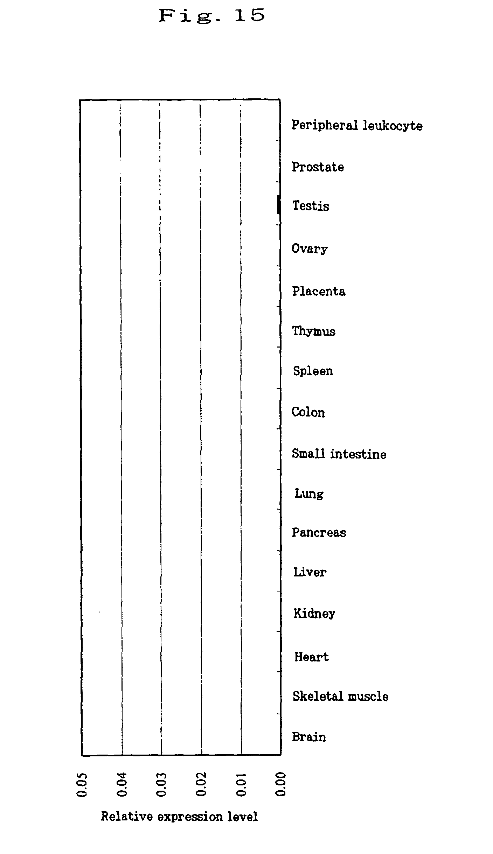

Fig. 15 is a drawing that shows an expression level of human TCH204 variant 3 gene in various human normal tissues derived cDNA. The relative expression level represented by ordinate indicates values multiplying the values obtained by dividing the expression level of the gene encoding the human TCH204 variant 3 per unit volume of cDNA (copies/µl) into the expression level of the GAPDH gene per unit volume of cDNA (copies/µl) by 100.

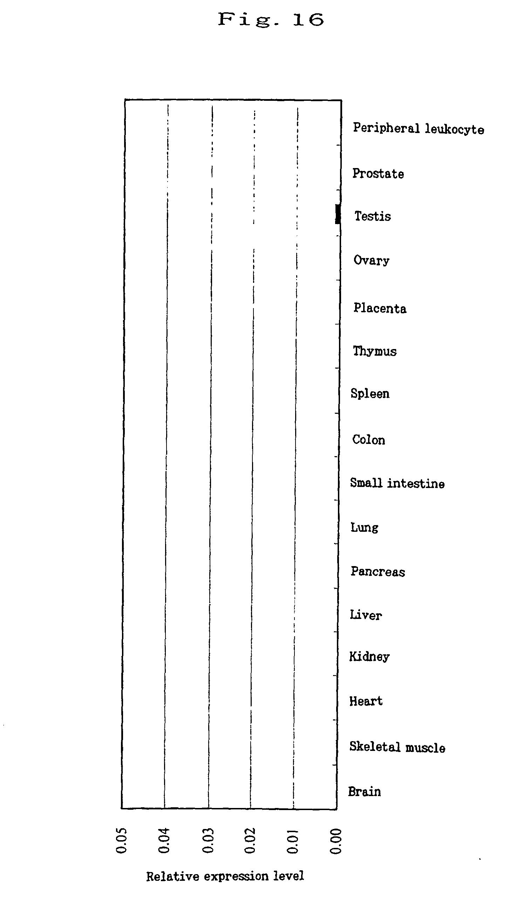

Fig. 16 is a drawing that shows an expression level of human TCH204 variant 4 gene in various human normal tissues derived cDNA. The relative expression level represented by ordinate indicates values multiplying the values obtained by dividing the expression level of the gene encoding the human TCH204 variant 4 per unit volume of cDNA (copies/µl) into the expression level of the GAPDH gene per unit volume of cDNA (copies/µl) by 100.

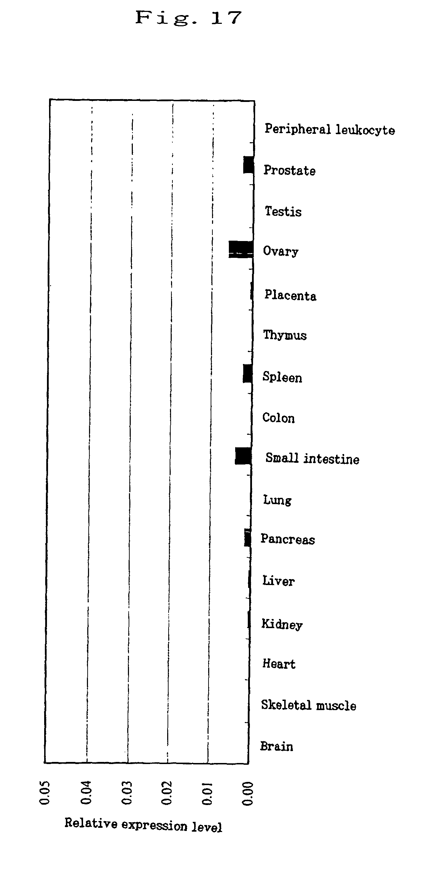

Fig. 17 is a drawing that shows an expression level of human TCH204 variant 5 gene in various human normal tissues derived cDNA. The relative expression level represented by ordinate indicates values multiplying the values obtained by dividing the expression level of the gene encoding the human TCH204 variant 5 per unit volume of cDNA (copies/µl) into the expression level of the GAPDH gene per unit volume of cDNA (copies/µl) by 100.

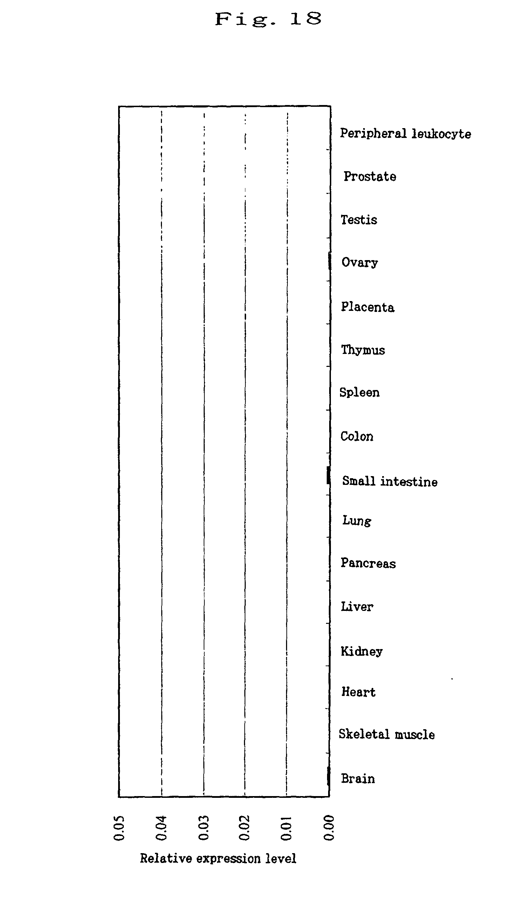

Fig. 18 is a drawing that shows an expression level of human TCH204 variant 6 gene in various human normal tissues derived cDNA. The relative expression level represented by ordinate indicates values multiplying the values obtained by dividing the expression level of the gene encoding the human TCH204 variant 6 per unit volume of cDNA (copies/µl) into the expression level of the GAPDH gene per unit volume of cDNA (copies/µl) by 100.

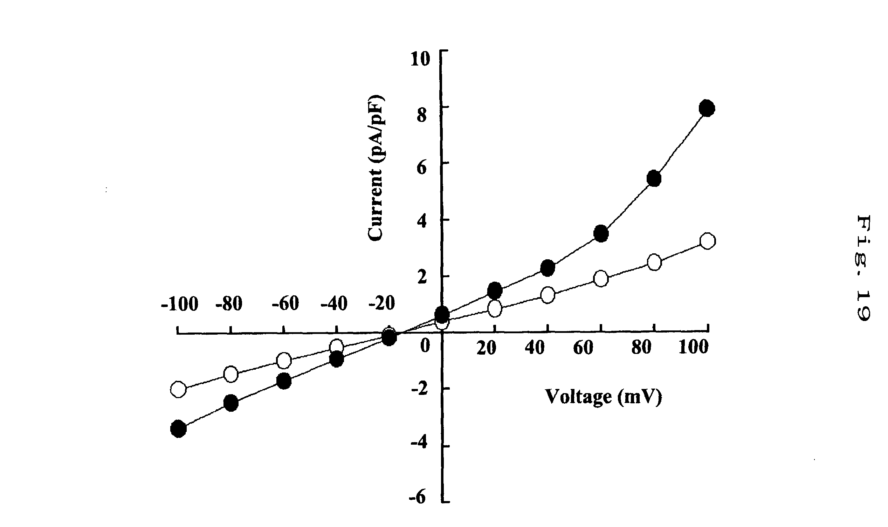

Fig. 19 is a drawing that shows a current-potential curve where the TCH204 variant 2 was transiently expressed in CHO-K1 cells. The membrane potential was held at -80 mV. Closed circles and open circles represent average values in the cells, in which the human TCH204 variant 2 vector was introduced (7 examples), and the cells, in which only the vector was introduced (5 examples), respectively.

### BEST MODE FOR CARRYING OUT THE INVENTION

The protein containing the same or substantially the same amino acid sequence as the amino acid sequence represented by SEQ ID NO: 1, SEQ ID NO: 8, SEQ ID NO: 11 or SEQ ID NO: 15 (hereinafter referred to as the protein of the present invention or the protein used in the present invention) may be any protein derived from any cells (e.g., liver cells, splenocytes, nerve cells, glial cells, pancreatic β cells, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophage, T cells, B cells, natural killer cells, mast cells, neutrophil, basophil, eosinophil, monocyte), megakaryocyte, synovial cells, chondrocytes, bone cells, osteoblasts, osteoclasts, mammary gland cells, hepatocytes or interstitial cells, the corresponding precursor cells, stem cells, cancer cells, etc.), or any tissues where such cells are present, e.g., brain or any region of the brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, peripheral blood, prostate, testis, ovary, placenta, uterus, bone, joint, skeletal muscle, etc. from human and other mammals (e.g., guinea pigs, rats, mice, chicken, rabbits, swine, sheep, bovine, monkeys, etc.). The protein may also be a synthetic protein.

The amino acid sequence which has substantially the same amino acid sequence as that represented by SEQ ID NO: 1 includes an amino acid sequence having at least about 75% homology, preferably at least about 80% homology, preferably at least about 85% homology, preferably at least about 90% homology, preferably at least about 95% homology, to the amino acid sequence represented by SEQ ID NO: 1.

Examples of the protein which contains substantially the same amino acid sequence as that represented by SEQ ID NO: 1 preferably include a protein containing substantially the same amino acid sequence as that represented by SEQ ID NO: 1 and having the activity substantially equivalent to the amino acid sequence represented by SEQ ID NO: 1, etc.

The amino acid sequence which has substantially the same amino acid sequence as that represented by SEQ ID NO: 8 includes an amino acid sequence having at least about 75% homology, preferably at least about 80% homology, preferably at least about 85% homology, preferably at least about 90% homology, preferably at least about 95% homology, preferably at least about 97% homology, to the amino acid sequence represented by SEQ ID NO: 8.

Examples of the protein which contains substantially the same amino acid sequence as that shown by SEQ ID NO: 8 preferably include a protein containing substantially the same amino acid sequence as that shown by SEQ ID NO: 8 and having the activity substantially equivalent to the amino acid sequence represented by SEQ ID NO: 8, etc. Specifically, it includes a protein containing the amino acid sequence represented by SEQ ID NO: 18, a protein containing the amino acid sequence represented by SEQ ID NO: 23, a protein containing the amino acid sequence represented by SEQ ID NO: 26, and the like.

The amino acid sequence which has substantially the same amino acid sequence as that represented by SEQ ID NO: 11 includes an amino acid sequence having at least about 75% homology, preferably at least about 80% homology, preferably at least about 85% homology, preferably at least about 90% homology, preferably at least about 95% homology, to the amino acid sequence represented by SEQ ID NO:11.

Examples of the protein which contains substantially the same amino acid sequence as that represented by SEQ ID NO: 11 preferably include a protein containing substantially the same amino acid sequence as that represented by SEQ ID NO: 11 and having the activity substantially equivalent to the amino acid sequence represented by SEQ ID NO: 11, etc.

The amino acid sequence which has substantially the same amino acid sequence as that represented by SEQ ID NO: 15 includes an amino acid sequence having at least about 80% homology, preferably at least about 85% homology, preferably at least about 90% homology, preferably at least about 95% homology, to the amino acid sequence represented by SEQ ID NO: 15.

Examples of the protein which contains substantially the same amino acid sequence as that represented by SEQ ID NO: 15 preferably include a protein containing substantially the same amino acid sequence as that represented by SEQ ID NO: 15 and having the activity substantially equivalent to the amino acid sequence represented by SEQ ID NO: 15, etc.

Examples of the substantially equivalent activity include transmission of K⁺ ion, etc. The term "substantially equivalent" is used to mean that the nature of the activity is the same. Therefore, although it is preferred that the transmission of K⁺ ion be equivalent (e.g., about 0.01- to about 100-fold, preferably about 0.1- to about 10-fold, more preferably about 0.5- to about 2-fold), quantitative factors such as a level of the activity, a molecular weight of the protein, etc. may differ.

The transmission of K⁺ ion can be determined according to a publicly known method with some modifications, for example, by the methods described in Receptors and Channels, Vol. 6, pp. 337 - 350, 1999 or modified methods thereof.

Proteins containing the following amino acid sequences, which are so-called muteins, are used as the protein of the present invention: (1) (i) amino acid sequences represented by SEQ ID NO: 1, wherein at least 1 or 2 amino acids (approximately 1 to 300 amino acids, preferably approximately 1 to 200 amino acids, preferably approximately 1 to 150 amino acids, preferably approximately 1 to 100 amino acids, preferably approximately 1 to 50 amino acids, preferably approximately 1 to 30 amino acids, preferably approximately 1 to 10 amino acids, most preferably several (1 to 5) amino acids) are deleted; (ii) amino acid sequences represented by SEQ 1D NO: 1, to which at least 1 or 2 amino acids (approximately 1 to 300 amino acids, preferably approximately 1 to 200 amino acids, preferably approximately 1 to 150 amino acids, preferably approximately 1 to 100 amino acids, preferably approximately 1 to 50 amino acids, preferably approximately 1 to 30 amino acids, preferably approximately 1 to 10 amino acids, most preferably several (1 to 5) amino acids) are added; (iii) amino acid sequences represented by SEQ ID NO: 1, in which at least 1 or 2 amino acids (approximately 1 to 300 amino acids, preferably approximately 1 to 200 amino acids, preferably approximately 1 to 150 amino acids, preferably approximately 1 to 100 amino acids, preferably approximately 1 to 50 amino acids, preferably approximately 1 to 30 amino acids, preferably approximately 1 to 10 amino acids, most preferably several (1 to 5) amino acids) are inserted; (iv) amino acid sequences represented by SEQ ID NO: 1, wherein at least 1 or 2 amino acids (approximately 1 to 300 amino acids, preferably approximately 1 to 200 amino acids, preferably approximately 1 to 150 amino acids, preferably approximately 1 to 100 amino acids, preferably approximately 1 to 50 amino acids, preferably approximately 1 to 30 amino acids, preferably approximately 1 to 10 amino acids, most preferably several (1 to 5) amino acids) are substituted by other amino acids; or (v) combination of the amino acid sequences described in the above, (2) (i) amino acid sequences represented by SEQ ID NO: 8, wherein at least 1 or 2 amino acids (approximately 1 to 300 amino acids, preferably approximately 1 to 200 amino acids, preferably approximately 1 to 150 amino acids, preferably approximately 1 to 100 amino acids, preferably approximately 1 to 50 amino acids, preferably approximately 1 to 30 amino acids, preferably approximately 1 to 10 amino acids, most preferably several (1 to 5) amino acids) are deleted; (ii) amino acid sequences represented by SEQ ID NO: 8, to which at least 1 or 2 amino acids (approximately 1 to 300 amino acids, preferably approximately 1 to 200 amino acids, preferably approximately 1 to 150 amino acids, preferably approximately 1 to 100 amino acids, preferably approximately 1 to 50 amino acids, preferably approximately 1 to 30 amino acids, preferably approximately 1 to 10 amino acids, most preferably several (1 to 5) amino acids) are added; (iii) amino acid sequences represented by SEQ ID NO: 8, in which at least 1 or 2 amino acids (approximately 1 to 300 amino acids, preferably approximately 1 to 200 amino acids, preferably approximately 1 to 150 amino acids, preferably approximately 1 to 100 amino acids, preferably approximately 1 to 50 amino acids, preferably approximately 1 to 30 amino acids, preferably approximately 1 to 10 amino acids, most preferably several (1 to 5) amino acids) are inserted; (iv) amino acid sequences represented by SEQ ID NO: 8, wherein at least I or 2 amino acids (approximately 1 to 300 amino acids, preferably approximately 1 to 200 amino acids, preferably approximately 1 to 150 amino acids, preferably approximately 1 to 100 amino acids, preferably approximately 1 to 50 amino acids, preferably approximately 1 to 30 amino acids, preferably approximately 1 to 10 amino acids, most preferably several (1 to 5) amino acids) are substituted by other amino acids; or (v) combination of the amino acid sequences described in the above, (3) (i) amino acid sequences represented by SEQ ID NO: 11, wherein at least 1 or 2 amino acids (approximately 1 to 300 amino acids, preferably approximately 1 to 200 amino acids, preferably approximately 1 to 150 amino acids, preferably approximately 1 to 100 amino acids, preferably approximately 1 to 50 amino acids, preferably approximately 1 to 30 amino acids, preferably approximately 1 to 10 amino acids, most preferably several (1 to 5) amino acids) are deleted; (ii) amino acid sequences represented by SEQ ID NO: 11, to which at least 1 or 2 amino acids (approximately 1 to 300 amino acids, preferably approximately 1 to 200 amino acids, preferably approximately 1 to 150 amino acids, preferably approximately I to 100 amino acids, preferably approximately 1 to 50 amino acids, preferably approximately 1 to 30 amino acids, preferably approximately 1 to 10 amino acids, most preferably several (1 to 5) amino acids) are added; (iii) amino acid sequences represented by SEQ ID NO: 11, in which at least 1 or 2 amino acids (approximately 1 to 300 amino acids, preferably approximately 1 to 200 amino acids, preferably approximately 1 to 150 amino acids, preferably approximately 1 to 100 amino acids, preferably approximately 1 to 50 amino acids, preferably approximately 1 to 30 amino acids, preferably approximately 1 to 10 amino acids, most preferably several (1 to 5) amino acids) are inserted; (iv) amino acid sequences represented by SEQ ID NO: 11, wherein at least 1 or 2 amino acids (approximately 1 to 300 amino acids, preferably approximately 1 to 200 amino acids, preferably approximately 1 to 150 amino acids, preferably approximately 1 to 100 amino acids, preferably approximately 1 to 50 amino acids, preferably approximately 1 to 30 amino acids, preferably approximately 1 to 10 amino acids, most preferably several (1 to 5) amino acids) are substituted by other amino acids; or (v) combination of the amino acid sequences described in the above, and (4) (i) amino acid sequences represented by SEQ ID NO: 15, wherein at least 1 or 2 amino acids (approximately 1 to 300 amino acids, preferably approximately 1 to 200 amino acids, preferably approximately 1 to 150 amino acids, preferably approximately 1 to 100 amino acids, preferably approximately 1 to 50 amino acids, preferably approximately 1 to 30 amino acids, preferably approximately 1 to 10 amino acids, most preferably several (1 to 5) amino acids) are deleted; (ii) amino acid sequences represented by SEQ ID NO: 15, to which at least 1 or 2 amino acids (approximately 1 to 300 amino acids, preferably approximately 1 to 200 amino acids, preferably approximately 1 to 150 amino acids, preferably approximately 1 to 100 amino acids, preferably approximately 1 to 50 amino acids, preferably approximately 1 to 30 amino acids, preferably approximately 1 to 10 amino acids, most preferably several (1 to 5) amino acids) are added; (iii) amino acid sequences represented by SEQ ID NO: 15, in which at least 1 or 2 amino acids (approximately 1 to 300 amino acids, preferably approximately 1 to 200 amino acids, preferably approximately 1 to 150 amino acids, preferably approximately 1 to 100 amino acids, preferably approximately 1 to 50 amino acids, preferably approximately 1 to 30 amino acids, preferably approximately 1 to 10 amino acids, most preferably several (1 to 5) amino acids) are inserted; (iv) amino acid sequences represented by SEQ ID NO: 15, wherein at least 1 or 2 amino acids (approximately 1 to 300 amino acids, preferably approximately 1 to 200 amino acids, preferably approximately 1 to 150 amino acids, preferably approximately 1 to 100 amino acids, preferably approximately 1 to 50 amino acids, preferably approximately 1 to 30 amino acids, preferably approximately 1 to 10 amino acids, most preferably several (1 to 5) amino acids) are substituted by other amino acids; or (v) combination of the amino acid sequences described in the above.

As described above, where an amino acid sequence is inserted, deleted or substituted, location of insertion, deletion or substitution is not restricted.

Throughout the present specification, the proteins are represented in accordance with the conventional way of describing peptides, that is, the N-terminus (amino terminus) at the left hand and the C-terminus (carboxyl terminus) at the right hand. In the proteins of the present invention including the proteins containing the amino acid sequence represented by SEQ ID NO: 1, the C-terminus may be in any form of a carboxyl group (-COOH), a carboxylate (-COO⁻), an amide (-CONH₂) or an ester (-COOR).

Examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, a-naphthyl, etc.; a C₇₋₁₄ aralkyl group such as a phenyl-C₁₋₂-alkyl group, e.g., benzyl, phenethyl, etc., or an a-naphthyl-C₁₋₂-alkyl group such as a-naphthylmethyl, etc.; pivaloyloxymethyl and the like.

Where the protein of the present invention has a carboxyl group (or a carboxylate) at a position other than the C-terminus, it may be amidated or esterified and such an amide or ester is also included within the protein of the present invention. The ester group may be the same group as that described with respect to the C-terminus described above.

Furthermore, examples of the protein of the present invention include variants of the above proteins, wherein the amino group at the N-terminal amino acid residue (e.g., methionine residue) is protected with a protecting group (for example, a C₁₋₆ acyl group such as a C₁₋₆ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₁₋₆ alkanoyl group, e.g., formyl group, acetyl group, etc.), or conjugated proteins such as glycoproteins bound to sugar chains.

Specific examples of the protein of the present invention include a protein containing an amino acid sequence represented by SEQ ID NO: 1, a protein containing an amino acid sequence represented by SEQ ID NO: 8, a protein containing an amino acid sequence represented by SEQ ID NO: 11, a protein containing an amino acid sequence represented by SEQ ID NO: 15, a protein containing an amino acid sequence represented by SEQ ID NO: 18, a protein containing an amino acid sequence represented by SEQ ID NO: 23, a protein containing an amino acid sequence represented by SEQ ID NO: 26, etc.

As partial peptides of the protein of the present invention, any partial peptide can be used so long as it can be a partial peptide of the protein of the present invention and preferably possess the same properties as that of the protein of the present invention described above.

Specifically, among the constituent amino acid sequence of the protein used in the present invention, a peptide, which comprises an amino acid sequence having at least 20 residues, preferably at least 50 residues, further preferably at least 70 residues, more preferably at least 100 residues and most preferably at least 200 residues, may be used.

The partial peptide used in the present invention may contain an amino acid sequence, wherein at least 1 or 2 amino acids (preferably approximately 1 to 10 amino acids, more preferably several (1 to 5) amino acids) are deleted; to which at least 1 or 2 amino acids (preferably approximately 1 to 20 amino acids, more preferably approximately 1 to 10 amino acids, and most preferably several (1 to 5) amino acids) are added; in which at least 1 or 2 amino acids (preferably approximately 1 to 10 amino acids, more preferably several and most preferably approximately 1 to 5 amino acids) are inserted; wherein at least 1 or 2 amino acids (preferably approximately 1 to 10 amino acids, more preferably several (1 to 5) amino acids) are substituted by other amino acids.

The partial peptide of the present invention includes, for example, a peptide having the 1st through the 63rd, the 84th through the 103rd, or the 271st through the 1118th amino acid sequence in the amino acid sequence represented by SEQ ID NO: 1; a peptide having the 1st through the 63rd, the 84th through the 103rd, or the 271st through the 1111st amino acid sequence in the amino acid sequence represented by SEQ ID NO: 8; a peptide having the 1st through the 63rd, the 84th through the 103rd, or the 271 st through the 1061st amino acid sequence in the amino acid sequence represented by SEQ ID NO: 11; a peptide having the 1st through the 63rd, the 84th through the 103rd, or the 271st through the 759th amino acid sequence in the amino acid sequence represented by SEQ ID NO: 15; a peptide having the 1st through the 63rd, the 84th through the 103rd, or the 271st through the 1135th amino acid sequence in the amino acid sequence represented by SEQ ID NO: 18; a peptide having the 1st through the 63rd, the 84th through the 103rd, or the 271 st through the 1142nd amino acid sequence in the amino acid sequence represented by SEQ ID NO: 23; a peptide having the 1st through the 63rd, the 84th through the 103rd, or the 271st through the 1111 st amino acid sequence in the amino acid sequence represented by SEQ ID NO: 26; and the like.

In the partial peptide of the present invention, the C-terminus may be any of a carboxyl group (-COOH), a carboxylate (-COO⁻), an amide (-CONH₂) or an ester (-COOR).

As well as the aforementioned protein used in the present invention, the partial peptide of the present invention further includes those having carboxyl group (or carboxylate) apart from the C-terminus, those in which the amino group of the amino acid residue of the N-terminal methionine residue is protected by a protecting group, those in which the N-terminal residue is cleaved in vivo and the produced glutamine residue is pyroglutaminated, those in which substituents on the side chains of amino acids in the molecule are protected by appropriate protecting groups, conjugated peptides such as so-called glycoproteins, to which sugar chains are bound, and the like.

The partial peptide used in the present invention can also be used as an antigen for preparing an antibody. For example, to prepare the antibody of the present invention, which will be described below, the partial peptide includes a peptide having the 22nd through the 35th, the 84th through the 103rd, the 282nd through the 302nd, or the 656th through the 673rd amino acid sequence in the amino acid sequence represented by SEQ ID NO: 1; a peptide having the 22nd through the 35th, the 84th through the 103rd, the 282nd through the 302nd, or the 649th through the 666th amino acid sequence in the amino acid sequence represented by SEQ ID NO: 8; a peptide having the 22nd through the 35th, the 84th through the 103rd, the 282nd through the 302nd, or the 599th through the 616th amino acid sequence in the amino acid sequence represented by SEQ ID NO: 11; a peptide having the 22nd through the 35th, the 84th through the 103rd, the 282nd through the 302nd, or the 649th through the 666th amino acid sequence in the amino acid sequence represented by SEQ ID NO: 15; a peptide having the 22nd through the 35th, the 84th through the 103rd, the 282nd through the 302nd, or the 649th through the 666th amino acid sequence in the amino acid sequence represented by SEQ ID NO: 18; a peptide having the 22nd through the 35th, the 84th through the 103rd, the 282nd through the 302nd, or the 649th through the 666th amino acid sequence in the amino acid sequence represented by SEQ ID NO: 23; a peptide having the 22nd through the 35th, the 84th through the 103rd, the 282nd through the 302nd, or the 649th through the 666th amino acid sequence in the amino acid sequence represented by SEQ ID NO: 26; and the like.

For salts of the protein or the partial peptide of the present invention, preferred are salts with physiologically acceptable acids (e.g., inorganic acids, organic acids) or basses (e.g., alkali metal salts), especially physiologically acceptable acid addition salts. Examples of the salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein of the present invention or salts thereof may be manufactured by a publicly known method used to purify a protein from human and other mammalian cells or tissues described above, or by culturing a transformant that contains the DNA encoding the protein, as will be later described. Furthermore, the protein or its salts may also be manufactured by the methods for synthesizing proteins or by modifications thereof, which will also be described hereinafter.

Where the protein or its salts are manufactured from human or other mammalian tissues or cells, human or other mammalian tissues or cells are homogenized, then extracted with an acid or the like, and the extract obtained is isolated and purified by a combination of chromatography techniques such as reverse phase chromatography, ion exchange chromatography, and the like.

To synthesize the protein of the present invention, its partial peptide, or salts or amides thereof according to the present invention, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenylhydroxymethyl) phenoxy resin, 4-(2',4'-dimethoxyphenyl-Fmoc-aminoethyl) phenoxy resin, etc. Using these resins, amino acids in which α-amino groups and functional groups on the side chains are appropriately protected are condensed on the resin in the order of the sequence of the objective protein according to various condensation methods publicly known in the art. At the end of the reaction, the receptor protein is cut out from the resin and at the same time, the protecting groups are removed. Then, intramolecular disulfide bond-forming reaction is performed in a highly diluted solution to obtain the objective protein or peptide, or amides thereof.

For condensation of the protected amino acids described above, a variety of activation reagents for protein synthesis may be used, and carbodiimides are particularly preferable. Examples of such carbodiimides include DCC, N,N'-diisopropylcarbodiimide, N-ethyl-N'-(3-dimethylaminoprolyl) carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole.

Examples of the protecting groups used to protect the amino groups of the starting compounds include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

A hydroxyl group of serine can be protected through, for example, its esterification or etherification. Examples of groups appropriately used for the esterification include a lower alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting compounds include the corresponding acid anhydrides, azides, activated esters (esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). As the activated amino acids, in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethane-sulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the protein, for example, the α-carboxyl group of the carboxy terminal amino acid is first protected by amidation; the peptide (protein) chain is then extended from the amino group side to a desired length. Thereafter, a protein in which only the protecting group of the N-terminal α-amino group in the peptide chain has been eliminated from the protein and a protein in which only the protecting group of the C-terminal carboxyl group has been eliminated are prepared. The two proteins are condensed in a mixture of the solvents described above. The details of the condensation reaction are the same as described above. After the protected protein obtained by the condensation is purified, all the protecting groups are eliminated by the method described above to give the desired crude protein. This crude protein is purified by various known purification means. Lyophilization of the major fraction gives the amide of the desired protein.

To prepare the esterified protein, for example, the α-carboxyl group of the carboxy terminal amino acid is condensed with a desired alcohol to prepare the amino acid ester, which is followed by procedure similar to the preparation of the amidated protein above to give the ester form of the desired protein.

The partial peptide or its salts in the protein of the present invention can be manufactured by publicly known methods for peptide synthesis, or by cleaving the protein of the present invention with an appropriate peptidase. For the methods for peptide synthesis, for example, either solid phase synthesis or liquid phase synthesis may be used. That is, the partial peptide or amino acids that can construct the protein of the present invention are condensed with the remaining part. Where the product contains protecting groups, these protecting groups are removed to give the desired peptide. Publicly known methods for condensation and elimination of the protecting groups are described in (a) - (e) below.
(a) M. Bodanszky & M.A. Ondetti: Peptide Synthesis, Interscience Publishers, New York (1966)
(b) Schroeder & Luebke: The Peptide, Academic Press, New York (1965)
(c) Nobuo Izumiya, et al.: *Peptide Gosei-no-Kiso to Jikken* (Basics and experiments of peptide synthesis), published by Maruzen Co. (1975)
(d) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977)
(e) Haruaki Yajima, ed.: *Zoku lyakuhin no Kaihatsu* (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and re-crystallization to give the partial peptide of the present invention. When the partial peptide obtained by the above methods is in a free form, the peptide can be converted into an appropriate salt by a publicly known method; when the protein is obtained in a salt form, it can be converted into a free form by a publicly known method.

The polynucleotide encoding the protein of the present invention may be any polynucleotide so long as it contains the base sequence encoding the protein of the present invention described above. Preferred is DNA. The DNA may be any of genomic DNA, genomic DNA library, cDNA derived from the cells and tissues described above, cDNA library derived from the cells and tissues described above and synthetic DNA.

The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA or mRNA fraction prepared from the cells and tissues described above.

Specifically, the DNA encoding the protein of the present invention may be (1) (i) DNA having the base sequence shown by SEQ ID NO: 2, or DNA having the base sequence hybridizable to the base sequence represented by SEQ ID NO: 2 under highly stringent conditions and encoding a protein having the activities substantially equivalent to those of the protein containing the amino acid sequence represented by SEQ ID NO: 1, (ii) DNA having the base sequence shown by SEQ ID NO: 7, or DNA having the base sequence hybridizable to the base sequence represented by SEQ ID NO: 7 under highly stringent conditions and encoding a protein having the activities substantially equivalent to those of the protein containing the amino acid sequence represented by SEQ ID NO: 1; (2) (i) DNA having the base sequence shown by SEQ ID NO: 9, or DNA having the base sequence hybridizable to the base sequence represented by SEQ ID NO: 9 under highly stringent conditions and encoding a protein having the activities substantially equivalent to those of the protein containing the amino acid sequence represented by SEQ ID NO: 8, (ii) DNA having the base sequence shown by SEQ ID NO: 10, or DNA having the base sequence hybridizable to the base sequence represented by SEQ ID NO: 10 under highly stringent conditions and encoding a protein having the activities substantially equivalent to those of the protein containing the amino acid sequence represented by SEQ ID NO: 8; (3) (i) DNA having the base sequence shown by SEQ ID NO: 12, or DNA having the base sequence hybridizable to the base sequence represented by SEQ ID NO: 12 under highly stringent conditions and encoding a protein having the activities substantially equivalent to those of the protein containing the amino acid sequence represented by SEQ ID NO: 11, (ii) DNA having the base sequence shown by SEQ ID NO: 13, or DNA having the base sequence hybridizable to the base sequence represented by SEQ ID NO: 13 under highly stringent conditions and encoding a protein having the activities substantially equivalent to those of the protein containing the amino acid sequence represented by SEQ ID NO: 11; or (4) (i) DNA having the base sequence shown by SEQ ID NO: 16, or DNA having the base sequence hybridizable to the base sequence represented by SEQ ID NO: 16 under highly stringent conditions and encoding a protein having the activities substantially equivalent to those of the protein containing the amino acid sequence represented by SEQ ID NO: 15, (ii) DNA having the base sequence shown by SEQ ID NO: 17, or DNA having the base sequence hybridizable to the base sequence represented by SEQ ID NO: 17 under highly stringent conditions and encoding a protein having the activities substantially equivalent to those of the protein containing the amino acid sequence represented by SEQ ID NO: 15.

Examples of the DNA hybridizable to the base sequence represented by SEQ ID NO: 2 or SEQ ID NO: 7 under highly stringent conditions include DNA containing a base sequence having at least about 55% homology, preferably at least about 60% homology, preferably at least about 65% homology, preferably at least about 70% homology, preferably at least about 75% homology, preferably at least about 80% homology, preferably at least about 85% homology, preferably at least about 90% homology, and most preferably at least about 95% homology, to the base sequence represented by SEQ ID NO: 2 or SEQ ID NO: 7.

Examples of the DNA hybridizable to the base sequence represented by SEQ ID NO: 9 or SEQ ID NO: 10 under highly stringent conditions include DNA containing a base sequence having at least about 65% homology, preferably at least about 70% homology, preferably at least about 75% homology, preferably at least about 80% homology, preferably at least about 85% homology, preferably at least about 90% homology, and most preferably at least about 95% homology, to the base sequence represented by SEQ ID NO: 9 or SEQ ID NO: 10. Specifically, it includes a DNA containing the base sequence represented by SEQ ID NO: 19, a DNA containing the base sequence represented by SEQ ID NO: 24, a DNA containing the base sequence represented by SEQ ID NO: 27, or the like.

Examples of the DNA hybridizable to the base sequence represented by SEQ ID NO: 12 or SEQ ID NO: 13 under highly stringent conditions include DNA containing a base sequence having at least about 60% homology, preferably at least about 65% homology, preferably at least about 70% homology, preferably at least about 75% homology, preferably at least about 80% homology, preferably at least about 85% homology, preferably at least about 90% homology, and most preferably at least about 95% homology, to the base sequence represented by SEQ ID NO: 12 or SEQ ID NO: 13.

Examples of the DNA hybridizable to the base sequence represented by SEQ ID NO: 16 or SEQ ID NO: 17 under highly stringent conditions include DNA containing a base sequence having at least about 60% homology, preferably at least about 65% homology, preferably at least about 70% homology, preferably at least about 75% homology, preferably at least about 80% homology, preferably at least about 85% homology, preferably at least about 90% homology, and most preferably at least about 95% homology, to the base sequence represented by SEQ ID NO: 16 or SEQ ID NO: 17.

The hybridization can be carried out by publicly known methods or by modifications of these methods, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under highly stringent conditions.

The highly stringent conditions used herein are, for example, those in a sodium concentration at about 19 mM to about 40 mM, preferably about 19 mM to about 20 mM at a temperature of about 50°C to about 70°C, preferably about 60°C to about 65°C. In particular, hybridization conditions in a sodium concentration of about 19 mM at a temperature of about 65°C are most preferred.

More specifically, for the DNA encoding the protein having the amino acid sequence represented by SEQ ID NO: 1, there may be employed DNA having the base sequence represented by SEQ ID NO: 2 or SEQ ID NO: 7. For the DNA encoding the protein having the amino acid sequence represented by SEQ ID NO: 8, there may be employed DNA having the base sequence represented by SEQ ID NO: 9 or SEQ ID NO: 10. For the DNA encoding the protein having the amino acid sequence represented by SEQ ID NO: 11, there may be employed DNA having the base sequence represented by SEQ ID NO: 12 or SEQ ID NO: 13. For the DNA encoding the protein having the amino acid sequence represented by SEQ ID NO: 15, there may be employed DNA having the base sequence represented by SEQ ID NO: 16 or SEQ ID NO: 17. For the DNA encoding the protein having the amino acid sequence represented by SEQ ID NO: 18, there may be employed DNA having the base sequence represented by SEQ ID NO: 19 or SEQ ID NO: 22. For the DNA encoding the protein having the amino acid sequence represented by SEQ ID NO: 23, there may be employed DNA having the base sequence represented by SEQ ID NO: 24 or SEQ ID NO: 25. For the DNA encoding the protein having the amino acid sequence represented by SEQ ID NO: 26, there may be employed DNA having the base sequence represented by SEQ ID NO: 27 or SEQ ID NO: 28.

For the DNA encoding a partial peptide used in the present invention, any DNA containing the base sequence encoding a partial peptide used in the present invention may be used. In addition, any one of genomic DNA, genomic DNA library, cDNA derived from the above described cells or tissues, cDNA library derived from the above described cells or tissues, synthetic DNA may be used.

For the DNA encoding the partial peptide used in the present invention, there may be employed DNA having a portion of the DNA having the base sequence represented by SEQ ID NO: 2 or SEQ ID NO: 7, or DNA containing the base sequence, which hybridizes to the base sequence represented by SEQ ID NO: 2 or SEQ ID NO: 7 under high stringent conditions, and containing a portion of the DNA encoding the protein having the activity substantially equivalent to that of the protein containing the amino acid sequence represented by SEQ ID NO: 1. For the DNA encoding the partial peptide used in the present invention, there may be employed DNA having a portion of the DNA having the base sequence represented by SEQ ID NO: 9 or SEQ ID NO: 10, or DNA containing the base sequence, which hybridizes to the base sequence represented by SEQ ID NO: 9 or SEQ ID NO: 10 under high stringent conditions, and containing a portion of the DNA encoding the protein having the activity substantially equivalent to that of the protein containing the amino acid sequence represented by SEQ ID NO: 8. For the DNA encoding the partial peptide used in the present invention, there may be employed DNA having a portion of the DNA having the base sequence represented by SEQ ID NO: 12 or SEQ ID NO: 13, or DNA containing the base sequence, which hybridizes to the base sequence represented by SEQ ID NO: 12 or SEQ ID NO: 13 under high stringent conditions, and containing a portion of the DNA encoding the protein having the activity substantially equivalent to that of the protein containing the amino acid sequence represented by SEQ ID NO: 11. For the DNA encoding the partial peptide used in the present invention, there may be employed DNA having a portion of the DNA having the base sequence represented by SEQ ID NO: 16 or SEQ ID NO: 17, or DNA containing the base sequence, which hybridizes to the base sequence represented by SEQ ID NO: 16 or SEQ ID NO: 17 under high stringent conditions, and containing a portion of the DNA encoding the protein having the activity substantially equivalent to that of the protein containing the amino acid sequence represented by SEQ ID NO: 15.

DNA hybridizable to the base sequence represented by SE ID NO: 2 or SEQ ID NO: 15, DNA hybridizable to the base sequence represented by SE ID NO: 9 or SEQ ID NO: 10, DNA hybridizable to the base sequence represented by SE ID NO: 12 or SEQ ID NO: 13, and DNA hybridizable to the base sequence represented by SE ID NO: 16 or SEQ ID NO: 17 show the same meaning as described above.

For a hybridization method and high stringent conditions, similar ones as described above are utilized. For cloning of the DNA that completely encodes the protein of the present invention or its partial peptide (hereinafter sometimes collectively referred to as the protein of the present invention), the DNA may be either amplified by PCR using synthetic DNA primers containing a part of the base sequence of DNA encoding the protein of the present invention, or the DNA inserted into an appropriate vector can be selected by hybridization with a labeled DNA fragment or synthetic DNA that encodes a part or entire region of the protein of the present invention. The hybridization can be carried out, for example, according to the method described in Molecular Cloning, 2nd, J. Sambrook et al., Cold Spring Harbor Lab. Press, 1989. The hybridization may also be performed using commercially available library in accordance with the protocol described in the attached instructions.

Conversion of the base sequence of the DNA can be effected by publicly known methods such as the ODA-LA PCR method, the Gapped duplex method or the Kunkel method or its modification by using PCR or a publicly known kit available as Mutan^{TM}-super Express Km or Mutan^{TM}-K (both manufactured by Takara Shuzo Co., Ltd.).

The cloned DNA encoding the protein can be used as it is, depending upon purpose or, if desired, after digestion with a restriction enzyme or after addition of a linker thereto. The DNA may contain ATG as a translation initiation codon at the 5' end thereof and may further contain TAA, TGA or TAG as a translation termination codon at the 3' end thereof. These translation initiation and termination codons may also be added by using an appropriate synthetic DNA adapter.

The expression vector for the protein of the present invention can be manufactured, for example, by (a) excising the desired DNA fragment from the DNA containing the DNA encoding the protein of the present invention, and then (b) ligating the DNA fragment with an appropriate expression vector downstream a promoter in the vector.

Examples of the vector include plasmids derived form E. coli (e.g., pBR322, pBR325, pUC12, pUC 13), plasmids derived from Bacillus subtilis (e.g., pUB110, pTP5, pC194), plasmids derived from yeast (e.g., pSH19, pSH15), bacteriophages such as ? phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRa promoter, SV40 promoter, LTR promoter, CMV promoter, HSV-TK promoter, etc.

Among them, CMV promoter or SRa promoter is preferably used. Where the host is bacteria of the genus Escherichia, preferred examples of the promoter include trp promoter, lac promoter, recA promoter, ?P_{L} promoter, lpp promoter, T7 promoter, etc. In the case of using bacteria of the genus Bacillus as the host, preferred example of the promoter are SPO1 promoter, SPO2 promoter and penP promoter. When yeast is used as the host, preferred examples of the promoter are PHO5 promoter, PGK promoter, GAP promoter and ADH promoter. When insect cells are used as the host, preferred examples of the promoter include polyhedrin prompter and P10 promoter.

In addition to the foregoing examples, the expression vector may further optionally contain an enhancer, a splicing signal, a polyA addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker in CHO (dhfr⁻) cells, selection can also be made on thymidine free media.

If necessary and desired, a signal sequence that matches with a host is added to the N-terminus of the protein of the present invention. Examples of the signal sequence that can be used are PhoA signal sequence, OmpA signal sequence, etc. in the case of using bacteria of the genus Escherichia as the host; a-amylase signal sequence, subtilisin signal sequence, etc. in the case of using bacteria of the genus Bacillus as the host; MFa signal sequence, SUC2 signal sequence, etc. in the case of using yeast as the host; and insulin signal sequence, a-interferon signal sequence, antibody molecule signal sequence, etc. in the case of using animal cells as the host, respectively.

As described above, using the vector containing the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

Examples of the bacteria belonging to the genus Bacillus include Bacillus subtilis MI114 (Gene, 24, 255 (1983)), 207-21 (Journal of Biochemistry, 95, 87 (1984)), etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cells (Sf cells), MG1 cells derived from mid-intestine of Trichoplusia ni, High Five^{TM} cells derived from egg of Trichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711) and Sf21 cells (both cells are described in Vaughn, J. L. et al., In Vivo, 13, 213-217 (1977).

As the insect, for example, a larva of Bombyx mori can be used (Maeda, et al., Nature, 315, 592 (1985)).

Examples of animal cells include monkey cells COS-7, Vero, Chinese hamster cells CHO (hereinafter referred to as CHO cells), dhfr gene deficient Chinese hamster cells CHO (hereinafter simply referred to as CHO(dhfr⁻) cell), mouse L cells, mouse AtT-20, mouse myeloma cells, rat GH3, human FL cells, etc.

Bacteria belonging to the genus Escherichia can be transformed, for example, by the method described in Proc. Natl. Acad. Sci. U.S.A., 69, 2110 (1972) or Gene, 17, 107 (1982).

Bacteria belonging to the genus Bacillus can be transformed, for example, by the method described in Molecular & General Genetics, 168, 111 (1979).

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in *Saibo Kogaku* (Cell Engineering), extra issue 8, *Shin Saibo Kogaku Jikken Protocol* (New Cell Engineering Experimental Protocol), 263-267 (1995), published by Shujunsha, or Virology, 52, 456 (1973).

Thus, the transformant transformed with the expression vector containing the DNA encoding the protein can be obtained.

Where the host is bacteria belonging to the genus Escherichia or the genus Bacillus, the transformant can be appropriately incubated in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast extract, vitamins, growth promoting factors etc. may also be added to the medium. Preferably, pH of the medium is adjusted to about 5 to about 8.

A preferred example of the medium for incubation of the bacteria belonging to the genus Escherichia is M9 medium supplemented with glucose and Casamino acids (Miller, Journal of Experiments in Molecular Genetics, 431-433, Cold Spring Harbor Laboratory, New York, 1972). If necessary and desired, a chemical such as 3β-indolylacrylic acid can be added to the medium thereby to activate the promoter efficiently.

Where the bacteria belonging to the genus Escherichia are used as the host, the transformant is usually cultivated at about 15°C to about 43°C for about 3 hours to about 24 hours. If necessary and desired, the culture may be aerated or agitated.

Where the bacteria belonging to the genus Bacillus are used as the host, the transformant is cultivated generally at about 30°C to about 40°C for about 6 hours to about 24 hours. If necessary and desired, the culture can be aerated or agitated.

Where yeast is used as the host, the transformant is cultivated, for example, in Burkholder's minimal medium (Bostian, K. L. et al., Proc. Natl. Acad. Sci. U.S.A., 77, 4505 (1980)) or in SD medium supplemented with 0.5% Casamino acids (Bitter, G. A. et al., Proc. Natl. Acad. Sci. U.S.A., 81, 5330 (1984)). Preferably, pH of the medium is adjusted to about 5 to about 8. In general, the transformant is cultivated at about 20°C to about 35°C for about 24 hours to about 72 hours. If necessary and desired, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary and desired, the culture can be aerated or agitated.

Where animal cells are employed as the host, the transformant is cultivated in, for example, MEM medium containing about 5% to about 20% fetal bovine serum (Science, 122, 501 (1952)), DMEM medium (Virology, 8, 396 (1959)), RPMI 1640 medium (The Journal of the American Medical Association, 199, 519 (1967)), 199 medium (Proceeding of the Society for the Biological Medicine, 73, 1 (1950)), etc. Preferably, pH of the medium is adjusted to about 6 to about 8. The transformant is usually cultivated at about 30°C to about 40°C for about 15 hours to about 60 hours and, if necessary and desired, the culture can be aerated or agitated.

As described above, the protein of the present invention can be produced into the cell, in the cell membrane or out of the cell of the transformant.

The protein of the present invention can be separated and purified from the culture described above by the following procedures.

When the protein of the present invention is extracted from the culture or cells, after cultivation the transformants or cells are collected by a publicly known method and suspended in a appropriate buffer. The transformants or cells are then disrupted by publicly known methods such as ultrasonication, a treatment with lysozyme and/or freeze-thaw cycling, followed by centrifugation, filtration, etc. Thus, the crude extract of the protein of the present invention can be obtained. The buffer used for the procedures may contain a protein modifier such as urea or guanidine hydrochloride, or a surfactant such as Triton X-100^{TM}, etc. When the protein is secreted in the culture, after completion of the cultivation the supernatant can be separated from the transformants or cells to collect the supernatant by a publicly known method.

The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, it can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein modifying enzyme so that the protein can be appropriately modified to partially remove a polypeptide. Examples of the protein-modifying enzyme include trypsin, chymotrypsin, arginyl endopeptidase, protein kinase, glycosidase or the like.

The activity of the thus produced protein of the present invention or salts thereof can be determined by a test binding to a labeled ligand, by an enzyme immunoassay using a specific antibody, or the like.

Antibodies to the protein of the present invention, its partial peptides, or salts thereof may be any of polyclonal antibodies and monoclonal antibodies, as long as they are capable of recognizing the protein of the present invention, its partial peptides, or salts thereof.

The antibodies to the protein of the present invention, its partial peptides, or salts thereof (hereinafter sometimes merely referred to as the protein of the present invention) may be manufactured by publicly known methods for manufacturing antibodies or antisera, using as antigens the protein of the present invention.

### [Preparation of monoclonal antibody]

### (a) Preparation of monoclonal antibody-producing cells

The protein of the present invention is administered to mammals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once in every two to six weeks and 2 to 10 times in total. Examples of the applicable mammals are monkeys, rabbits, dogs, guinea pigs, mice, rats, sheep and goats, with mice and rats being preferred.

In the preparation of monoclonal antibody-producing cells, warm-blooded animals, e.g., mice, immunized with an antigen wherein the antibody titer is noted is selected, then the spleen or lymph node is collected after 2 to 5 days from the final immunization and antibody-producing cells contained therein are fused with myeloma cells to give monoclonal antibody-producing hybridomas. Measurement of the antibody titer in antisera may be made, for example, by reacting a labeled form of the protein, which will be described later, with the antiserum followed by assaying the binding activity of the labeling agent bound to the antibody. The fusion may be operated, for example, by the known Koehler and Milstein method (Nature, 256, 495, 1975). Examples of the fusion accelerator are polyethylene glycol (PEG), Sendai virus, etc., of which PEG is preferably employed.

Examples of the myeloma cells are NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at about 20 to about 40°C, preferably at about 30 to about 37°C for about 1 to about 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with the protein as an antigen directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase.

The monoclonal antibody can be selected by publicly known methods or by modifications of these methods. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20°C to 40°C, preferably at about 37°C, for 5 days to 3 weeks, preferably 1 to 2 weeks. The cultivation can be conducted normally in 5% CO₂. The antibody titer of the culture supernatant of hybridomas can be determined as in the assay for the antibody titer in antisera described above.

### (b) Purification of monoclonal antibody

Separation and purification of a monoclonal antibody can be carried out by methods applied to conventional separation and purification of immunoglobulins, as in the conventional methods for separation and purification of polyclonal antibodies [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to obtain the antibody].

### [Preparation of polyclonal antibody]

The polyclonal antibody of the present invention can be manufactured by publicly known methods or modifications thereof. For example, a complex of immunogen (antigen such as the protein of the present invention) and a carrier protein is prepared, and a mammal is immunized with the complex in a manner similar to the method described above for the manufacture of monoclonal antibodies. The product containing the antibody to the protein of the present invention is collected from the immunized animal followed by separation and purification of the antibody.

In the complex of an immunogen and a carrier protein used to immunize a mammal, the type of carrier protein and the mixing ratio of a carrier to hapten may be any type and in any ratio, as long as the antibody is efficiently produced to the hapten immunized by crosslinking to the carrier. For example, bovine serum albumin, bovine thyroglobulins, keyhole limpet hemocyanin, etc. is coupled to hapten in a carrier-to-hapten weight ratio of approximately 0.1 to 20, preferably about 1 to about 5.

A variety of condensing agents can be used for the coupling of a carrier to hapten. Glutaraldehyde, carbodiimide, maleimide activated ester, activated ester reagents containing thiol group or dithiopyridyl group, etc. are used for the coupling.

The condensation product is administered to warm-blooded animals either solely or together with carriers or diluents to the site in which the antibody can be produce by the administration. In order to potentiate the antibody productivity upon the administration, complete Freund's adjuvant or incomplete Freund's adjuvant may be administered. The administration is usually made once approximately in every 2 to 6 weeks and about 3 to about 10 times in total.

The polyclonal antibody can be collected from the blood, ascites, etc., preferably from the blood of mammals immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulin performed as applied to the separation and purification of monoclonal antibodies described hereinabove.

For an antisense nucleotide having a complementary or substantially complementary base sequence to that of the DNA encoding the protein or its partial peptide of the present invention, or a portion thereof (hereinafter, in the description about the antisense nucleotide, it is merely abbreviated these DNAs as the DNA of the present invention), any antisense nucleotides may be used, as long as it has a complementary or substantially complementary base sequence to that of the DNA of the present invention, or a portion thereof, and has a function suppressing an expression of the DNA. Above all, antisense DNA is preferred.

Examples of the substantially complementary base sequence to that of the DNA of the present invention include the base sequences having at least about 70% homology, preferably at least about 80% homology, more preferably at least about 90% homology, and most preferably at least about 95% homology to the entire or partial base sequence of the complementary base sequence of the DNA of the present invention (i.e., the complementary strand of the DNA of the present invention). In particular, among the entire base sequence of the complementary strand of the DNA of the present invention, preferred is an antisense nucleotide having at least about 70% homology, preferably at least about 80% homology, more preferably at least about 90% homology, and most preferably at least about 95% homology to the complementary strand of the base sequence encoding a portion of the N-terminal region of the protein of the present invention (e.g., the base sequence adjacent to initiation codon).

Specifically, it includes:
(1) An antisense nucleotide having a complementary or substantially complementary base sequence to that of the DNA having the base sequence represented by SEQ ID NO: 2 or SEQ ID NO: 7, or a portion thereof, preferably an antisense nucleotide having a complementary base sequence to that of the DNA having the base sequence represented by SEQ ID NO: 2 or SEQ ID NO: 7, or a portion thereof;
(2) An antisense nucleotide having a complementary or substantially complementary base sequence to that of the DNA having the base sequence represented by SEQ ID NO: 9 or SEQ ID NO: 10, or a portion thereof, preferably an antisense nucleotide having a complementary base sequence to that of the DNA having the base sequence represented by SEQ ID NO: 9 or SEQ ID NO: 10, or a portion thereof;
(3) An antisense nucleotide having a complementary or substantially complementary base sequence to that of the DNA having the base sequence represented by SEQ ID NO: 12 or SEQ ID NO: 13, or a portion thereof, preferably an antisense nucleotide having a complementary base sequence to that of the DNA having the base sequence represented by SEQ ID NO: 12 or SEQ ID NO: 13, or a portion thereof;
(4) An antisense nucleotide having a complementary or substantially complementary base sequence to that of the DNA having the base sequence represented by SEQ ID NO: 16 or SEQ ID NO: 17, or a portion thereof, preferably an antisense nucleotide having a complementary base sequence to that of the DNA having the base sequence represented by SEQ ID NO: 16 or SEQ ID NO: 17, or a portion thereof;
(5) An antisense nucleotide having a complementary or substantially complementary base sequence to that of the DNA having the base sequence represented by SEQ ID NO: 19 or SEQ ID NO: 22, or a portion thereof, preferably an antisense nucleotide having a complementary base sequence to that of the DNA having the base sequence represented by SEQ ID NO: 19 or SEQ ID NO: 22, or a portion thereof;
(6) An antisense nucleotide having a complementary or substantially complementary base sequence to that of the DNA having the base sequence represented by SEQ ID NO: 24 or SEQ ID NO: 25, or a portion thereof, preferably an antisense nucleotide having a complementary base sequence to that of the DNA having the base sequence represented by SEQ ID NO: 24 or SEQ ID NO: 25, or a portion thereof;
(7) An antisense nucleotide having a complementary or substantially complementary base sequence to that of the DNA having the base sequence represented by SEQ ID NO: 27 or SEQ ID NO: 28, or a portion thereof, preferably an antisense nucleotide having a complementary base sequence to that of the DNA having the base sequence represented by SEQ ID NO: 27 or SEQ ID NO: 28, or a portion thereof; and the like.

The antisense nucleotide comprises usually about 10 to 40 and preferably 15 to 30 bases.

To prevent degradation by hydrolase such as nuclease, a phosphate residue (phosphate) of each nucleotide constituting the antisense nucleotide may be, for example, substituted through chemical modification by the phosphate residue such as phosphorothioate, methylphosphonate, phosphorodithionate, etc. These antisense nucleotides can be manufactured by using a publicly known DNA synthesizer.

According to the present invention, antisense polynucleotides (nucleic acids) that can inhibit the replication or expression of the gene encoding the protein of the present invention can be designed and synthesized based on the base sequence information of the cloned or determined DNA encoding the protein. Such a polynucleotide (nucleic acid) is capable of hybridizing to RNA of the gene encoding the protein of the present invention to inhibit the synthesis or function of said RNA or capable of modulating or controlling the expression of the gene encoding the protein of the present invention via interaction with the protein-associated RNA. Polynucleotides complementary to the selected sequences of RNA associated with the protein of the present invention and polynucleotides specifically hybridizable to the protein-associated RNA are useful in modulating or controlling the expression of the gene encoding the protein of the present invention in vivo and in vitro, and useful for the treatment or diagnosis of diseases. The term "corresponding" is used to mean homologous to or complementary to a particular sequence of the nucleotide, base sequence or nucleic acid including the gene. The term "corresponding" between nucleotides, base sequences or nucleic acids and peptides (proteins) usually refer to amino acids of a peptide (protein) under the order derived from the sequence of nucleotides (nucleic acids) or their complements. In the gene encoding the protein of the present invention, the 5' end hairpin loop, 5' end 6-base-pair repeats, 5' end untranslated region, polypeptide translation initiation codon, protein coding region, ORF translation termination codon, 3' end untranslated region, 3' end palindrome region, and 3' end hairpin loop, may be selected as preferred target regions, though any other region may be selected as a target in the gene encoding the protein.

The relationship between the targeted nucleic acids and the polynucleotides complementary to at least a part of the target, that is, the relationship between the target and the polynucleotides hybridizable to the target, can be denoted to be "antisense". Examples of the antisense polynucleotides include polynucleotides containing 2-deoxy-D-ribose, polynucleotides containing D-ribose, any other type of polynucleotides which are N-glycosides of a purine or pyrimidine base, or other polymers containing non-nucleotide backbones (e.g., protein nucleic acids and synthetic sequence-specific nucleic acid polymers commercially available) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., a anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, increasing the cellular permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense nucleic acid of the present invention may contain altered or modified sugars, bases or linkages. The antisense nucleic acid may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense nucleic acid can be examined using the transformant of the present invention, the gene expression system of the present invention in vivo and in vitro, or the translation system of the G protein-coupled receptor protein in vivo and in vitro. The nucleic acid can be applied to cells by a variety of publicly known methods.

Hereinafter, the protein of the present invention, its partial peptides, or salts thereof (hereinafter sometimes referred to as the protein of the present invention), the DNA encoding the protein of the present invention or its partial peptides (hereinafter sometimes referred to as the DNA of the present invention), the antibodies to the protein of the present invention (hereinafter sometimes referred to as the antibodies of the present invention), and the antisense nucleotides (hereinafter sometimes referred to as the antisense nucleotide of the present invention) are specifically described for the use or applications.

A pharmaceutical comprising a compound or a salt thereof that inhibits the activities of the protein of the present invention can be used by, for example, suppressing the transmission of K⁺ ion, as a prophylactic and/or therapeutic agent for alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like. Preferred is a prophylactic and/or therapeutic agent for alimentary diseases, reproductive system diseases, respiratory diseases or the like.

A pharmaceutical comprising a compound or a salt thereof that enhances the activities of the protein of the present invention can be used by, for example, enhancing the transmission of K⁺ ion, as a prophylactic and/or therapeutic agent for alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like. Preferred is a prophylactic and/or therapeutic agent for alimentary diseases, reproductive system diseases, respiratory diseases or the like.

### [1] A prophylactic and/or therapeutic agent for various diseases associated with the protein of the present invention

The protein of the present invention possesses a transmission activity of K⁺ ion (e.g., an activity that detects a cell membrane potential or intracellular Ca²⁺ ion concentration and regulates the transmission of K+ ion) and the like, and thus plays an important role for regulation of cell excitement.

Therefore, when the DNA encoding the protein of the present invention has abnormality or deficiency, or when a reduced expression of the protein of the present invention is detected, various diseases such as alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), and the like, are developed.

Therefore, the protein of the present invention and the DNA of the present invention can be used for a pharmaceutical such as a prophylactic and/or therapeutic agent for alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like.

When a patient has a reduced level of, or deficient of the protein, etc. of the present invention in his or her body and thus may not maintain homeostasis in the body or may not exhibit the transmission of K⁺ ion sufficiently or properly, the protein of the present invention can provide its role sufficiently or properly for the patient, (a) by administering the DNA of the present invention to the patient to express the protein of the present invention in the body, (b) by inserting the DNA of the present invention into a cell, expressing the protein of the present invention and then transplanting the cell to the patient, or (c) by administering the protein of the present invention to the patient, etc.

Where the DNA of the present invention is used as the prophylactic and/or therapeutic agents described above, the DNA *per se* is administered directly to warm-blooded animal; alternatively, the DNA is inserted into an appropriate vector such as retrovirus vector, adenovirus vector, adenovirus-associated virus vector, etc. and then administered to warm-blooded animal in a conventional manner. The DNA of the present invention may also be administered as intact DNA, or with adjuvants to assist its uptake by gene gun or through a catheter such as a catheter with a hydrogel.

Where the protein of the present invention is used as the aforesaid prophylactic and/or therapeutic agents, the protein is advantageously used on a purified level of at least 90%, preferably at least 95%, more preferably at least 98% and most preferably at least 99%.

The protein of the present invention can be used orally, for example, in the form of tablets which may be sugar coated if necessary, capsules, elixirs, microcapsules etc., or parenterally in the form of injectable preparations such as a sterile solution and a suspension in water or with other pharmaceutically acceptable liquid. These preparations can be manufactured by mixing the protein of the present invention with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The active ingredient in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated according to a conventional manner used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition.

Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80^{TM} and HCO-50), etc. Examples of the oily medium include sesame oil and soybean oil, which may also be used in combination with a dissolution aid such as benzyl benzoate and benzyl alcohol. The prophylactic/therapeutic agent described above may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

The vector in which the DNA of the present invention has been inserted may also be prepared into pharmaceutical preparations in a manner similar to the procedures above. Such preparations are generally used parenterally.

Since the thus obtained pharmaceutical preparation is safe and low toxic, the preparation can be administered to warm-blooded animal (e.g., human, rat, mouse, guinea pig, rabbit, chicken, sheep, swine, bovine, horse, cat, dog, monkey, chimpanzee, etc.).

The dose of the protein of the present invention varies depending on target disease, subject to be administered, route for administration, etc.; for example, in oral administration for the treatment of irritable bowel syndrome, the dose is normally about 0.1 mg to about 100 mg, preferably about 1.0 to about 50 mg, and more preferably about 1.0 to about 20 mg per day for adult (as 60 kg body weight). In parenteral administration, the single dose varies depending on subject to be administered, target disease, etc. but it is advantageous for the treatment of irritable bowel syndrome to administer the active ingredient intravenously at a daily dose of about 0.01 to about 30 mg, preferably about 0.1 to about 20 mg, and more preferably about 0.1 to about 10 mg for adult (as 60 kg body weight). For other animal species, the corresponding dose as converted per 60 kg body weight can be administered.

### [2] A screening of candidate compounds for pharmaceutical against diseases

The protein of the present invention is useful for an agent for screening a compound that enhances or inhibits activities of the protein of the present invention, or a salt thereof.

The present invention provides (1) a method for screening a compound that enhances or inhibits activities of the protein of the present invention (e.g., transmission of K⁺ ion, etc.), or a salt thereof (hereinafter, sometimes referred to as an enhancer or an inhibitor), which comprises using the protein of the present invention. More specifically, for example, it provides (2) a method for screening an enhancer or an inhibitor, which comprises making a comparison between (i) transmission (activity) of K⁺ ion in the cells having an ability of producing the protein of the present invention and (ii) transmission (activity) of K⁺ ion in the mixture of the cells having an ability of producing the protein of the present invention and a test compound.

Specifically, the above-described method for screening comprises measuring a membrane potential accompanied by transmission of K⁺ ion using fluorescent dye in the case of (i) and (ii), and comparing as an index of transmission of K⁺ ion.

The test compounds include, for example, peptides, proteins, non-peptidic compounds, synthetic compounds, fermentation products, cell extracts, plant extracts, animal tissue extracts and the like. These compounds may be either novel compounds or publicly known compounds.

In order to carry out the above-described method for screening, first, the cells having an ability of producing the protein of the present invention is prepared by suspending in a buffer appropriate for the screening. For the buffer, any buffer that does not interfere with the transmission (activity) of K⁺ ion of the protein of the present invention is usable and examples of such a buffer are phosphate buffer, borate buffer, etc., having pH of 4 to 10 (preferably pH of 6 to 8).

For the cells having an ability of producing the protein of the present invention, for example, a host transformed with a vector comprising the DNA encoding the aforementioned protein of the present invention (a transformant) may be utilized. For the host, for example, animal cells such as CHO cells, etc. are preferably used. For the screening, a transformant, wherein the protein of the present invention will be expressed on the cell membrane by culturing with the method described above, is preferably used.

The transmission of K⁺ ion of the protein of the present invention can be measured by a publicly known method, e.g., the method described in Receptors and Channels, Vol. 6, pp. 337 - 350, 1999, or by a modification thereof.

For example, when transmission of K⁺ ion in the above (ii) is enhanced at least about 10%, preferably at least about 30% and more preferably at least about 50% to that the above (i), the test compound can be selected as a compound that enhances activities of the protein of the present invention, or a salt thereof.

In addition, when transmission of K⁺ ion in the above (ii) is inhibited (or suppressed) at least about 10%, preferably at least about 30% and more preferably at least about 50% to that the above (i), the test compound can be selected as a compound that inhibits activities of the protein of the present invention, or a salt thereof.

By screening a compound that activates or inhibits an enzyme activityin the case where genes such as secretion-type alkaline phosphatase, luciferase, etc. are inserted downstream the promoter for the gene encoding the protein of the present invention and expressed in various cells described above, and the test compound is brought into contact with the cells, the compound that enhances or inhibits the expression of the protein of the present invention, i.e., enhances or inhibits the activity of the protein of the present invention, or a salt thereof, can be screened.

The polynucleotide encoding the protein of the present invention is useful for a reagent for screening a compound that enhances or inhibits the expression of the gene encoding the protein of the present invention, or a salt thereof.

The present invention provides (3) a method for screening a compound that enhances or inhibits expression of the gene encoding the protein of the present invention, or a salt thereof (hereinafter, sometimes referred to as an enhancer or an inhibitor), which comprises using a polynucleotide encoding the protein of the present invention. More specifically, for example, it provides (4) a method for screening an enhancer or an inhibitor, which comprises making a comparison between (iii) the case where cells having an ability of producing the protein of the present invention are cultured and (iv) the case where a mixture of cells having an ability of producing the protein of the present invention and a test compound is cultured.

Specifically, the above-described method for screening comprises measuring an expression level of the gene encoding the protein of the present invention (specifically, an level of the protein of the present invention or a level of mRNA encoding the aforementioned protein) in the case of (iii) and (iv), and comparing.

The test compounds include, for example, peptides, proteins, non-peptidic compounds, synthetic compounds, fermentation products, cell extracts, plant extracts, animal tissue extracts and the like. These compounds may be either novel compounds or publicly known compounds.

In order to carry out the above-described method for screening, first, the cells having an ability of producing the protein of the present invention is prepared by suspending in a buffer appropriate for the screening. For the buffer, any buffer that does not interfere with the transmission (activity) of K⁺ ion of the protein of the present invention is usable and examples of such a buffer are phosphate buffer, borate buffer, etc., having pH of 4 to 10 (preferably pH of 6 to 8).

For the cells having an ability of producing the protein of the present invention, for example, a host transformed with a vector comprising the DNA encoding the aforementioned protein of the present invention (a transformant) may be utilized. For the host, for example, animal cells such as CHO cells, etc. are preferably used. For the screening, a transformant, wherein the protein of the present invention will be expressed on the cell membrane by culturing with the method described above, is preferably used.

The determination of the level of the protein of the present invention can be carried out by a publicly known method, e.g., measured the level of the protein of the present invention in the cell extracts, etc., using the antibody recognizing the protein of the present invention according to the method such as Western analysis and ELISA techniques, or a modification thereof.

The expression level of the gene encoding the protein of the present invention can be assayed according to the publicly known method, e.g., Northern blotting, Reverse transcription-polymerase chain reaction (RT-PCR) or Real time PCR analysis system (ABI, TaqMan polymerase chain reaction), or a modification thereof.

For example, when the expression level of the gene encoding the protein of the present invention in the above (ii) is enhanced at least about 10%, preferably at least about 30% and more preferably at least about 50% to that the above (i), the test compound can be selected as a compound that enhances the expression level of the gene encoding the protein of the present invention, or a salt thereof.

In addition, when the expression level of the gene encoding the protein of the present invention in the above (ii) is inhibited at least about 10%, preferably at least about 30% and more preferably at least about 50% to that the above (i), the test compound can be selected as a compound that inhibits the expression level of the gene encoding the protein of the present invention, or a salt thereof.

Further, the antibody of the present invention is useful for a reagent for screening a compound that enhances or inhibits the expression of the protein of the present invention, or a salt thereof.

The present invention provides (5) a method for screening a compound that enhances or inhibits expression of the protein of the present invention, or a salt thereof (hereinafter, sometimes referred to as an enhancer or an inhibitor), which comprises using an antibody of the present invention. More specifically, for example, it provides (6) a method for screening an enhancer or an inhibitor, which comprises making a comparison between (v) the case where cells having an ability of producing the protein of the present invention are cultured and (vi) the case where a mixture of cells having an ability of producing the protein of the present invention and a test compound is cultured.

Specifically, the above-described method for screening comprises measuring an expression level of the protein of the present invention (specifically, an level of the protein of the present invention) in the case of (v) and (vi), e.g., detecting the expression of the protein of the present invention, or quantifying the expression level of the protein of the present invention, and comparing.

The test compounds include, for example, peptides, proteins, non-peptidic compounds, synthetic compounds, fermentation products, cell extracts, plant extracts, animal tissue extracts and the like. These compounds may be either novel compounds or publicly known compounds.

In order to carry out the above-described method for screening, first, the cells having an ability of producing the protein of the present invention is prepared by suspending in a buffer appropriate for the screening. For the buffer, any buffer that does not interfere with the transmission (activity) of K⁺ ion of the protein of the present invention is usable and examples of such a buffer are phosphate buffer, borate buffer, etc., having pH of 4 to 10 (preferably pH of 6 to 8).

For the cells having an ability of producing the protein of the present invention, for example, a host transformed with a vector comprising the DNA encoding the aforementioned protein of the present invention (a transformant) may be utilized. For the host, for example, animal cells such as CHO cells, etc. are preferably used. For the screening, a transformant, wherein the protein of the present invention will be expressed on the cell membrane by culturing with the method described above, is preferably used.

The determination of the level of the protein of the present invention can be carried out by a publicly known method, e.g., measured the level of the protein of the present invention in the cell extracts, etc., using the antibody recognizing the protein of the present invention according to the method such as Western analysis and ELISA techniques, or a modification thereof.

For example, when the expression level of the protein of the present invention in the above (vi) is enhanced at least about 10%, preferably at least about 30% and more preferably at least about 50% to that the above (v), the test compound can be selected as a compound that enhances the expression level of the protein of the present invention, or a salt thereof.

For example, when the expression level of the protein of the present invention in the above (vi) is inhibited at least about 10%, preferably at least about 30% and more preferably at least about 50% to that the above (v), the test compound can be selected as a compound that inhibits the expression level of the protein of the present invention, or a salt thereof.

A kit for screening of the present invention comprises the protein of the present invention, its partial peptide or salts thereof, or cells having an ability of producing the protein of the present invention or a partial peptide thereof.

A compound or a salt thereof obtained by using the screening method or the screening kit of the present invention is the above-described test compound, e.g., a compound or a salt thereof, which is selected from peptide, protein, non-peptidic compound, synthetic compound, fermentation product, cell extract, plant extract, animal tissue extract, blood plasma and the like. In addition, it is also a compound that enhances or inhibited the activity of the protein of the present invention (e.g., transmission of K⁺ ion), or a salt thereof.

As a salt form of the compound, it is usable for the same as the salt form of the protein of the present invention described above.

The compound that enhances activities of the protein of the present invention, or a salt thereof is useful as a pharmaceutical such as a prophylactic and/or therapeutic agent for alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like.

The compound that inhibits activities of the protein of the present invention, or a salt thereof is useful as a pharmaceutical such as a prophylactic and/or therapeutic agent for alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like.

The compound that enhances expression of the gene encoding the protein of the present invention, or a salt thereof is useful as a pharmaceutical such as a prophylactic and/or therapeutic agent for alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like.

Further, the compound that inhibits expression of the gene encoding the protein of the present invention, or a salt thereof is useful as a pharmaceutical such as a prophylactic and/or therapeutic agent for alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like.

The compound that enhances expression of the protein of the present invention, or a salt thereof is useful as a pharmaceutical such as a prophylactic and/or therapeutic agent for alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like.

In addition, the compound that inhibits expression of the the protein of the present invention, or a salt thereof is useful as a pharmaceutical such as a prophylactic and/or therapeutic agent for alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like.

When the compound or a salt thereof obtained by using the screening method or the screening kit of the present invention is used as the above-described prophylactic and/or therapeutic agent, it may be prepared in a conventional manner, to tablets, capsules, elixirs, microcapsules, sterile solution or suspension.

Since the preparation thus obtained is safe and low toxic, it can be administered to human or other warm-blooded animals (e.g., mouse, rat, rabbit, sheep, swine, bovine, horse, fowl, cat, dog, monkey, chimpanzee, etc.).

Although the amount of the compound or its salt to be administered varies depending upon its function, target disease, subject to be administered, route of administration, etc., when the compound that enhances the activity of the protein of the present invention is orally administered, the compound is generally administered to an adult patient with irritable bowel syndrome (as 60 kg body weight) in a dose of about 0.1 mg/day to about 100 mg/day, preferably about 1.0 mg/day to about 50 mg/day, more preferably about 1.0 mg to about 20 mg. For parenteral administration, a single dose of the compound varies depending upon subject to be administered, target disease, etc., but when the compound is administered to an adult patient with irritable bowel syndrome (as 60 kg body weight) in the form of an injectable preparation, it is generally advantageous to administer the compound intravenously in a dose of about 0.01 mg/day to about 30 mg/day, preferably about 0.1 mg/day to about 20 mg/day, more preferably about 0.1 mg/day to about 10 mg/day. As for other animals, the compound can be administered in the above amount with converting it into that for the body weight of 60 kg.

### [3] Quantification of the protein of the present invention, its partial peptide or salts thereof

The antibodies of the present invention are capable of specifically recognizing the protein of the present invention. Therefore, the antibodies can be used to quantify the protein of the present invention in a test fluid, especially for quantification by the sandwich immunoassay.

That is, the present invention provides, for example, the following quantification methods:
(i) A method of quantifying the protein of the present invention in a test fluid, which comprises competitively reacting the antibody of the present invention with the test fluid and a labeled form of the protein of the present invention, and measuring the ratio of the labeled protein of the present invention bound to the antibody; and
(ii) A method of quantifying the protein of the present invention in a test fluid, which comprises reacting the test fluid with the antibody of the present invention immobilized on a carrier and a labeled form of the antibody of the present invention simultaneously or sequentially, and measuring the activity of the label on the immobilized carrier.

In (ii) described above, it is preferred that one antibody recognizes the N-terminal region of the protein of the present invention, and another antibody reacts with the C-terminal region of the protein of the present invention.

Using monoclonal antibodies to the protein of the present invention (hereinafter sometimes referred to as the monoclonal antibodies of the present invention), the protein of the present invention can be assayed and also detected by tissue staining or the like. For this purpose, an antibody molecule itself may be used, or F(ab')₂, Fab' or Fab fractions of the antibody molecule may also be used. Assay methods using antibodies to the receptor protein etc. of the present invention are not particularly limited. Any assay method can be used, so long as the amount of antibody, antigen, or antibody-antigen complex corresponding to the amount of antigen (e.g., the amount of the receptor protein) in the test fluid can be detected by chemical or physical means and the amount of the antigen can be calculated from a standard curve prepared from standard solutions containing known amounts of the antigen. For example, nephrometry, competitive methods, immunometric method, and sandwich method are appropriately used, with the sandwich method described below being most preferable in terms of sensitivity and specificity.

As the labeling agent for the methods using labeled substances, there are employed, for example, radioisotopes, enzymes, fluorescent substances, luminescent substances, etc. For the radioisotope, for example, [¹²⁵I], [¹³¹I], [³H] and [¹⁴C] are used. As the enzyme described above, stable enzymes with high specific activity are preferred; for example, β-galactosidase, β-glucosidase, alkaline phosphatase, peroxidase, malate dehydrogenase and the like are used. Example of the fluorescent substance used is fluorescamine and fluorescein isothiocyanate are used. For the luminescent substance, for example, luminol, luminol derivatives, luciferin, and lucigenin can be used. Furthermore, the biotin-avidin system may be used for binding antibody or antigen to the label.

For immobilization of antigen or antibody, physical adsorption may be used. Chemical binding methods conventionally used for insolubilization or immobilization of proteins or enzymes may also be used. For the carrier, for example, insoluble polysaccharides such as agarose, dextran, cellulose, etc.; synthetic resin such as polystyrene, polyacrylamide, silicon, etc., and glass or the like. are used.

In the sandwich method, the immobilized monoclonal antibody of the present invention is reacted with a test fluid (primary reaction), then with the labeled monoclonal antibody of the present invention (secondary reaction), and the activity of the label on the immobilizing carrier is measured, whereby the amount of the protein of the present invention in the test fluid can be quantified. The order of the primary and secondary reactions may be reversed, and the reactions may be performed simultaneously or with an interval. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity.

In the methods of assaying the protein of the present invention by the sandwich method, antibodies that bind to different sites of the protein are preferably used as the monoclonal antibodies of the present invention for the primary and secondary reactions. That is, in the antibodies used for the primary and secondary reactions are, for example, when the antibody used in the secondary reaction recognizes the C-terminal region of the protein, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, competitive method, immunometric method, nephrometry, etc.

In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed. For applying these immunological methods to the measurement methods of the present invention, any particular conditions or procedures are not required. Systems for measuring the protein of the present invention or its salts are constructed by adding the usual technical consideration in the art to the conventional conditions and procedures. For the details of these general technical means, reference can be made to the following reviews and texts.

[For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immonoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing).]

As described above, the protein of the present invention or its salts can be quantified with high sensitivity, using the antibodies of the present invention.

When the reduced concentration of the protein of the present invention was detected by quantifying the concentration of the protein of the present invention using the antibody of the present invention, it can be diagnosed that it is highly possible to develop, for example, alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like. In addition, when the increased concentration of the protein of the present invention was detected, it can be diagnosed that it is highly possible to develop, for example, alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like.

Further, the antibody of the present invention may be used for detecting the protein of the present invention existed in the test sample such as body fluid and tissues. Furthermore, it can be used for preparing an antibody column for purification of the protein of the present invention, detecting the protein of the present invention in each fraction of purification, analyzing a profile of the protein of the present invention in the cells tested, and the like.

### [4] Gene diagnostic agent

By using the DNA of the invention, e.g., as a probe, an abnormality (gene abnormality) of the DNA or mRNA encoding the protein or its partial peptide of the invention in human or other warm-blooded animal (e.g., rat, mouse, guinea pig, rabbit, chicken, sheep, swine, bovine, horse, cat, dog, monkey, chimpanzee, etc.) can be detected. Therefore, the DNA of the invention is useful as a gene diagnostic agent for damages to the DNA or mRNA, its mutation or decreased expression, or increased expression or overexpression of the DNA or mRNA.

The gene diagnosis described above using the DNA of the invention can be performed by, for example, publicly known Northern hybridization or PCR-SSCP assay (Genomics, 5, 874-879 (1989); Proceedings of the National Academy of Sciences of the United States of America, 86, 2766-2770 (1989)), etc.

For example, when increased expression is detected by northern hybridization, it can be diagnosed that it is highly likely to suffer from alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like. On the contrary, when reduced expression is detected by northern hybridization or DNA microarray, or when DNA mutation is detected by PCR-SSCP, it can be diagnosed that it is highly likely to suffer from alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like.

### [5] Pharmaceuticals comprising antisense nucleotide

Since the antisense nucleotide of the present invention, which binds to the DNA of the invention complementarily and suppresses the DNA expression, is low toxic and can suppress in vivo the functions of the protein of the present invention or the DNA of the present invention (e.g., transmission of K⁺ ion), it can be used as the agent for the treatment/prevention of alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like. Preferred is a preventive and/or therapeutic agent for alimentary diseases, reproductive system diseases, respiratory diseases and the like.

When the antisense nucleotide described above may be employed as the above prophylactic and/or therapeutic agents, it can be prepared according to the publicly known methods and can be administered.

For example, when the antisense nucleotide described above is used, it is administered solely, or it is inserted into an appropriate vector such as retrovirus vector, adenovirus vector, adenovirus-associated virus vector, etc., which is then administered orally or parenterally to human or other mammals (e.g., rat, rabbit, sheep, swine, bovine, cat, dog, monkey, etc.) in a conventional manner. The antisense nucleotide may be administered as it stands, or may be prepared into a dosage form together with a physiologically acceptable carrier to increase its uptake and administered by gene gun or through a catheter such as a catheter with a hydrogel.

Although the amount of the antisense nucleotide to be administered varies depending upon particular disease, subject to be administered, route of administration, etc., when the antisense nucleotide is locally administered to large intestine for prevention and/ or treatment of irritable bowel syndrome, the antisense nucleotide may be administered, in adult (60 kg of body weight), in a dose of about 0.1 mg/day to about 100 mg/day.

Further, the antisense nucleotide may also be employed as an oligonucleotide probe for diagnosis to examine the presence of the DNA of the invention in tissues or cells and the conditions of its expression.

The present invention further provides:
(i) A double-stranded RNA containing a part of the RNA encoding the protein of the present invention and its complementary RNA;
(ii) A pharmaceutical comprising the double-stranded RNA described above;
(iii) A ribozyme containing a part of the RNA encoding the protein of the present invention;
(iv) A pharmaceutical comprising the ribozyme described above;
(v) An expression vector containing the gene (the DNA) encoding the ribozyme described above; and the like.

As in the antisense nucleotide described above, the double-stranded RNA, ribozyme, etc. can destroy the RNA transcribed from the DNA of the present invention or suppress the functions of the RNA. Further, they can suppress the in vivo function of the protein or the DNA of the invention. Therefore, they can be used as preventive/therapeutic agents for alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like. Preferred is a prophylactic and/or therapeutic agent for alimentary diseases, reproductive system diseases, respiratory diseases and the like.

The double-stranded RNA can be manufactured by designing the same based on the sequence of the polynucleotide of the present invention, by publicly known methods (e.g., Nature, 411, 494, 2001) with a modification.

The ribozyme can be manufactured by designing the same based on the sequence of the polynucleotide of the present invention, by a modification of publicly known methods (e.g., TRENDS in Molecular Medicine, 7, 221, 2001). For example, the ribozyme can be manufactured by replacing a partial sequence of the publicly known ribozyme with a part of the RNA encoding the protein of the present invention. The part of the RNA encoding the protein of the present invention includes a contiguous part to the consensus sequence NUX (in the sequence, N means all bases and X means bases except for G), which can be cleaved by a publicly known ribozyme.

Where the double-stranded RNA or ribozyme described above is used as the prophylactic and/or therapeutic agent described above, the RNA or ribozyme may be prepared into pharmaceutical preparations, as in the antisense polynucleotide, which are provided for administration. In addition, the expression vector described in (v) above, may be used as the prophylactic and/or therapeutic agent, just like the publicly known gene therapy.

### [6] Pharmaceuticals comprising the antibody of the present invention

The antibody of the present invention, which possesses a neutralizing activity to the activity of the protein of the present invention, can be used as a prophylactic and/or therapeutic agent for alimentary diseases (e.g.., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), and the like. Preferred are prophylactic and/or therapeutic agents for alimentary diseases, reproductive system diseases, respiratory diseases and the like.

Since the prophylactic and/or therapeutic agent for the above-mentioned diseases comprising the antibody of the present invention is low toxic, it can orally or parenterally (e.g., by intravenous injection) be administered to human or other mammals (e.g., rat, rabbit, sheep, swine, bovine, cat, dog, monkey) as a liquid formulation or an appropriate dosage form of pharmaceutical compositions. Although the amount of the antibody to be administered varies depending upon particular disease, subject to be administered, route of administration, etc., when the antibody is used for prevention and/ or treatment of an adult patient with irritable bowel syndrome, it is generally advantageous to administer the antibody intravenously in a dose of about 0.01 mg/kg of body weight to about 20 mg/kg of body weight, preferably about 0.1 mg/kg of body weight to about 10 mg/kg of body weight, further preferably about 0.1 mg/kg of body weight to about 5 mg/kg of body weight. In other parenteral or oral administration, an amount based on the aforementioned amount may be administered. When symptom is particularly severe, the amount can be increased depending on the symptom.

The antibody of the present invention may be administered as it is or as an appropriate pharmaceutical composition. The pharmaceutical composition used in the administration described above comprises the aforementioned antibody or a salt thereof, and a pharmacologically acceptable carrier, diluent or excipient. Such composition is provided as a dosage form suitable for oral or parenteral administration.

In addition, each composition described above may comprise other active ingredients unless undesirable interactions occurs by blending with the antibody.

### [7] Preparation of an animal having the DNA of the present invention

The present invention provides a non-human mammal bearing DNA encoding the protein of the present invention, which is exogenous (hereinafter abbreviated as the exogenous DNA of the present invention) or its variant DNA (sometimes simply referred to as the exogenous variant DNA of the present invention).

Thus, the present invention provides:
(1) A non-human mammal bearing the exogenous DNA or its variant DNA;
(2) The mammal according to (1), wherein the non-human mammal is a rodent;
(3) The mammal according to (2), wherein the rodent is mouse or rat; and,
(4) A recombinant vector bearing the exogenous DNA of the present invention or its variant DNA and capable of expressing in a mammal.
The non-human mammal bearing the exogenous DNA of the present invention or its variant DNA (hereinafter simply referred to as the DNA transgenic animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method etc. In addition, it is possible to transfect the exogenous DNA of the present invention into a somatic cell, a living organ, a tissue cell, or the like by the DNA transfection methods, and utilize the transformant for cell culture, tissue culture, etc. Further, these cells may be fused with the above-described germinal cell by a publicly known cell fusion method to prepare the transgenic animal of the present invention.

Examples of the non-human mammal that can be used include bovine, swine, sheep, goat, rabbits, dogs, cats, guinea pigs, hamsters, mice, rats and the like. Above all, preferred are rodents, especially mice (e.g., C57B1/6 strain, DBA2 strain, etc. for a pure line and for a cross line, B6C3F₁ strain, BDF₁ strain B6D2F₁ strain, BALB/c strain, ICR strain, etc.) or rats (Wistar, SD, etc.), since they are relatively short in ontogeny and life cycle from a standpoint of preparing model animals for human disease.

"Mammals" in a recombinant vector that can be expressed in the mammals include the aforesaid non-human mammals and human.

The exogenous DNA of the present invention refers to the DNA of the present invention that is once isolated and extracted from mammals, not the DNA of the present invention inherently possessed by the non-human mammals.

The mutant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean DNA that expresses the abnormal protein of the present invention and exemplified by DNA that expresses a polypeptide for suppressing the functions of the normal protein of the present invention.

The exogenous DNA of the present invention may be any one of those derived from a mammal of the same species as, or a different species from, the mammal as the target animal. In transfecting the DNA of the present invention, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target non-human mammal downstream various promoters which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the polypeptide of the present invention, there are *Escherichia* coli-derived plasmids, *Bacillus subtilis-derived* plasmids, yeast-derived plasmids, bacteriophages such as λ phage, retroviruses such as Moloney leukemia virus, etc., and animal viruses such as vaccinia virus, baculovirus, etc. Of these vectors, *Escherichia* coli-derived plasmids, *Bacillus* subtilis-derived plasmids, or yeast-derived plasmids, etc. are preferably used.

Examples of these promoters for regulating the DNA expression include (1) promoters for DNA derived from viruses (e.g., simian virus, cytomegalovirus, Moloney leukemia virus, JC virus, breast cancer virus, poliovirus, etc.), and (2) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), polypeptide chain elongation factor 1a (EF-1a), β actin, a and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle, a actin, preproencephalin A, vasopressin, etc. Among them, cytomegalovirus promoters, human polypeptide elongation factor 1a (EF-1a) promoters, human and chicken β actin promoters etc., which protein can highly express in the whole body are preferred.

It is preferred that the vectors described above have a sequence for terminating the transcription of the desired messenger RNA in the DNA transgenic animal (generally termed terminator); for example, a sequence of each DNA derived from viruses and various mammals. SV40 terminator of the simian virus and the like are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal protein of the present invention can be obtained using as a starting material the entire genomic DNA or its portion of liver, kidney, thyroid cell or fibroblast origin from human or various mammals (e.g., rabbits, dogs, cats, guinea pigs, hamsters, rats, mice, etc.) or of various commercially available genomic DNA libraries, or using complementary DNA prepared by a publicly known method from RNA of liver, kidney, thyroid cell or fibroblast origin as a starting material. Alternatively, the translational region for a normal polypeptide translational region obtained by the cell or tissue described above can be made variant by point mutagenesis.

The translational region can be prepared by a conventional DNA engineering technique in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by mating.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the DNA of the present invention is excessively present in all of the germinal cells and somatic cells thereof. The offspring of the animal that inherits the exogenous DNA of the present invention have excessively the DNA of the present invention in all of the germinal cells and somatic cells thereof.

By obtaining a homozygotic animal having the transfected DNA in both of homologous chromosomes and mating a male and female of the animal, all offspring can be passaged to retain the DNA.

In a non-human mammal bearing the normal DNA of the present invention, the normal DNA of the present invention has expressed to a high level, and may eventually develop the function inactive type inadaptability of the protein of the present invention by accelerating the functions of endogenous normal DNA. Therefore, the animal can be utilized as a pathologic model animal for such a disease. Specifically, using the normal DNA transgenic animal of the present invention, it is possible to elucidate the mechanism of the function inactive type inadaptability of the protein of the present invention and the pathological mechanism of the disease associated with the protein of the present invention and to determine how to treat the disease.

Further, since a mammal transfected the exogenous normal DNA of the present invention exhibits an increasing symptom of the protein of the present invention librated, the animal is usable for screening of treatment agent for the disease associated with the protein of the present invention.

On the other hand, non-human mammal having the exogenous abnormal DNA of the present invention can be passaged under normal breeding conditions as the DNA-bearing animal by confirming the stable retaining of the exogenous DNA via crossing. Further, the exogenous DNA to be subjected can be utilized as a starting material by inserting the DNA into the plasmid described above. The DNA construct with promoter can be prepared with conventional DNA engineering techniques. The transfection of the abnormal DNA of the present invention at the fertilized egg cell stage is preserved to be present in all of the germinal and somatic cells of the mammals to be subjected. The fact that the abnormal DNA of the present invention is present in the germinal cells of the animal after DNA transfection means that all of the offspring of the prepared animal have the abnormal DNA of the present invention in all of the germinal and somatic cells. Such an offspring passaged the exogenous DNA of the present invention contains the abnormal DNA of the present invention in all of the germinal and somatic cells. A homozygous animal having the introduced DNA on both of homologous chromosomes can be acquired and then by mating these male and female animals, all the offspring can be bled to have the DNA.

Since non-human mammal having the abnormal DNA of the present invention may express the abnormal DNA of the present invention at a high level, the animal may be the function inactivation type inadaptability of the protein of the present invention by inhibiting the function of the endogenous normal DNA and can be utilized as its disease model animal. For example, using the abnormal DNA-transferred animal of the present invention, it is possible to elucidate the mechanism of inadaptability of the protein of the present invention and to perform to study a method for treatment of this disease.

More specifically, the transgenic animal of the present invention expressing the abnormal DNA of the present invention to a high level is also expected to serve as an experimental model for the elucidation of the mechanism of the functional inhibition (dominant negative effect) of normal protein by the abnormal protein of the present invention in the function inactive type inadaptability of the protein of the present invention.

A mammal bearing the abnormal exogenous DNA of the present invention is also expected to serve for screening a candidate drug for the treatment of the function inactive type inadaptability of the protein of the present invention, since the protein of the present invention is increased in such an animal in its free form.

Other potential applications of two kinds of the transgenic animals described above include:
(i) Use as a cell source for tissue culture;
(ii) Elucidation of the relation to a protein that is specifically expressed or activated by the protein of the present invention, by direct analysis of DNA or RNA in tissue of the DNA transgenic animal of the present invention or by analysis of the polypeptide tissue expressed by the DNA;
(iii) Research in the function of cells derived from tissues that are cultured usually only with difficulty, using cells of tissue bearing the DNA cultured by a standard tissue culture technique;
(iv) Screening for a medicine that enhances the functions of cells using the cells described in (iii) above; and
(v) Isolation and purification of the variant protein of the present invention and preparation of an antibody thereto.

Furthermore, clinical conditions of a disease associated wit the protein of the present invention, including the function inactive type inadaptability of the protein of the present invention can be determined using the DNA transgenic animal of the present invention. In addition, pathological findings on each organ in a disease model associated with the protein of the present invention can be obtained in more detail, leading to the development of a new method for treatment as well as the research and therapy of any secondary diseases associated with the disease.

It is also possible to obtain a free DNA-transfected cell by withdrawing each organ from the DNA transgenic animal of the present invention, mincing the organ and degrading with a proteinase such as trypsin, etc., followed by establishing the line of culturing or cultured cells. Furthermore, the DNA transgenic animal of the present invention can serve as identification of cells capable of producing the protein of the present invention, and as studies on association with apoptosis, differentiation or propagation or on the mechanism of signal transduction in these properties to inspect any abnormality therein. Thus the DNA transgenic animal of the present invention can provide an effective research material for the protein of the present invention and for elucidating the function and effect thereof.

To develop a therapeutic drug for the treatment of diseases associated with the protein of the present invention, including the function inactive type inadaptability of the protein of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the protein of the present invention, using the DNA transgenic animal of the present invention or a vector capable of expressing the exogenous DNA of the present invention.

### [8] Knockout animals

The present invention provides a non-human mammalian embryonic stem cell, wherein the DNA of the present invention is inactivated, and a non-human mammal deficient in expressing the DNA of the present invention.

That is, the present invention provides:
(1) A non-human embryonic stem cell in which the DNA of the present invention is inactivated;
(2) The embryonic stem cell according to (1), wherein the DNA is inactivated by introducing a reporter gene (e.g., β-galactosidase gene derived from Escherichia coli);
(3) The embryonic stem cell according to (1), which is resistant to neomycin;
(4) The embryonic stem cell according to (1), wherein the non-human mammal is a rodent;
(5) The embryonic stem cell according to (4), wherein the rodent is mouse;
(6) A non-human mammal deficient in expressing the DNA of the present invention, wherein the DNA of the present invention is inactivated;
(7) The non-human mammal according to (6), wherein the DNA is inactivated by inserting a reporter gene (e.g., β-galactosidase derived from Escherichia coli) therein and the reporter gene is capable of being expressed under control of a promoter for the DNA of the present invention;
(8) The non-human mammal according to (6), which is a rodent;
(9) The non-human mammal according to (8), wherein the rodent is mouse; and
(10) A method for screening a compound or its salt that promotes or inhibits the promoter activity for the DNA of the present invention, which comprises administering a test compound to the mammal of (7) and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the polypeptide of the present invention (hereinafter sometimes referred to as the knockout DNA of the present invention) by substantially inactivating the activities of the polypeptide of the present invention encoded by the DNA (hereinafter merely referred to as ES cell).

As the non-human mammal, the same examples as described above apply.

Methods for artificially mutating the DNA of the present invention include, for example, deletion of a part or all of the DNA sequence and insertion of or substitution with other DNA, by genetic engineering. By these mutations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating to a chromosome of the subject animal by, e.g., homologous recombination, a DNA sequence which terminates gene transcription (e.g., polyA additional signal, etc.) in the intron between exons to, thus inhibit the synthesis of complete messenger RNA and eventually destroy the gene (hereinafter simply referred to as targeting vector). The thus-obtained ES cells to the Southern hybridization analysis with a DNA sequence on or near the DNA of the present invention as a probe, or to PCR analysis with a DNA sequence on the targeting vector and another DNA sequence near the DNA of the present invention which is not included in the targeting vector as primers, to select the knockout ES cell of the present invention.

The parent ES cells to inactivate the DNA of the present invention by homologous recombination, etc. may be of a strain already established as described above, or may be originally established in accordance with a modification of the publicly known method by Evans and Kaufman *supra.* For example, in the case of mouse ES cells, currently it is common practice to use ES cells of the 129 strain. However, since their immunological background is obscure, the C57BL/6 mouse or the BDF₁ mouse (F₁ hybrid between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The BDF₁ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. Except for this fact, by collecting embryos at the 8-cell stage and culturing those until the blastocyte stage, a large number of early stage embryos may efficiently be obtained.

Although the ES cells used may be of either sex, male ES cells are generally more convenient for generation of a germ cell line chimera and are therefore preferred. It is desirable to identify sexes as soon as possible also in order to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about 10⁶ cells; therefore, the first selection of ES cells at the early stage of culture can be based on sex identification, and male cells can be selected early, which saves a significant amount of time at the early stage of culture.

Second selection can be achieved by, for example, number of chromosome confirmation by the G-banding method. It is usually desirable that the chromosome number of the obtained ES cells be 100% of the normal number. However, when it is difficult to obtain the cells having the normal number of chromosomes due to physical operation etc. in cell establishment, it is desirable that the ES cell be again cloned to a normal cell (e.g., in mouse cells having the number of chromosomes being 2n = 40) after the gene of the ES cells is rendered knockout.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably about 5% carbon dioxide and about 95% air, or about 5% oxygen, about 5% carbon dioxide and 90% air) in the presence of LIF (1-10000 U/ml) on appropriate feeder cells such as STO fibroblasts, treated with a trypsin/EDTA solution (normally about 0.001 % to about 0.5% trypsin/about 0.1 to about 5 mM EDTA, preferably about 0.1% trypsin/1 mM EDTA) at the time of passage to obtain separate single cells, which are then seeded on freshly prepared feeder cells. This passage is normally conducted every 1 to 3 days; it is desirable that cells be observed at passage and cells found to be morphologically abnormal in culture, if any, be abandoned.

By allowing ES cells to reach a high density in mono-layers or to form cell aggregates in suspension under appropriate conditions, it is possible to spontaneously differentiate them to various cell types, for example, pariental muscle, visceral muscles, cardiac muscle or the like (M. J. Evans and M. H. Kaufman, Nature, 292, 154, 1981; G R. Martin, Proc. Natl. Acad. Sci. U.S.A., 78, 7634, 1981; T. C. Doetschman et al., Journal of Embryology Experimental Morphology, 87, 27, 1985). The cells deficient in expression of the DNA of the present invention, which are obtainable from the differentiated ES cells of the present invention, are useful for studying the functions of the polypeptide of the present invention cytologically or molecular biologically.

The non-human mammal deficient in expression of the DNA of the present invention can be identified from a normal animal by measuring the amount of mRNA in the subject animal by a publicly known method, and indirectly comparing the degrees of expression.

As the non-human mammal, the same examples *supra* can be applied.

With respect to the non-human mammal deficient in expression of the DNA of the present invention, the DNA of the present invention can be made knockout by transfecting a targeting vector, prepared as described above, to mouse embryonic stem cells or mouse oocytes, and conducting homologous recombination in which a targeting vector DNA sequence, wherein the DNA of the present invention is inactivated by the transfection, is replaced with the DNA of the present invention on a chromosome of a mouse embryonic stem cell or mouse oocyte.

The knockout cells with the DNA of the present invention disrupted can be identified by Southern hybridization analysis with a DNA sequence on or near the DNA of the present invention as a probe, or by PCR analysis using a DNA sequence on the targeting vector and another DNA sequence derived from mouse, which is not included in the targeting vector, as primers. When non-human mammalian embryonic stem cells are used, a cell line wherein the DNA of the present invention is inactivated by homologous recombination is cloned; the resulting cloned cell line is injected to, e.g., a non-human mammalian embryo or blastocyte, at an appropriate stage such as the 8-cell stage. The resulting chimeric embryos are transplanted to the uterus of the pseudopregnant non-human mammal. The prepared animal is a chimeric animal composed of both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the receptor protein of the present invention. The individuals deficient in homozygous expression of the receptor protein of the present invention can be obtained from offspring of the intercross between the heterozygotes.

When an oocyte is used, a DNA solution may be injected, e.g., to the nucleus of the oocyte by microinjection thereby to obtain a transgenic non-human mammal having a targeting vector introduced in a chromosome thereof. From such transgenic non-human mammals, those having a mutation at the locus of the DNA of the present invention can be obtained by selection based on homologous recombination.

As described above, individuals in which the DNA of the present invention is rendered knockout permit passage rearing under ordinary rearing conditions, after the individuals obtained by their crossing have proven to have been knockout.

Furthermore, the genital system may be obtained and maintained by conventional methods. That is, by crossing male and female animals each having the inactivated DNA, homozygoous animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell in which the DNA of the present invention is inactivated is very useful for preparing a non-human mammal deficient in expression of the DNA of the present invention.

Since the non-human mammal in which the DNA of the present invention is inactivated lacks various biological activities derived from the protein of the present invention, such an animal can be a disease model suspected of inactivated biological activities of the protein of the present invention and thus, offers an effective study to investigate causes for and therapy for these diseases.

### [8a] A Method for screening of compounds having therapeutic and/or prophylactic effects for diseases caused by deficiency, damages, etc. of the DNA of the present invention

The non-human mammal deficient in expression of the DNA of the present invention can be employed for the screening of compounds having therapeutic and/or prophylactic effects for diseases caused by deficiency, damages, etc. of the DNA of the present invention.

That is, the present invention provides a method for screening of a compound having therapeutic and/or prophylactic effects for diseases caused by deficiency, damages, etc. of the DNA of the present invention, which comprises administering a test compound to the non-human mammal deficient in expression of the DNA of the present invention and observing and measuring a change occurred in the animal.

As the non-human mammal deficient in expression of the DNA of the present invention, which can be employed for the screening method, the same examples as given hereinabove apply.

Examples of the test compounds include peptides, proteins, non-peptide compounds, synthetic compounds, fermentation products, cell extracts, plant extracts, animal tissue extracts, blood plasma and the like. These compounds may be novel compounds or publicly known compounds.

Specifically, the non-human mammal deficient in the expression of the DNA of the present invention is treated with a test compound, and by comparison with an intact animal for control, a change in each organ, tissue, disease conditions, etc. of the animal is used as an index to assess the therapeutic and/or prophylactic effects of the test compound.

For treating an animal to be tested with a test compound, for example, oral administration, intravenous injection, etc. are applied and the treatment is appropriately selected depending upon conditions of the test animal, properties of the test compound, etc. Furthermore, an amount of a test compound administered can be selected depending on administration route, nature of the test compound, and the like.

For example, in the case of screening a compound having a therapeutic and/or prophylactic effect for irritable bowel syndrome, a test compound may be administered to the non-human mammal deficient in the expression of the DNA of the present invention. Then changes in amount of bowel movement depending on stress caused by restriction, is measured with time.

The compound obtained using the above screening method is a compound selected from the test compounds described above and exhibits a therapeutic and/or prophylactic effect for the diseases caused by deficiencies, damages, etc. of the protein of the present invention. Therefore, the compound can be employed as a safe and low toxic pharmaceutical such as the prophylactic and/or therapeutic agent for these diseases. Furthermore, compounds derived from such a compound obtained by the above screening can be likewise employed.

The compound obtained by the screening method may be used in the form of salts with physiologically acceptable acids (e.g., inorganic acids or organic acids) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

A pharmaceutical containing the compound obtained by the above screening method or salts thereof may be manufactured in a manner similar to the method for preparing the pharmaceutical comprising the protein of the present invention described hereinabove.

Since the preparation thus obtained is safe and low toxic, it can be administered to human or other mammals (e.g., rat, mouse, guinea pig, rabbit, sheep, swine, bovine, horse, cat, dog, monkey, etc.).

Although the amount of the compound or its salt to be administered varies depending upon particular disease, subject to be administered, route of administration, etc., but when the compound is orally administered, the compound is generally administered to an adult patient with irritable bowel syndrome (as 60 kg body weight) in a dose of about 0.1 mg/day to about 100 mg/day, preferably about 1.0 mg/day to about 50 mg/day, more preferably about 1.0 mg to about 20 mg. For parenteral administration, a single dose of the compound varies depending upon subject to be administered, target disease, etc., but when the compound is administered to an adult patient with irritable bowel syndrome (as 60 kg body weight) in the form of an injectable preparation, it is generally advantageous to administer the compound intravenously in a dose of about 0.01 mg/day to about 30 mg/day, preferably about 0.1 mg/day to about 20 mg/day, more preferably about 0.1 mg/day to about 10 mg/day. As for other animals, the compound can be administered in the above amount with converting it into that for the body weight of 60 kg.

### [8b] A Method for screening a compound that promotes or inhibits the activities of a promoter to the DNA of the present invention

The present invention provides a method for screening a compound or its salt that promotes or inhibits the activities of a promoter to the DNA of the present invention, which comprises administering a test compound to a non-human mammal deficient in expression of the DNA of the present invention and detecting expression of the reporter gene.

In the screening method described above, as the non-human mammal deficient in expression of the DNA of the present invention, an animal in which the DNA of the present invention is inactivated by introducing a reporter gene and the reporter gene can be expressed under control of a promoter to the DNA of the present invention is used from the aforementioned non-human mammal deficient in expression of the DNA of the present invention.

The same examples of the test compound apply to specific compounds used for the screening.

As the reporter gene, the same specific examples described above apply, and β-galactosidase (lacZ), soluble alkaline phosphatase gene, luciferase gene and the like are preferably employed.

Since a reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the protein of the present invention is substituted with, e.g., β-galactosidase gene (lacZ) derived from Escherichia coli, β-galactosidase is expressed in a tissue where the protein of the present invention should originally be expressed, instead of the protein of the present invention. Thus, the state of expression of the protein of the present invention can be readily observed in vivo of an animal by staining with a reagent such as 5-bromo-4-chloro-3-indolyl-β-galactopyranoside (X-gal), which is substrate for β-galactosidase. Specifically, a mouse deficient in the protein of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After that, by washing the tissue preparation with 1 mM EDTA/PBS solution, the β-galactosidase reaction is terminated, and the color formed is observed. Alternatively, mRNA encoding lacZ may be detected in a conventional manner.

The compound or salts thereof obtained using the above screening method, are compounds that are selected from the test compounds described above and that promote or inhibit the promoter activity to the DNA of the present invention.

The compound obtained by the screening method above may be used in the form of salts with physiologically acceptable acids (e.g., inorganic acids or organic acids) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like. Since the compounds or salts thereof that enhance the promoter activity to the DNA of the present invention can promote the expression of the protein of the present invention, or can promote the functions of the protein, they are useful as pharmaceuticals such as the prophylactic and/or therapeutic agent for alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like.

Further, the compounds or salts thereof that inhibit the promoter activity to the DNA of the present invention can inhibit the expression of the protein of the present invention, or can inhibit the functions of the protein, they are useful as a prophylactic and/or therapeutic agent for alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like.

In addition, compound derived from the compounds obtained by the screening above may be likewise employed.

A pharmaceutical containing the compounds or salts thereof obtained by the screening method may be manufactured in a manner similar to the method for preparing the pharmaceutical containing the protein of the present invention described above.

Since the pharmaceutical preparation thus obtained is safe and low toxic, it can be administered to human or other mammals (e.g., rat, mouse, guinea pig, rabbit, sheep, swine, bovine, horse, cat, dog, monkey, etc.).

The dose of the compound or salts thereof varies depending on target disease, subject to be administered, route for administration, etc.; for example, when the compound that enhances the promoter activity to the DNA of the present invention is orally administered, the dose is normally about 0.1 to about 100 mg, preferably about 1.0 to about 50 mg, more preferably about 1.0 to about 20 mg per day for adult patient with irritable bowel syndrome (as 60 kg body weight). In parenteral administration, a single dose of the compound varies depending on subject to be administered, target disease, etc. but when the compound that enhances the promoter activity to the DNA of the present invention is administered in the form of injectable preparation, it is advantageous to administer the compound intravenously at a single dose of about 0.01 to about 30 mg/day, preferably about 0.1 to about 20 mg/day, more preferably about 0.1 to about 10 mg/day for adult patient with irritable bowel syndrome (as 60 kg body weight). For other animal species, the corresponding dose as converted per 60 kg weight can be administered.

On the other hand, when the compound that inhibits the promoter activity to the DNA of the present invention is orally administered, the dose is normally about 0.1 to about 100 mg, preferably about 1.0 to about 50 mg, more preferably about 1.0 to about 20 mg per day for adult patient with irritable bowel syndrome (as 60 kg body weight). In parenteral administration, a single dose of the compound varies depending on subject to be administered, target disease, etc. but when the compound that inhibits the promoter activity to the DNA of the present invention is administered in the form of injectable preparation, it is advantageous to administer the compound intravenously at a single dose of about 0.01 to about 30 mg/day, preferably about 0.1 to about 20 mg/day, more preferably about 0.1 to about 10 mg/day for adult patient with irritable bowel syndrome (as 60 kg body weight). For other animal species, the corresponding dose as converted per 60 kg weight can be administered.

As stated above, the non-human mammal deficient in expression of the DNA of the present invention is extremely useful for screening the compound or its salt that promotes or inhibits the activity of a promoter to the DNA of the present invention and can greatly contribute to the elucidation of causes for various diseases suspected of deficiency in expression of the DNA of the present invention and for the development of medicine for prevention and/or treatment of these diseases.

Furthermore, a so-called transgenic animal (gene introduced animal) can be prepared by using DNA containing a promoter region of the protein of the present invention, ligating genes encoding various proteins downstream and injecting the same into oocyte of an animal. It is then possible to synthesize the protein therein specifically and study its activity in vivo. When an appropriate reporter gene is ligated to the promoter site above and a cell line that express the gene is established, the resulting system can be utilized as the survey system for a low molecular compound having the action of specifically promoting or inhibiting the in vivo productivity of the protein per se of the present invention.

In the specification and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.
- DNA: : deoxyribonucleic acid
- cDNA: : complementary deoxyribonucleic acid
- A: : adenine
- T: : thymine
- G: : guanine
- C: : cytosine
- RNA: : ribonucleic acid
- mRNA: : messenger ribonucleic acid
- dATP: : deoxyadenosine triphosphate
- dTTP: : deoxythymidine triphosphate
- dGTP: : deoxyguanosine triphosphate
- dCTP: : deoxycytidine triphosphate
- ATP: : adenosine triphosphate
- EDTA: : ethylenediaminetetraacetic acid
- SDS: : sodium dodecyl sulfate
- Gly: : glycine
- Ala: : alanine
- Val: : valine
- Leu: : leucine
- Ile: : isoleucine
- Ser: : serine
- Thr: : threonine
- Cys: : cysteine
- Met: : methionine
- Glu: : glutamic acid
- Asp: : aspartic acid
- Lys: : lysine
- Arg: : arginine
- His: : histidine
- Phe: : phenylalanine
- Tyr: : tyrosine
- Trp: : tryptophan
- Pro: : proline
- Asn: : asparagine
- Gln: : glutamine
- pGlu: : pyroglutamic acid

The substituents, protective groups and reagents, which are frequently used throughout the specification, are shown by the following abbreviations.
- Me: : methyl group
- Et: : ethyl group
- Bu: : butyl group
- Ph: : phenyl group
- TC: : thiazolidine-4(R)-carboxamide group
- Tos: : p-toluenesulfonyl
- CHO: : formyl
- Bzl: : benzyl
- Cl₂-Bzl: : 2,6-dichlorobenzyl
- Bom: : benzyloxymethyl
- Z: : benzyloxycarbonyl
- Cl-Z: : 2-chlorobenzyloxycarbonyl
- Br-Z: : 2-bromobenzyloxycarbonyl
- Boc: : t-butoxycarbonyl
- DNP: : dinitrophenol
- Trt: : trityl
- Bum: : t-butoxymethyl
- Fmoc: : N-9-fluorenylmethoxycarbonyl
- HOBt: : 1-hydroxybenztriazole
- HOOBt: :3,4-dihydro-3-hydroxy-4-oxo-1,2,3-benzotriazine
- HONB: :1-hydroxy-5-norbornene-2,3-dicarboximide
- DCC: : N,N'-dicyclohexylcarbodiimide

The sequence identification numbers in the sequence listing of the specification indicates the following sequence, respectively.

### [SEQ ID NO: 1]

This shows the amino acid sequence of human TCH204 variant 1 protein, which was acquired in EXAMPLE 1.

### [SEQ ID NO: 2]

This shows the base sequence of DNA encoding human TCH204 variant 1 protein, which possesses the amino acid sequence represented by SEQ ID NO: 1.

### [SEQ ID NO: 3]

This shows the base sequence of synthetic DNA primer used in EXAMPLE 1 and EXAMPLE 2.

### [SEQ ID NO: 4]

This shows the base sequence of synthetic DNA primer used in EXAMPLE 1 and EXAMPLE 2.

### [SEQ ID NO: 5]

This shows the base sequence of synthetic DNA primer used in EXAMPLE 1, EXAMPLE 2 and EXAMPLE 4.

### [SEQ ID NO: 6]

This shows the base sequence of synthetic DNA primer used in EXAMPLE 1, EXAMPLE 2 and EXAMPLE 4.

### [SEQ ID NO: 7]

This shows the base sequence of DNA encoding human TCH204 variant 1, which was acquired in EXAMPLE 1 and inserted into plasmid.

### [SEQ ID NO: 8]

This shows the amino acid sequence of human TCH204 variant 2 protein, which was acquired in EXAMPLE 2.

### [SEQ ID NO: 9]

This shows the base sequence of DNA encoding human TCH204 variant 2 protein, which possesses the amino acid sequence represented by SEQ ID NO: 8.

### [SEQ ID NO: 10]

This shows the base sequence of DNA encoding human TCH204 variant 2, which was acquired in EXAMPLE 2 and inserted into plasmid.

### [SEQ ID NO: 11]

This shows the amino acid sequence of human TCH204 variant 3 protein, which was acquired in EXAMPLE 3.

### [SEQ ID NO: 12]

This shows the base sequence of DNA encoding human TCH204 variant 3 protein, which possesses the amino acid sequence represented by SEQ ID NO: 11.

### [SEQ ID NO: 13]

This shows the base sequence of DNA encoding human TCH204 variant 3, which was acquired in EXAMPLE 3 and inserted into plasmid.

### [SEQ ID NO: 14]

This shows the base sequence of synthetic DNA primer used in EXAMPLE 4.

### [SEQ ID NO: 15]

This shows the amino acid sequence of human TCH204 variant 4 protein, which was acquired in EXAMPLE 4.

### [SEQ ID NO: 16]

This shows the base sequence of DNA encoding human TCH204 variant 4 protein, which possesses the amino acid sequence represented by SEQ ID NO: 15.

### [SEQ ID NO: 17]

This shows the base sequence of DNA encoding human TCH204 variant 4, which was acquired in EXAMPLE 4 and inserted into plasmid.

### [SEQ ID NO: 18]

This shows the amino acid sequence of mouse TCH204 variant 6 protein, which was acquired in EXAMPLE 5.

### [SEQ ID NO: 19]

This shows the base sequence of DNA encoding mouse TCH204 variant 6 protein, which possesses the amino acid sequence represented by SEQ ID NO: 18.

### [SEQ ID NO: 20]

This shows the base sequence of primer F02 used in EXAMPLE 5.

### [SEQ ID NO: 21]

This shows the base sequence of primer R02 used in EXAMPLE 5.

### [SEQ ID NO: 22]

This shows the base sequence of DNA encoding mouse TCH204 variant 6, which was acquired in EXAMPLE 5 and inserted into plasmid.

### [SEQ ID NO: 23]

This shows the amino acid sequence of mouse TCH204 variant 5 protein, which was acquired in EXAMPLE 6.

### [SEQ ID NO: 24]

This shows the base sequence of DNA encoding mouse TCH204 variant 5 protein, which possesses the amino acid sequence represented by SEQ ID NO: 23.

### [SEQ ID NO: 25]

This shows the base sequence of DNA encoding mouse TCH204 variant 5, which was acquired in EXAMPLE 6 and inserted into plasmid.

### [SEQ ID NO: 26]

This shows the amino acid sequence of mouse TCH204 variant 2 protein, which was acquired in EXAMPLE 7.

### [SEQ ID NO: 27]

This shows the base sequence of DNA encoding mouse TCH204 variant 2 protein, which possesses the amino acid sequence represented by SEQ ID NO: 26.

### [SEQ ID NO: 28]

This shows the base sequence of DNA encoding mouse TCH204 variant 2, which was acquired in EXAMPLE 7 and inserted into plasmid.

### [SEQ ID NO: 29]

This shows the base sequence of synthetic DNA primer used in EXAMPLE 8.

### [SEQ ID NO: 30]

This shows the base sequence of synthetic DNA primer used in EXAMPLE 8.

### [SEQ ID NO: 31]

This shows the base sequence of synthetic DNA probe for TaqMan PCR used in EXAMPLE 8.

### [SEQ ID NO: 32]

This shows the base sequence of synthetic DNA primer used in EXAMPLE 8.

### [SEQ ID NO: 33]

This shows the base sequence of synthetic DNA probe for TaqMan PCR used in EXAMPLE 8.

### [SEQ ID NO: 34]

This shows the base sequence of synthetic DNA primer used in EXAMPLE 8.

### [SEQ ID NO: 35]

This shows the base sequence of synthetic DNA probe for TaqMan PCR used in EXAMPLE 8.

### [SEQ ID NO: 36]

This shows the base sequence of synthetic DNA primer used in EXAMPLE 8.

### [SEQ ID NO: 37]

This shows the base sequence of synthetic DNA primer used in EXAMPLE 8.

### [SEQ ID NO: 38]

This shows the base sequence of synthetic DNA probe for TaqMan PCR used in EXAMPLE 8.

### [SEQ ID NO: 39]

This shows the base sequence of synthetic DNA primer used in EXAMPLE 8.

### [SEQ ID NO: 40]

This shows the base sequence of synthetic DNA primer used in EXAMPLE 8.

### [SEQ ID NO: 41]

This shows the base sequence of synthetic DNA probe for TaqMan PCR used in EXAMPLE 8.

### [SEQ ID NO: 42]

This shows the base sequence of synthetic DNA primer used in EXAMPLE 8.

### [SEQ ID NO: 43]

This shows the base sequence of synthetic DNA primer used in EXAMPLE 8.

### [SEQ ID NO: 44]

This shows the base sequence of synthetic DNA probe for TaqMan PCR used in EXAMPLE 8.

The transformant Escherichia coli TOPO10/pCRII-TCH204V1 obtained in EXAMPLE 1 described below was as Escherichia coli TOP10/pCRII-TCH204V1 on deposit with International Patent Organisms Depository, National Institute of Advanced Industrial Science and Technology, located at Central 6, 1-1-1 Higashi, Tsukuba, Ibaraki, 305-8566, Japan, as the Accession Number FERM BP-8053 on May 27, 2002 and with Institute for Fermentation (IFO), located at 2-17-85 Juso-honmachi, Yodogawa-ku, Osaka-shi, Osaka, Japan, as the Accession Number IFO 16794 on May 14, 2002.

The transformant Escherichia coli TOPO10/pCRII-TCH204V2 obtained in EXAMPLE 2 described below was as Escherichia coli TOP10/pCRII-TCH204V2 on deposit with International Patent Organisms Depository, National Institute of Advanced Industrial Science and Technology, located at Central 6, 1-1-1 Higashi, Tsukuba, Ibaraki, 305-8566, Japan, as the Accession Number FERM BP-8054 on May 27, 2002 and with Institute for Fermentation (IFO), located at 2-17-85 Juso-honmachi, Yodogawa-ku, Osaka-shi, Osaka, Japan, as the Accession Number IFO 16795 on May 14, 2002. The transformant Escherichia coli TOPO10/pCRII-mTCH204V6 obtained in EXAMPLE 5 described below was as Escherichia coli TOP10/pCRII-mTCH204V6 on deposit with International Patent Organisms Depository, National Institute of Advanced Industrial Science and Technology, located at Central 6, 1-1-1 Higashi, Tsukuba, Ibaraki, 305-8566, Japan, as the Accession Number FERM BP-8119 on July 16, 2002.

The present invention is described in detail below with reference to EXAMPLES, but is not deemed to limit the scope of the present invention thereto. The gene manipulation procedures using Escherichia coli were performed according to the methods described in the Molecular Cloning.

### EXAMPLE 1

### Cloning of cDNA coding for human TCH204 variant 1 gene

Using the following synthetic DNA primers and human lung-derived cDNA as a template, amplification by PCR method was performed. The reaction solution comprised of 2.0 µl of human lung Marathon-Ready cDNA (CLONTECH), 2 µM each of synthetic DNA primers (SEQ ID NO: 3 and SEQ ID NO: 4), 0.2 mM dNTPs, 0.125 µl of Pyrobest DNA Polymerase (Takara Shuzo) and Pyrobest buffer attached to the enzyme to make the total volume 20 µl. The cycle for amplification was carried out using a thermal cycler (PE Biosystems) by heating of 94°C for 5 minutes, then a cycle set to include 94°C for 20 seconds followed by 72°C for 5 minutes, which was repeated 5 times, 94°C for 20 seconds followed by 70°C for 5 minutes, which was repeated 5 times, 94°C for 20 seconds followed by 68°C for 5 minutes, which was repeated 30 times, and finally, incubation at 72°C for 5 minutes. Then, the reaction solution comprised of 5 µl of the PCR reaction solution diluted 50-fold with DNase-, RNase-free distilled water, 2 µM each of synthetic DNA primers (SEQ ID NO: 5 and SEQ ID NO: 6), 0.2 mM dNTPs, 0.313 µl of Pyrobest DNA Polymerase (Takara Shuzo) and Pyrobest buffer attached to the enzyme to make the total volume 50 µl. The cycle for amplification was carried out using a thermal cycler (PE Biosystems) by heating of 94°C for 5 minutes, then a cycle set to include 94°C for 20 seconds followed by 72°C for 5 minutes, which was repeated 5 times, 94°C for 20 seconds followed by 70°C for 5 minutes, which was repeated 5 times, 94°C for 20 seconds followed by 68°C for 5 minutes, which was repeated 30 times, and finally, incubation at 72°C for 5 minutes. The amplified DNA was isolated by 1.5% agarose gel electrophoresis and about 3.5 kb band was excised by razor blade. The DNA was recovered using the QIAquick Gel Extraction Kit (QIAGEN). The DNA was cloned into pCRII-TOPO vector in accordance with the protocol of TOPO TA Cloning Kit Dual Promoter (Invitrogen). After transformation of Escherichia coli TOP10 competent cell (Invitrogen) by introducing the above-mentioned vector, clones harboring cDNA insert fragment were selected on LB agar medium containing kanamycin and X-gal. All the white-colored clones were isolated with sterilized toothpick, and then the transformants were obtained. Respective clones were cultured in LB medium containing kanamycin for overnight. Subsequently, the plasmid DNA was prepared using QIAprep Miniprep (QIAGEN). The reaction for determination of the base sequence was carried out using BigDye Terminator Cycle Sequencing Ready Reaction Kit (Applied Biosystems). As a result, with the DNA sequencer, ABI PPISM 3100 Analyzer (Applied Biosystems), the base sequence of cDNA fragment inserted was decoded.

The 3517 bases were inserted into the acquired plasmid (SEQ ID NO: 7), and ORF encoding 1118 amino acid residues were present in the cDNA fragment (SEQ ID NO: 1). The protein containing the amino acid sequence represented by SEQ ID NO: 1 was designated human TCH204 variant 1 protein. The transformant having the plasmid containing the above-mentioned cDNA fragment (SEQ ID NO: 7) was designated Escherichia coli TOP10/pCRII-TCH204V1.

Whereas the 2916th base in the sequence represented by SEQ ID NO: 2 was A, the corresponding base of the other human TCH204 variant obtained in EXAMPLES 2, 3 and 4 described later was G It is considered that the base substitution was occurred by SNP. In addition, the base encodes Gln, the 972nd residue of the amino acid sequence represented by SEQ ID NO: 1, but the substitution of A to G does not accompany a substitution of amino acid.

When homology with OWL by using Blast P [Nucleic Acids Res., 25, 3389, 1997] was examined, the human TCH204 variant 1 protein showed 74.2% homology to chicken calcium-dependent potassium channel cSlack (GenBank Accession #: AAM18770), 65.7% homology to rat calcium-dependent potassium channel Slack (Nature Neuroscience Vol. 1: 462, 1998; GenBank Accession #: AAC83350) and 63.2% homology to human potassium channel subunit protein KIAA1422 (GenBank Accession #: CAD 13242), respectively.

Further, from an analysis by hydropathy analysis software and a comparison of the amino acid sequence between Slack and KIAA1422 (Figs. 1 through 3), it is presumable that the human TCH204 variant 1 protein is a six transmembrane type protein and possesses a structure having a pore region between the fifth transmembrane region and the sixth transmembrane region.

As described above, it is considered that cDNA encoding the human TCH204 variant 1 protein is novel gene belonging calcium dependent potassium channel. The calcium dependent potassium channel is classified into three groups, i.e., Big-K (conductance: 100 - 220 ps), Intermediate-K (conductance: 20 - 85 ps) and Small-K (conductance: 2 - 20 ps) by channel conductance. Table I shows a homology between the human TCH204 variant 1 protein and a representative molecule in each family. In Table 1, BK, IK and SK represent Big-K, Intermediate-K and Small-K, respectively.

**[Table 1]**

| Class | Molecule | Accession No. | Report | Homology |
|---|---|---|---|---|
| BK | hSlo | AAA85104 | Molecular Brain Res., Vol. 27, p. 189,1994 | 14% |
| IK | hIK 1 | AAC23541 | Proc. Natl. Acad. Sci. USA, Vol. 94, p. 11651, 1997 | 11% |
| SK | hSK1 | AAB09562 | Science, Vol. 273, p. 1709, 1996 | 11 % |

### EXAMPLE 2

### Cloning of cDNA coding for human TCH204 variant 2 gene

Using the following synthetic DNA primers and human testis-derived cDNA as a template, amplification by PCR method was performed. The reaction solution comprised of 2.0 µl of human testis Marathon-Ready cDNA (CLONTECH), 2 µM each of synthetic DNA primers (SEQ ID NO: 3 and SEQ ID NO: 4), 0.2 mM dNTPs, 0.125 µl of Pyrobest DNA Polymerase (Takara Shuzo) and Pyrobest buffer attached to the enzyme to make the total volume 20 µl. The cycle for amplification was carried out using a thermal cycler (PE Biosystems) by heating of 94°C for 5 minutes, then a cycle set to include 94°C for 20 seconds followed by 67°C for 30 seconds and 72°C for 5 minutes, which was repeated 35 times, and finally, incubation at 72°C for 5 minutes. Then, the reaction solution comprised of 2 µl of the PCR reaction solution diluted 50-fold with DNase-, RNase-free distilled water, 2 µM each of synthetic DNA primers (SEQ ID NO: 5 and SEQ ID NO: 6), 0.2 mM dNTPs, 0.125 µl of Pyrobest DNA Polymerase (Takara Shuzo) and Pyrobest buffer attached to the enzyme to make the total volume 50 µl. The cycle for amplification was carried out using a thermal cycler (PE Biosystems) by heating of 94°C for 5 minutes, then a cycle set to include 94°C for 20 seconds followed by 67°C for 30 seconds and 72°C for 5 minutes, which was repeated 35 times, and finally, incubation at 72°C for 5 minutes. The amplified DNA was isolated by 1.5% agarose gel electrophoresis and about 3.5 kb band was excised by razor blade. The DNA was recovered using the QIAquick Gel Extraction Kit (QIAGEN). The DNA was cloned into pCRII-TOPO vector in accordance with the protocol of TOPO TA Cloning Kit Dual Promoter (Invitrogen). After transformation of Escherichia coli TOP10 competent cell (Invitrogen) by introducing the above-mentioned vector, clones harboring cDNA insert fragment were selected on LB agar medium containing kanamycin and X-gal. All the white-colored clones were isolated with sterilized toothpick, and then the transformants were obtained. Respective clones were cultured in LB medium containing kanamycin for overnight. Subsequently, the plasmid DNA was prepared using QIAprep Miniprep (QIAGEN). The reaction for determination of the base sequence was carried out using BigDye Terminator Cycle Sequencing Ready Reaction Kit (Applied Biosystems). As a result, with the DNA sequencer, ABI PPISM 3100 Analyzer (Applied Biosystems), the base sequence of cDNA fragment inserted was decoded.

The 3496 bases (SEQ ID NO: 10) were inserted into the acquired plasmid, and ORF encoding 1111 amino acid residues (SEQ ID NO: 8) were present in the cDNA fragment (SEQ ID NO: 9). The protein containing the amino acid sequence represented by SEQ ID NO: 8 was designated human TCH204 variant 2 protein. The transformant having the plasmid containing the above-mentioned cDNA fragment (SEQ ID NO: 10) was designated Escherichia coli TOP10/pCRII-TCH204V2.

### EXAMPLE 3

### Cloning of cDNA coding for human TCH204 variant 3 gene

Among a portion of the clones of Escherichia coli transformed with the same DNA as that of the PCR product used for obtaining the human TCH204 variant 2 gene in EXAMPLE 2, transformants harboring the human TCH204 variant 3 gene are present.

The 3346 bases (SEQ ID NO: 13) were inserted into the acquired plasmid, and ORF encoding 1061 amino acid residues (SEQ ID NO: 11) were present in the cDNA fragment (SEQ ID NO: 12). The protein containing the amino acid sequence represented by SEQ ID NO: 11 was designated human TCH204 variant 3 protein. The transformant having the plasmid containing the above-mentioned cDNA fragment (SEQ ID NO: 13) was designated Escherichia coli TOP10/pCRII-TCH204V3.

### EXAMPLE 4

### Cloning of cDNA coding for human TCH204 variant 4 gene

Using the following synthetic DNA primers and human testis-derived cDNA as a template, amplification by PCR method was performed. The reaction solution comprised of 2.0 µl of human testis Marathon-Ready cDNA (CLONTECH), 2 µM each of synthetic DNA primers (SEQ ID NO: 5 and SEQ ID NO: 6), 0.2 mM dNTPs, 0.125 µl of Pyrobest DNA Polymerase (Takara Shuzo) and Pyrobest buffer attached to the enzyme to make the total volume 20 µl. The cycle for amplification was carried out using a thermal cycler (PE Biosystems) by heating of 94°C for 5 minutes, then a cycle set to include 94°C for 10 seconds followed by 72°C for 7 minutes, which was repeated 5 times, 94°C for 10 seconds followed by 70°C for 7 minutes, which was repeated 5 times, 94°C for 10 seconds followed by 68°C for 7 minutes, which was repeated 25 times, and finally, incubation at 68°C for 10 minutes. Then, the reaction solution comprised of 2 µl of the PCR reaction solution diluted 50-fold with DNase-, RNase-free distilled water, 2 µM each of synthetic DNA primers (SEQ ID NO: 14 and SEQ ID NO: 6), 0.2 mM dNTPs, 0.125 µl of Pyrobest DNA Polymerase (Takara Shuzo) and Pyrobest buffer attached to the enzyme to make the total volume 50 µl. The cycle for amplification was carried out using a thermal cycler (PE Biosystems) by heating of 94°C for 5 minutes, then a cycle set to include 94°C for 10 seconds followed by 72°C for 7 minutes, which was repeated 5 times, 94°C for 10 seconds followed by 70°C for 7 minutes, which was repeated 5 times, 94°C for 10 seconds followed by 68°C for 7 minutes, which was repeated 25 times, and finally, incubation at 72°C for 5 minutes. The amplified DNA was isolated by 1.5% agarose gel electrophoresis and about 2.5 kb band was excised by razor blade. The DNA was recovered using the QIAquick Gel Extraction Kit (QIAGEN). The DNA was cloned into pCRII-TOPO vector in accordance with the protocol of TOPO TA Cloning Kit Dual Promoter (Invitrogen). After transformation of Escherichia coli TOP10 competent cell (Invitrogen) by introducing the above-mentioned vector, clones harboring cDNA insert fragment were selected on LB agar medium containing kanamycin and X-gal. All the white-colored clones were isolated with sterilized toothpick, and then the transformants were obtained. Respective clones were cultured in LB medium containing kanamycin for overnight. Subsequently, the plasmid DNA was prepared using QIAprep Miniprep (QIAGEN). The reaction for determination of the base sequence was carried out using BigDye Terminator Cycle Sequencing Ready Reaction Kit (Applied Biosystems). As a result, with the DNA sequencer, ABI PPISM 3100 Analyzer (Applied Biosystems), the base sequence of cDNA fragment inserted was decoded.

The 2576 bases (SEQ ID NO: 17) were inserted into the acquired plasmid, and ORF encoding 759 amino acid residues (SEQ ID NO: 15) were present in the cDNA fragment (SEQ ID NO: 16). The protein containing the amino acid sequence represented by SEQ ID NO: 15 was designated human TCH204 variant 4 protein. The transformant having the plasmid containing the above-mentioned cDNA fragment (SEQ ID NO: 17) was designated Escherichia coli TOP10/pCRII-TCH204V4.

The respective amino acid sequences of the human TCH204 variant 1 protein, the human TCH204 variant 2 protein, the human TCH204 variant 3 and the human TCH204 variant 4 protein are shown in Figs. 4 through 6.

### EXAMPLE 5

### Cloning of cDNA coding for mouse TCH204 variant 6 gene

Using the following synthetic DNA primers and mouse brain-derived cDNA as a template, amplification by PCR method was performed. The reaction solution comprised of 2.0 µl of mouse brain Marathon-Ready cDNA (CLONTECH), 2 µM each of synthetic DNA primers (SEQ ID NO: 20 and SEQ ID NO: 21), 0.2 mM dNTPs, 0.125 µl of Pyrobest DNA Polymerase (Takara Shuzo) and Pyrobest buffer attached to the enzyme to make the total volume 20 µl. The cycle for amplification was carried out using a thermal cycler (PE Biosystems) by heating of 94°C for 5 minutes, then a cycle set to include 94°C for 20 seconds followed by 68°C for 30 seconds and 72°C for 5 minutes, which was repeated 40 times, and finally, incubation at 72°C for 5 minutes. The amplified DNA was isolated by 1.5% agarose gel electrophoresis and about 3.5 kb band was excised by razor blade. The DNA was recovered using the QIAquick Gel Extraction Kit (QIAGEN). The DNA was cloned into pCRII-TOPO vector in accordance with the protocol of TOPO TA Cloning Kit Dual Promoter (Invitrogen). After transformation of Escherichia coli TOP10 competent cell (Invitrogen) by introducing the above-mentioned vector, clones harboring cDNA insert fragment were selected on LB agar medium containing kanamycin and X-gal. All the white-colored clones were isolated with sterilized toothpick, and then the transformants were obtained. Respective clones were cultured in LB medium containing kanamycin for overnight. Subsequently, the plasmid DNA was prepared using QIAprep Miniprep (QIAGEN). The reaction for determination of the base sequence was carried out using BigDye Terminator Cycle Sequencing Ready Reaction Kit (Applied Biosystems). As a result, with the DNA sequencer, ABI PPISM 3100 Analyzer (Applied Biosystems), the base sequence of cDNA fragment inserted was decoded. The 3498 bases were inserted into the acquired plasmid (SEQ ID NO: 22), and ORF encoding 1135 amino acid residues (SEQ ID NO: 18) were present in the cDNA fragment (SEQ ID NO: 19). The protein containing the amino acid sequence was designated human TCH204 variant 6 protein. The transformant having the plasmid containing the above-mentioned cDNA fragment was designated Escherichia coli TOP10/pCRII-mTCH204V6.

When homology with OWL by using Blast P [Nucleic Acids Res., 25, 3389, 1997] was examined, the human TCH204 variant 6 protein showed 96.4% homology to the human TCH204 variant 1 and 97.5% homology to the human calcium dependent potassium channel Slo4 (described in WO 02/40649 publication). Further, from an analysis by hydropathy analysis software and a comparison of the amino acid sequence between the human TCH204 and the Slo4, it is presumable that the protein is a six transmembrane type protein and possesses a structure having a membrane insertion region between the fifth transmembrane region and the sixth transmembrane region (Figs. 7 through 9).

As described above, it is considered that cDNA encoding the mouse TCH204 variant 6 protein is novel gene belonging calcium dependent potassium channel.

The calcium dependent potassium channel is classified into three groups, i.e., Big-K (conductance: 100 - 220 ps), Intermediate-K (conductance: 20 - 85 ps) and Small-K (conductance: 2 - 20 ps) by channel conductance. Table 2 shows a homology between the mouse TCH204 variant 6 protein and a representative molecule from mouse in each family. In Table 2, BK, IK and SK represent Big-K, Intermediate-K and Small-K, respectively.

**[Table 2]**

| Class | Molecule | Accession No. | Report | Homology |
|---|---|---|---|---|
| BK | mSlo | NP_034740 | Science, Vol. 261, p.221,1993 | 14% |
| IK | mIK1 | AAC32829 | | 13% |
| SK | mSK1 | Q9EQR3 | Biochim. Biophys. Acta Vol. 1518, p. 36, 2001 | 12% |

### EXAMPLE 6

### Cloning of cDNA coding for mouse TCH204 variant 5 gene

Among a portion of the clones of Escherichia coli transformed with the PCR product obtained in EXAMPLE 5, transformants harboring the mouse TCH204 variant 5 gene are present. The 3523 bases (SEQ ID NO: 25) were inserted into the acquired plasmid, and ORF encoding 1142 amino acid residues (SEQ ID NO: 23) were present in the cDNA fragment (SEQ ID NO: 24). The protein containing the amino acid sequence was designated mouse TCH204 variant 5 protein. The transformant having the plasmid containing the above-mentioned cDNA fragment was designated Escherichia coli TOP10/pCRII-mTCH204V5.

### EXAMPLE 7

### Cloning of cDNA coding for mouse TCH204 variant 2 gene

Among. a portion of the clones of Escherichia coli transformed with the PCR product obtained in EXAMPLE 5, transformants harboring the mouse TCH204 variant 2 gene are present. The 3430 bases (SEQ ID NO: 28) were inserted into the acquired plasmid, and ORF encoding 1111 amino acid residues (SEQ ID NO: 26) were present in the cDNA fragment (SEQ ID NO: 27). The protein containing the amino acid sequence was designated mouse TCH204 variant 2 protein. The transformant having the plasmid containing the above-mentioned cDNA fragment was designated Escherichia coli TOP10/pCRII-mTCH204V2.

Whereas the 2548th base in the sequence represented by SEQ ID NO: 27 was C, A at the 2745th position and A at the 2943rd position, the corresponding base of the other human TCH204 variant obtained in EXAMPLES 5 and 6 described above was T, G and G, respectively. It is considered that the base substitutions were occurred by SNP. In addition, the respective base substitutions do not accompany a substitution of amino acid.

### EXAMPLE 8

### Analysis for tissue distribution of gene product of each human TCH204 variant

For separately quantifying an expression level of each variant gene, sequences for primers and probes were designed from the sequences of respective human TCH204 variants.

For the human TCH204 variant 1, the synthetic DNA primers of SEQ ID NO: 29 and SEQ ID NO: 30, and the synthetic DNA probe for TaqMan PCR of SEQ ID NO: 31; for the human TCH204 variant 2, the synthetic DNA primers of SEQ ID NO: 32 and SEQ ID NO: 30, and the synthetic DNA probe for TaqMan PCR of SEQ ID NO: 33; for the human TCH204 variant 3, the synthetic DNA primers of SEQ ID NO: 32 and SEQ ID NO: 34, and the synthetic DNA probe for TaqMan PCR of SEQ ID NO: 35; for the human TCH204 variant 4, the synthetic DNA primers of SEQ ID NO: 36 and SEQ ID NO: 37, and the synthetic DNA probe for TaqMan PCR of SEQ ID NO: 38; for the human TCH204 variant 5, the synthetic DNA primers of SEQ ID NO: 39 and SEQ ID NO: 40, and the synthetic DNA probe for TaqMan PCR of SEQ ID NO: 41; and for the human TCH204 variant 6, the synthetic DNA primers of SEQ ID NO: 42 and SEQ ID NO: 43, and the synthetic DNA probe for TaqMan PCR of SEQ ID NO: 44; these synthetic DNA were designed, respectively.

In addition, the human TCH204 variant 5 shows the sequence disclosed as Potassium Channel 54414 in WO 02/055701 publication, and the human TCH204 variant 6 shows the sequence disclosed as human Slo4 in WO 02/40469 publication. These sequences have at least 95% homology to the human TCH204 variant 1, variant 2, variant 3 and variant 4, respectively, but did not coincide with the sequence thereof.

When we examined if the interested variant can only be quantified using the primers and probe, which was designed for quantification of the specific variant, with a plasmid DNA containing the base sequence encoding whole length amino acid sequence of each human TCH204 variant as a standard DNA, in the case of primers and probes for the human TCH204 variant 1, variant 3, variant 4, variant 5 and variant 6, only the interested variant were determined. On the other hand, it was found that in the case of primers and probe for the human TCH204 variant 2, the human TCH204 variant 1, variant 3, variant 4, variant 5 and variant 6 were determined in addition to the human TCH204 variant 2. However, due to the relation for the genetic sequences between the human TCH204 variant 2 and the other human TCH204 variants, merger of the human TCH204 variant 1, variant 3, variant 4, variant 5 and variant 6 was inevitable. Therefore, the quantification for the human TCH204 variant 2 was carried out with the inclusion of the human TCH204 variant 1, variant 3, variant 4, variant 5 and variant 6. The expression level of the human TCH204 variant 2 was estimated by subtracting respective expression level of the other human TCH204 variants from the aforementioned result.

The reaction composition for the quantification by the TaqMan PCR system was as follows: 0.8 µl of human various tissue-derived cDNA (CLONTECH, Human MTC Panel I and II), 10 µl of TaqMan Universal PCR Master Mix (Applied Biosystems), 0.2 µM each of synthetic DNA primers designed for the quantification of each human TCH204 variant and 0.2 µM synthetic DNA probe for the TaqMan to make the total volume 20 µl. The reaction composition for the quantification of glyceraldehyde phosphate dehydrogenase (GAPDH) in each cDNA was as follows: 0.8 µl of human various tissue-derived cDNA (CLONTECH, Human MTC Panel I and II), 10 µl of TaqMan Universal PCR Master Mix (Applied Biosystems), 0.2 µM each of synthetic DNA primers attached to the GAPDH Control Reagent 402869 (Applied Biosystems) and 0.1 µM synthetic DNA probe for the TaqMan attached to the same kit to make the total volume 20 µl. For the reaction, ABI PRISM 7900 Sequence Detection System was used. The reaction was performed at 50°C for 2 minute and 95°C for 10 minutes and then repeated 40 cycles set to include 95°C for 15 seconds and 60°C for one minute. Simultaneously at completion of the reaction, the quantification was conducted. The various human tissue-derived cDNAs used for determination were listed in Table 3.

**[Table 3]**

| | |
|---|---|
| Sample Name | Human Tissues, which are included in sample |
| Human MTC Panel I | Heart, Brain, Placenta, Lung, Liver, Skeletal muscle, Kidney, Pancreas |
| Human MTC Panel II | Spleen, Thymus, Prostate, Testis, Ovary, Small intestine, Colon, Peripheral Blood Leukocyte |

The results are shown in Figs. 13 through 18.

The results are represented by the relative expression level of each human TCH204 variant against the expression level of the GAPDH gene in each cDNA sample, i.e., the values obtained by dividing the expression level of each human TCH204 variant per unit volume of cDNA (copies/µl) into the expression level of the GAPDH gene per unit volume of cDNA (copies/µl).

The human TCH204 variant 1 was slightly expressed in ovary and testis.

In the case of the human TCH204 variant 2 system, the expression was detected in lung, small intestine, ovary, testis and prostate. As described above, since the human TCH204 variant 2 is quantified in the inclusion of the expression level of the other human TCH204 variants, it should be estimated by subtracting the expression level of the other human TCH204 variants. By this treatment, it was presumed that majority of the expression detected in lung, ovary and testis is derived from the human TCH204 variant 2.

In the human TCH204 variant 3, no expression was detected in the tissues other than testis. Further, the expression in testis was slight.

In the human TCH204 variant 4, as well as the case of the human TCH204 variant 3, no expression was detected in the tissues other than testis. Further, the expression in testis was slight.

The expression of the human TCH204 variant 5 was detected in small intestine, spleen, ovary and prostate.

The expression of the human TCH204 variant 6 was slightly detected in small intestine and ovary.

### EXAMPLE 9

### Electrophysiological properties of human TCH204 variant 2 gene product

To determine if an ion channel is formed by the human TCH204 variant 2, whole cell recording by fixing membrane potential was performed using patch-clamp techniques.

The human TCH204 variant 2, which was inserted into vector pcDNA3.1(+) (Invitrogen) using Nucleofector (AMAXA), was transiently expressed in CHO-K 1 cells (7 examples). After 24 or 48 hours, measurement was done. As a control, the cells, in which only the vector was introduced, were used (5 examples). A glass tube (GC150-10, Harvard-Clerk) was used as an electrode for patch. Further, the electrode was processed to make the chip resistance 5 - 10 megaohm. The compositions of filling solution and outer solution of the cells was in accordance with the description in the reference (Nature Neurosci., Vol. 1, pp. 462 - 469, 1998). The filling solution consisted of 32.5 mM KCI, 97.5 mM K-gluconate, 0.1 mM CaCL₂ and 10 mM HEPES (pH was adjusted to 7.2 with KOH). The outer solution of the cells consisted of 140 mM NaCl, 3.0 mM KCI, 1.0 mM CaCl₂-2H₂O, 29 mM D-glucose, 25 mM HEPES and 1 µM tetrodotoxin (pH was adjusted to 7.4 with NaOH). For whole cell recording, the membrane potential was held at -80mV with amplifier for patch, Axopatch 200B (Axon Instruments), and sampling at 5 kHz was carried out through A/D converter, which is filtered at 2 kHz (Digidata 1320A, Axon Instruments). These recording and data processing are totally performed on pCLAMP8 software (Axon Instruments). When a step pulse every 20 mV from -100 mV to +100 mV for 300 mili-seconds was given to the human TCH204 variant 2-introduced cells at 2 seconds-intervals, generation of potential-dependent electric current, which possesses an extrovert rectified component as compared to that of the control cells, was detected. The results are shown in Fig. 19. Isostatic potential for this current demonstrates a dependency on a K⁺ ion concentration in the outer solution of the cells. From the results, it was demonstrated that the human TCH204 variant 2 forms ion channel, presumably K⁺ ion-selective potential-dependent ion channel.

### INDUSTRIAL APPLICABILITY

The protein, the polynucleotide, the antibody and the like of the present invention are useful as a diagnostic marker for alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like. Compounds that activate or inhibit the activities of the protein, the polynucleotide or the antibody, compounds that activate or inhibit the expression of the gene encoding the protein, compounds that activate or inhibit the expression of the protein and the like, which are obtained by the screening method using the protein, can be used as a prophylactic and/or therapeutic agent for alimentary diseases (e.g., irritable bowel syndrome, chronic ulcerative colitis, Crohn's disease, ischemic colitis, gastritis, peptic ulcer, proctitis, diarrhea, reflux esophagitis, duodenitis, etc.), reproductive system diseases (e.g., prostate hyperplasia, prostatitis, testis neurosis, ovarian cystoma, etc.), respiratory diseases (e.g., chronic obstructive pulmonary disease, asthma, etc.), central nervous system diseases (e.g., Alzheimer's disease, parkinsonian syndrome, schizophrenia, cerebrovascular dementia, cerebral ischemia, convulsions, etc.), diabetes mellitus, hypertension, circulatory diseases (e.g., heart failure, arrhythmia, long QT syndrome, arteriosclerosis, angina, etc.), pancreatic diseases (e.g., pancreatitis, hypopancreatism such as cystic fibrosis pancreas, etc.), liver diseases (e.g., cirrhosis hepatic, etc.), kidney diseases (e.g., kidney failure, uremia, etc.), cancer (e.g., lung cancer, kidney cancer, liver cancer, non-small cell lung cancer, ovarian cancer, prostate cancer, stomach cancer, bladder cancer, breast cancer, cancer of uterine cervix, colon cancer, rectum cancer, pancreatic cancer, tymoma, testicular tumor, esophageal cancer, myosarcoma, etc.), or the like. Preferably, they can be used as a prophylactic and/or therapeutic agent for alimentary diseases, reproductive system diseases, respiratory diseases, or the like.

## Claims

1. A protein comprising the same or substantially the same amino acid sequence as the amino acid sequence represented by SEQ ID NO: 1, SEQ ID NO: 8, SEQ ID NO: 11 or SEQ ID NO: 15, or a salt thereof.

2. The protein according to claim 1 or a salt thereof, wherein the protein comprising the same or substantially the same amino acid sequence as the amino acid sequence represented by SEQ ID NO: 1, SEQ ID NO: 8, SEQ ID NO: 11 or SEQ ID NO: 15 is a protein consisting of the amino acid sequence represented by SEQ ID NO: 18, SEQ ID NO: 23 or SEQ ID NO: 26.

3. A protein consisting of the amino acid sequence represented by SEQ ID NO: 1, or a salt thereof.

4. A protein consisting of the amino acid sequence represented by SEQ ID NO: 8, or a salt thereof.

5. A protein consisting of the amino acid sequence represented by SEQ ID NO: 11, or a salt thereof.

6. A protein consisting of the amino acid sequence represented by SEQ ID NO: 15, or a salt thereof.

7. A partial peptide of the protein according to claim 1, or a salt thereof.

8. A polynucleotide comprising the polynucleotide encoding the protein according to claim 1 or the partial peptide according to claim 7.

9. The polynucleotide according to claim 8, which is a DNA.

10. A polynucleotide consisting of the base sequence represented by SEQ ID NO: 2, SEQ ID NO: 9, SEQ ID NO: 12 or SEQ ID NO: 16.

11. A polynucleotide consisting of the base sequence represented by SEQ ID NO: 19, SEQ ID NO: 24 or SEQ ID NO: 27.

12. A recombinant vector comprising the polynucleotide according to claim 8.

13. A transformant, which is transformed with the recombinant vector according to claim 12.

14. A method for manufacturing the protein according to claim 1 or the partial peptide according to claim 7 or salts thereof, which comprises culturing the transformant according to claim 13, producing and accumulating the protein according to claim 1 or the partial peptide according to claim 7 and collecting them.

15. A medicament comprising the protein according to claim 1 or the partial peptide according to claim 7 or salts thereof.

16. A medicament comprising the polynucleotide according to claim 8.

17. A diagnostic comprising the polynucleotide according to claim 8.

18. An antibody to the protein according to claim 1 or the partial peptide according to claim 7 or salts thereof.

19. A diagnostic comprising the antibody according to claim 18.

20. A medicament comprising the antibody according to claim 18.

21. A polynucleotide comprising a base sequence complementary or substantially complementary to the base sequence of the polynucleotide according to claim 8 or a portion thereof.

22. A medicament comprising the polynucleotide according to claim 21.

23. A method for screening a compound that enhances or inhibits activities of the protein according to claim 1 or the partial peptide according to claim 7 or salts thereof, or a salt thereof, which comprises using the protein according to claim 1 or the partial peptide according to claim 7 or salts thereof.

24. A kit for screening a compound that enhances or inhibits activities of the protein according to claim 1 or the partial peptide according to claim 7 or salts thereof, or a salt thereof, which comprises the protein according to claim 1 or the partial peptide according to claim 7 or salts thereof.

25. A compound that enhances or inhibits activities of the protein according to claim 1 or the partial peptide according to claim 7 or salts thereof, or a salt thereof, which is obtained by using the screening method according to claim 23 or the screening kit according to claim 24.

26. A medicament comprising the compound according to claim 25 or a salt thereof.

27. A method for screening a compound that enhances or inhibits an expression of the gene encoding the protein according to claim 1, which comprises using the polynucleotide according to claim 8.

28. A kit for screening a compound that enhances or inhibits an expression of the gene encoding the protein according to claim 1, which comprises the polynucleotide according to claim 8.

29. A compound that enhances or inhibits an expression of the gene encoding the protein according to claim 1, which is obtained by the screening method according to claim 27 or the screening kit according to claim 28.

30. A medicament comprising the compound according to claim 29 or a salt thereof.

31. A method for quantifying the protein according to claim 1, which comprises using the antibody according to claim 18.

32. A method for diagnosing a disease associated with the function of the protein according to claim 1, which comprises using the quantification method according to claim 31.

33. A method for screening a compound that enhances or inhibits an expression of the gene encoding the protein according to claim 1, which comprises using the antibody according to claim 18.

34. A kit for screening a compound that enhances or inhibits an expression of the gene encoding the protein according to claim 1, which comprises the polynucleotide according to claim 18.

35. A compound that enhances or inhibits an expression of the gene encoding the protein according to claim 1, which is obtained by the screening method according to claim 33 or the screening kit according to claim 34.

36. A medicament comprising the compound according to claim 35 or a salt thereof.

37. The medicament according to claim 16, claim 20, claim 22, claim 26, claim 30 or claim 36, which is a preventive and/or therapeutic agent for alimentary diseases, genital diseases or respiratory diseases.

38. The diagnostic according to claim 17 or claim 19, which is a diagnostic for alimentary diseases, genital diseases or respiratory diseases.

39. A preventing and/or treating method for alimentary diseases, genital diseases or respiratory diseases, which comprises administrating to mammal an effective amount of the compound according to claim 25, claim 29 or claim 35 or salts thereof.

40. Use of the compound according to claim 25, claim 29 or claim 35 or salts thereof for manufacturing a preventive and/or therapeutic agent for alimentary diseases, genital diseases or respiratory diseases.
